Europäisches Patentamt
European Patent Office
Office européen des brevets

(19) (11) Veröffentlichungsnummer: **0 278 530 A2**

(12) # EUROPÄISCHE PATENTANMELDUNG

(21) Anmeldenummer: 88102408.7

(51) Int. Cl.4: **C07K 5/06**, A61K 37/02

(22) Anmeldetag: 22.08.81

(30) Priorität: 30.08.80 DE 3032709
08.05.81 DE 3118191

(43) Veröffentlichungstag der Anmeldung:
17.08.88 Patentblatt 88/33

(60) Veröffentlichungsnummer der früheren Anmeldung nach Art. 76 EPÜ: 0 046 953

(84) Benannte Vertragsstaaten:
AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Geiger, Rolf, Prof. Dr.
Heinrich-Bleicher-Strasse 33
D-6000 Frankfurt am Main 50(DE)
Erfinder: Teetz, Volker, Dr.
An der Tann 20
D-6238 Hofheim am Taunus(DE)
Erfinder: Urbach, Hansjörg, Dr.
Le Lavandoustrasse 41
D-6242 Kronberg/Taunus(DE)
Erfinder: Schölkens, Bernward, Dr.
Hölderlinstrasse 62
D-6233 Kelkheim (Taunus)(DE)
Erfinder: Henning, Rainer, Dr.
Rotenhofstrasse 31
D-6234 Hattersheim am Main(DE)

(54) **Aminosäurederivate, Verfahren zu ihrer Herstellung, diese enthaltende Mittel und deren Verwendung.**

(57) Aminosäurederivate der Formel I

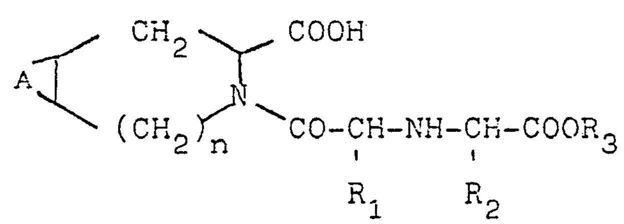

worin 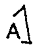, n, $R^1$, $R^2$ und $R^3$ die angegebenen Bedeutungen haben und deren Salze, Verfahren zu ihrer Herstellung, pharmazeutische Präparate auf Basis dieser Verbindungen sowie ihre Verwendung als Arzneimittel.

EP 0 278 530 A2

## Aminosäurederivate, Verfahren zu ihrer Herstellung, diese enthaltende Mittel und deren Verwendung

Die Erfindung betrifft eine Verbindung der Formel I

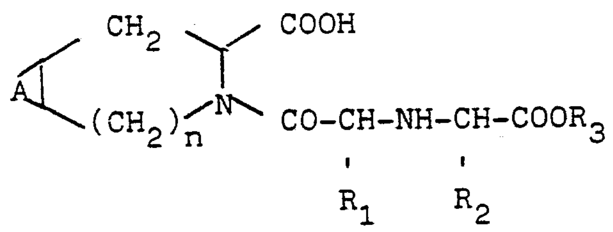

(I)

in welcher bedeutet
n = 0 und A einen Benzol-oder Cyclohexanring oder
n = 1 und A einen Cyclohexanring,
$R_1$ und $R_2$, die gleich oder verschieden und auch ihrerseits substituiert sein können, je
-Alkyl oder Alkenyl mit bis zu 6 C-Atomen,
-Cycloalkyl oder Cycloalkenyl mit je 5 bis 7 C-Atomen,
-Aryl oder teilhydriertes Aryl mit 6 bis 10 C-Atomen,
-ein mono-oder bicyclischer Heterocyclus mit 5 bis 7 bzw. 8 bis 10 Gliedern, davon 1 bis 2 -S-oder -O- und/oder bis zu 4 -N-Atomen,
$R_3$ Wasserstoff, Alkyl mit 1 bis 6 C-Atomen, Alkenyl mit 2 bis 6 C-Atomen oder Aralkyl mit 7 bis 14 C-Atomen.
und deren physiologisch verträgliche Salze.

Als bevorzugte Beispiel für $R_1$ und $R_2$ seien insbesondere genannt, Methyl, Ethyl, n-Propyl, Isopropyl, n-Butyl, Isobutyl, tert.-Butyl, sowie die geradkettigen und verzweigten Pentyle und Hexyle, ferner Cyclopentyl, Cyclohexyl, Cycloheptyl, Cyclopentenyl, Cyclohexenyl oder Cycloheptenyl, Phenyl, Napthyl, Di-und Tetrahydronaphthyl, Benzyl, Phenethyl, p-Fluorphenethyl, o-Methylphenethyl, p-Methoxyphenethyl, 2,4-Dichlorphenethyl, Cyclohexyläthyl.

Die 5-bis 7-gliedrigen mono-oder 9-, 10-gliedrigen bicyclische Heterocyclen können unsubstituiert sein, aber auch einen oder mehrere gleiche oder verschiedene Substituenten tragen wie Halogen, Sauerstoff (auch Sulfoxid oder Sulfon), Hydroxy, Carboxy, Carbonamido, Sulfonamido, Nitro, Alkyl und Aralkyl mit bis zu 9 C-Atomen, Methoxy oder Ethoxy. Im Falle einer Substitution ist Mono-oder Disubstitution bevorzugt.

Als bevorzugte Bedeutungen für $R_3$ seien genannt: Wasserstoff, Ethyl, Butyl, t-Butyl, Benzyl, p-Nitrobenzyl.

Die als $R_1$oder $R_2$ infragekommenden Alkyle oder Alkenyle können geradkettig oder verzweigt sein. Sie können einen oder mehrere gleiche oder verschiedene Substituenten tragen, insbesondere:

Cycloalkyl oder Cycloalkenyl mit 5-7 C-Atomen, Hydroxy Alkoxy mit 1 bis 2 C-Atomen, Aryloxy mit 1 bis 2 C-Atomen im Alkylteil, wobei der Alkylteil durch Methoxy, Ethoxy, Carboxy, Carbonamido, Amino oder Alkylamino und der Arylteil durch die genannten Substituenten und zusätzlich durch Halogen oder Nitro substituiert sein kann,

Amino,

Mono-, Di-oder Trialkyl-oder -cycloalkyl-amino mit bis zu insgesamt 7 C-Atomen in den Alkyl-oder Cycloalkylgruppen, das gegebenenfalls durch Hydroxy, Carboxy, Carbonamido, Carbethoxy, Amino, Alkyl-oder Dialkylamino, Piperidino oder Morpholino im Alkylrest substituiert sein kann,

Alkyl-, Aryl-, Aralkyl-oxycarbonylamino oder -ureido, Formyl, Alkanoyl-, Aroyl-oder Aralkanoylamino mit bis zu 10 C-Atomen, Arylamino, Aralkylamino, in denen der Arylteil durch Alkyl oder Alkoxy mit 1 bis 2 C-Atomen, Methylendioxy, Amino, Hydroxy, Acetoxy, Carboxy, Carbonamido, Carbethoxy, Halogen, oder Nitro mono-oder disubstituiert sein kann,

Alkyl-, Aryl-oder Aralkylmercapto mit bis zu 7 C-Atomen, wobei der Alkylteil durch Methoxy, Ethoxy, Hydroxy, Carboxy, Carbonamido, Carbethoxy, Amino oder Alkylamino und der Arylteil durch die genannten Reste und zusätzlich durch Halogen, Nitro oder Sulfonamido substituiert sein kann, ferner deren Sulfoxide und Sulfone,

Carboxy, Carbethoxy, Carbonamido, Alkyl-, Cycloalkyl-, Cycloalkylen-oder Dialkylaminocarbonyl mit bis

zu 6 C-Atomen, Aryl-oder Aralkylaminocarbonyl,

Guanido,

Phenyl, Naphthyl, Di-und Tetrahydronaphthyl, das durch Halogen, Hydroxy, Acetoxy, Carboxy, Carbonamido, Sulfonamido, Nitro, Methyl, Ethyl, Methoxy oder Ethoxy mono-oder disubstituiert sein kann,

5 bis 7-gliedrige mono-oder 9-bis 10-gliedrige bicyclische Heterocyclen, gegebenenfalls 1 bis 2 S-oder O-Atome und/oder bis 4 N-Atome pro Ring enthaltend, die gegebenenfalls wie oben substituiert sein können.

Unter Alkyl ist, falls im Einzelfall nichts anderes gesagt, vorzugsweise ein geradkettiges oder verzweigtes Alkyl mit 1 bis 6 C-Atomen zu verstehen. Gleiches gilt für Alkanoyl, Alkylamino, Alkylmercapto.

Aryl bedeutet bevorzugt Phenyl, durch Halogen, Alkyl oder Alkoxy substituiertes Phenyl oder Naphthyl. Gleiches gilt für Aroyl, Arylamino, Arylmercapto.

Aralkyl umfaßt bevorzugt Benzyl, Phenethyl und die entsprechenden, im Phenylkern mit Halogen, Nitro, Alkyl oder Alkoxy substituierten Verbindungen. Gleiches gilt für Aralkanoyl, Aralkylamino, Aralkylmercapto.

Verbindungen der allgemeinen Formel I, in der $R_1$ die Seitenkette einer natürlich vorkommenden L-Aminosäure darstellt, z.B. Methyl, Isobutyl, Methylthioethyl, Carboxymethyl, Carboxyethyl, Amino-n-butyl, Guanido-n-propyl, Imidazol-4-ethyl, Benzyl, 4-Hydroxybenzyl oder 3-Indolmethyl, sowie deren funktionelle Derivate wie Ether, Ester oder Amide sind besonders bevorzugt. Der Rest $R_1$ kann aber auch Teil von Mercapto-, Hydroxy-oder Aminosäuren sein, die nicht in der Natur vorkommen.

Gegenstand der Erfindung ist ferner ein Verfahren zur Herstellung der Verbindungen der Formel I, das dadurch gekennzeichnet ist, daß man

(a) eine Verbindung der Formel II mit einer Verbindung der Formel III umsetzt

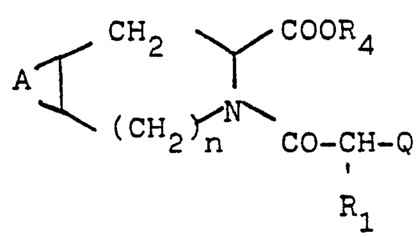

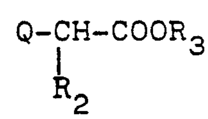

II III

in denen ein Q eine nucleofuge Gruppe und das andere Q $-NH_2$ bedeutet und $R_4$ für H, Methyl, Ethyl, Benzyl oder tert.-Butyl steht, und gegebenenfalls einer erhaltenen Ester in die Carbonsäure ($R_3$ und/oder $R_4$ = Wasserstoff) überführt, oder

(b) eine Verbindung der Formel IV

$POOC-\underset{R_1}{CH}-NH-\underset{R_2}{CH}-COOP$ IV

worin P H bedeutet, eines der beiden P jedoch auch die Bedeutung von $R_3$ oder tert.-Butyl haben kann, mit einer Verbindung der Formel V

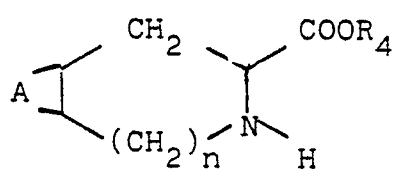

V

in Gegenwart eines Kondensationsmittels umsetzt, und gegebenenfalls eine oder beide Estergruppen in die Carbonsäure überführt, oder

(c) eine Verbindung der Formel VI mit einer Verbindung der Formel VII umsetzt

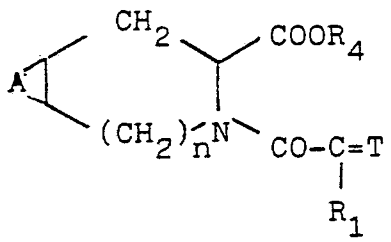
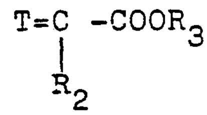

VI VII

in denen ein T für eine $NH_2$-Gruppe und das andere T für ein Sauerstoffatom steht, und die erhaltene Schiffsche Base reduziert.

Zur Herstellung der Verbindungen I kann man beispielsweise von 2-Halogencarbonsäurederivaten II oder III ausgehen, die mit Nucleophilen reagieren. Diese Reaktion wird bevorzugt in mit Wasser mischbaren organischen Lösungsmitteln, gegebenenfalls in Anwesenheit von Wasser, durchgeführt, wobei eine anorganische oder tertiäre oder quartäre organische Base zugegeben wird. Wenn die Reaktionspartner in aprotischen organischen Lösungsmittel vorzuziehen, wobei als Base vorteilhaft ein Trialkylamin, Tetraalkylammoniumhydroxyd oder Tetramethylguanidin oder eine Suspension eines Alkali-oder Erdalkalicarbonats zugesetzt werden.

Die Ausgangsprodukte

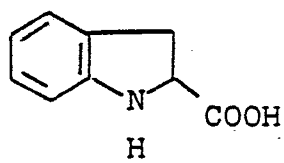
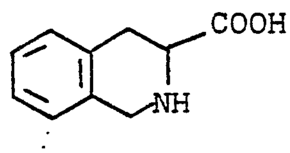

VIII IX

sind literaturbekannt, ihre spezifische pharmakologische Wirkung als Bestandteil der erfindungsgemäßen Verbindungen der Formel I wurde jedoch bisher nicht beschrieben. Die Verbindung VIII kann nach Aust. J. Chem. 20 (1967), Seite 1935, und die Verbindung IX nach J. Amer. Chem. Soc. 70 (1948), Seite 182 hergestellt werden.

Die Ausgangsprodukte

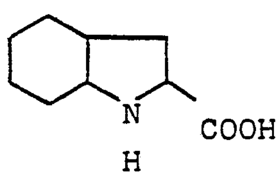
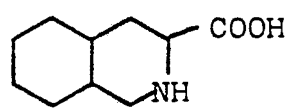

X XI

werden durch katalytische Hydrierung, bevorzugt an Rhodiumkatalysatoren, aus den Verbindungen der Formel VIII bzw. IX hergestellt.

Aus ihnen erhält man die Ester mit $R_4$ in bekannter Weise und kondensiert sie mit Halogencarbonsäuren zu den Verbindungen II (Q = Halogen) entweder über die Säurechloride, gemischte Anhydride, Aktivester oder andere Methoden, wie sie in Houben-Weyl, Methoden der Organischen Chemie, Band 15, ausführlich beschrieben sind. Bei $R_1$ = $CH_3$ kann mann z.B. von der aus L-Milchsäure leicht zugänglichen D-2-Chlorpropionsäure ausgehen.

Die Zwischenprodukte der Formel II (Q = $NH_2$) werden aus den Carbonsäureestern der allgemeinen Formel V durch Kondensation mit einer N-geschützten 2-Aminocarbonsäure erhalten. Die Schutzgruppe wird nach beendeter Kondensation wieder abgespalten. Als Schutzgruppe kommt z.B. Benzyloxycarbonyl in Frage.

Die Kondensation kann nicht nur mit DCC/HOBt (Dicyclohexylcarbodiimid/1-Hydroxybenzotriazol) sondern auch mit einem anderen gegeigneten Kondensationsmittel z.B. dem in Houben-Weyl, Band 15 beschriebenen, durchgeführt werden.

Die Verfahrensweise (b) geht von Verbindungen der Formel IV aus.

Sind $R_1$ und $R_2$ identisch, so kann P Wasserstoff sein. Man kondensiert dann z.B. in Gegenwart von Dicyclohexylcarbodiimid, mit einem Äquivalent einer Verbindung der Formel V und spaltet $R_4$ in bekannter Weise ab. Die Reaktion verläuft möglicherweise über das isolierbare Anhydrid XII, das anschließend mit einer Verbindung der Formel V geöffnet wird.

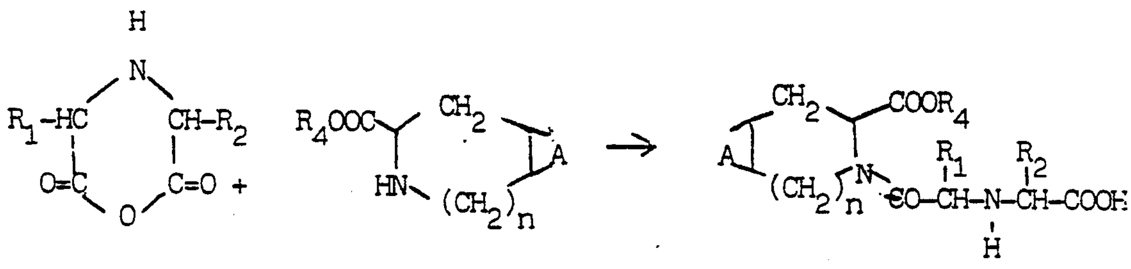

XII. V

Sind die Reste $R_1$ und $R_2$ in der Verbindung IV umgleich, so ist vorzugsweise nur einer der Reste P Wasserstoff, der andere sollte vorzugsweise die Bedeutung von $R^3$ haben oder tert.-Butyl sein. Man kondensiert dann in beschriebener Weise mit den Verbindungen der Formel V und überführt gegebenenfalls eine oder beide Estergruppen in die Carbonsäure.

Die fur T = $NH_2$ in den Formeln VI bzw. VII bedeutsame Kondensation mit 2-Ketocarbonsöuren bzw. deren Estern gemäß (c) führt in an sich bekannter Weise über Schiff'sche Basen z.B. nach Reduktion mit Natriumborhydrid oder Natriumcyanoborhydrid, durch katalytische Hydrierung oder elektrolytische Reduktion zu den entsprechenden Verbindungen der Formel I in guter Reinheit.

Die neuen Verbindungen besitzen unter anderem 3 chirale Zentren an $\alpha$-C-Atomen. Die Anordnung der Liganden entspricht bevorzugt der L-Konfiguration. Im allgemeinen können durch Kristallisation sterisch weitgehend einheitliche Verbindungen der Formel I erhalten werden. Bevorzugt sind Gegenstromverteilung oder präparative HPLC zur Anreicherung sterisch einheitlicher Formen.

Auf der Stufe der Zwischenprodukte X und XI kann mann gegebenenfalls Diastereomere durch Kristallisation oder präparative HPLC trennen, so daß nach dem oben beschriebenen Verfahren Produkte der Formel I erhalten werden können, die an allen chiralen Zentren eine einheitliche sterische Anrodnung besitzen.

Die neuen Verbindungen der Formel I besitzen eine langdauernde, intensive blutdrucksenkende Wirkung.

Sie können zur Bekämpfung des Bluthochdrucks verschiedener Genese eingesetzt und für sich oder in Kombination mit anderen blutdrucksenkenden, gefäßerweiternden oder diuretisch wirksamen Verbindungen angewandt werden. Typische Vertreter dieser Wirkklassen sind z.B. in Erhart-Ruschig, Arzneimittel, 2. Auflage, Weinheim 1972, beschreiben. Die Anwendung kann intravenös, subcutan oder peroral erfolgen.

Die Dosierung bei peroraler Gabe liegt bei 20 - 200 mg je Einzeldosis. Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden. Bei intravenöser oder subcutaner Verabreichung sollte die Einzeldosis zwischen 0,01 und 10 mg liegen.

Die Verbindungen der Formel I liegen als innere Salze vor. Falls beide Carboxylgruppen frei sind, können zusätzlich Alkali-und Erdalkalisalze und solche mit physiologisch unbedenklichen Aminen gebildet werden. Ferner kann eine vorhandene freie Aminogruppe mit einer Mineralsäure oder organischen Säure zu einem Salz umgesetzt werden. Auch die anderen Verbindungen der Formel I können in freier Form oder als solche Salze angewandt werden.

Die nachfolgenden Beispiele sollen die erfindungsgemäßen Verfahrensweisen erläutern, ohne die Erfindung auf die hier stellvertretend genannten Substanzen zu beschränken.

Beispiele

Folgende Abkürzungen werden gebraucht:
1.2.3.4-Tetrahydroisochinolin-3-carbonsäure Tic
Dekanhydroisochinolin-3-carbonsäure Dic
Indolin-2-carbonsäure Idc
Oktahydroindol-2-carbonsäure Oic
Benzyloxycarbonyl Z
tert-Butyloxycarbonyl Boc
tert-Butyl tBu, $Bu^t$, tert-$C_4H_9$
4-Nitrobenzyl Nb
Dicyclohexylcarbodiimid DCC
Dimethylformamid DMF
Dimethylacetamid DMA
N-Ethylmorpholin NEM
Cyclohexylamin CA
Dicyclohexylamin DCA

Soweit kein anderes Verfahren angegeben ist, werden die in den folgenden Beispielen beschriebenen Verbindungen zur Analyse und biologischen Bestimmung einer HPLC-Reinigung unterworfen.

Beispiel 1

N-(1-Carboxy-3-phenylpropyl)-L-alanyl-1,2,3,4-tetrahydroisochinolin-3-carbonsäure

a) 2-Carbobenzoxy-3-carboxy-1,2,3,4-tetrahydroisochinolin (Z-Tic)

188 g (1,05 Mol) 3-carboxy-1,2,3,4-tetrahydroisochinolin gibt man bei 0° zu 1050 ml 1 N -NaOH, und tropft dann bei dieser Temperatur unter Rühren gleichzeitig 160 ml Chlorkohlensäurebenzylester und weitere 1050 ml 1N-NaOH ein. Anschließend rührt man 2 Stunden bei Raumtemperatur. Man extrahiert dreimal mit Äther und säuert die alkalisch-wässrige Phase mit konz. HCl auf pH 1 an. Das ausgefallende Öl wird in Essigester extrahiert. Man wäscht die Essigesterlösung mit Wasser, bis die Wasserphase ein pH von 3,0 zeigt. Nach Trocknung über Natriumsulfat kristallisiert das Produkt aus der Essigesterlösung nach Einengen und Anreiben. Man setzt 1,5 Liter Diisopropyläther zu der Kristallsuspension und rührt eine Stunde bei Raumtemperatur, saugt ab, und trocknet das Produkt im Hochvakuum über Phosphorpentoxyd.
Ausbeute: 256,7 g Schmp. 138-39°C

b) 2-Carbobenzoxy-3-carboxy-1,2,3,4-tetrahydroisochinolin-tert.-butylester

Zur Lösung von 248,8 g (0,8 Mol) 2-Carbobenzoxy3-carboxy-1,2,3,4-tetrahydroisochinolin in 1600 ml Methylenchlorid gibt man 312 ml tert.Butanol und 8 g 4-Dimethylaminopyridin. Man kühlt auf -5 °C und setzt dann portionsweise die Lösung von 176 g Dicyclohexylcarbodiimid in 350 ml Methylenchlorid zu. Man rührt 5 Stunden bei 0° und läßt dann 16 Stunden bei Raumtemperatur stehen. Nach Absaugen vom Dicyclohexylharnstoff extrahiert man die Reaktionslösung dreimal mit gesättigter Natriumbicarbonatlösung. Man trocknet über Magnesiumsulfat, engt i.V. bei Raumtemperatur bis zur öligen Konsistenz ein. Es verbleiben 286 g Produkt als gelbliches Öl (97% d.Th.)
NMR: 7.30s (5H); 7,20s (4H); 5,1-4,3m (3H); 5,0s (2H); 1,46s (9H)

c) 3-Carboxy-1,2,3,4-tetrahydroisochinolin-tert-butylester-hydrochlorid

284 g 2-Carbobenzoxy-3-carboxy-1,2,3,4-tetrahydroisochinolin-tert.-butylester (0,775 Mol) löst man in 3 Liter Methanol, gibt 15 g 10 % Pd-Bariumsulfatkatalysator zu und hydriert mit Wasserstoff bei Normaldruck. Der pH-Wert der Lösung wird durch Zutropfen von 1 n methanolischer HCl auf pH 4,0 (Glaselektrode) gehalten. Man saugt vom Katalysator ab und dampft die Lösung im Vakuum bei Raumtemperatur zur Trockne ein. Das kristallisierte Produkt wird mit wasserfreiem Äther verrieben, abgesaugt und i.V. über Phosphorpentoxyd getrocknet.

Ausbeute: 156 g Schmp. 180 ° (Zers.)

Das Tosylat kann entweder durch Zugabe von methanolischer Toluolsulfosäure anstelle von methanolischer Salzsäure während der Hydrierung erhalten werden, oder man löst das Hydrochlorid in Wasser, versetzt mit der berechneten Menge Natriumtosylat und kühlt unter Animpfen und Rühren auf ca. 4 ° C ab. Dabei fällt das Tosylat in kristalliner Form aus. Die Kristalle werden abfiltriert, mit Wasser gewaschen und getrocknet.

Schmp. 139 - 140 ° (Zers.)

d) Carbobenzoxy-L-alanyl-3-carboxyl-1,2,3,4-tetrahydroisochinolin-tert.-butylester

Man suspendiert 27 g (0,15 Mol) 3-Carboxy-1,2,3,4-tetra-hydroisochinolin-tert.-butylesterhydrochlorid in 200 ml Dimethylformamid, kühlt unter Rühren und Feuchtigkeitsausschluß auf -5°C, und gibt 19 ml (0,15 Mol) N-Äthylmorpholin sowie die Lösung von 33,4 g Carbobenzoxyalanin, 20 g N-Hydroxybenzotriazol und 33 g Dicyclohexylcarbodiimid in 100 ml Di-methylformamid zu. Man rührt eine Stunde bei 0 °C, läßt über Nacht bei +4 °C stehen und arbeitet dann nach einstündigem Rühren bei Raumtemperatur auf. Man saugt vom ausgeschiedenen Dicyclohexylharnstoff ab und bringt die Reaktionslösung im Hochvakuum bei Raumtemperatur zur Trockne. Das verbliebene Öl nimmt man in 1 l Essigester auf und wäscht dreimal mit je 150 ml gesättigter Natriumcarbonatlösung, 5 % Kaliumbisulfatlösung und einmal mit Wasser. Nach Trocknung über wasserfreiem Natriumsulfat wird das Lösungsmittel i.V. bei Raumtemperatur abdestilliert.

Ausbeute: 59g Oel

e) L-Alanyl-3-carboxy-1,2,3,4-tetrahydroisochinolin-tert.-butylester-hydrochlorid (Ala-Tic-O-But)

Man hydriert 15 g Carbobenzoxy-alanyl-3-carboxy-1,2,3,4-tetrahydroisochinolin-tert.-butylester in 400 ml Methanol mit 8 g 10 % Pd/Bariumsulfatkatalysator. Das scheinbare pH der Lösung (Glaselektrode) wird durch Zugabe von 1 N methanolischer Salzsäure bei 4,0 gehalten. Nach 8 Stunden saugt man vom Katalysator ab und dampft die Lösung i.V. bei Raumtemperatur zur Trockne. Den festen Rückstand digeriert man mit Diisopropyläther und trocknet i.V..

Schmp. 110° (Zers.)

Analog Ala-Tic-OBut werden dargestellt:

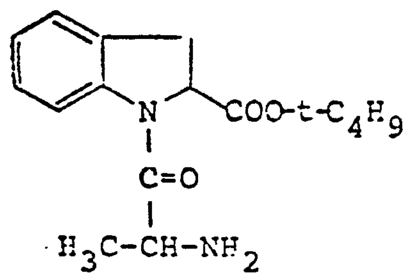

7.3-6.5m (4H); 4,4t (1H); 3,8-3,0 m+d (3H); 1,4s (9H); 1,2d (3H)

| | |
|---|---|
| 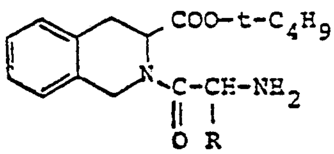 | nachstehenden Verbindungen sind folgende Signale gemeinsam: 7,2s (4H); 5,1-4,3m (3H); 3,9-3,0m (4H); 1,4s (9H) |
| für R : H | 7,2s (4H); 3,9-3,0m (5H) |
| 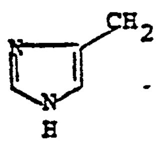 | 13,1s (1H); 7,5s (1H); 6,8s (1H); 2,8m (2H) |
| 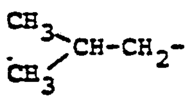 | 1,5m (3H); 0,9d (6H) |
| $CH_2F-$ | 5,1-4,3m (5H) |
| 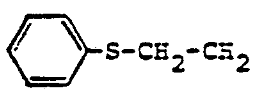 | 7,2-7,0m (9H); 2,7-2,0m (4H) |
| 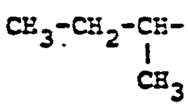 | 1,5-1,0m (9H) |
| 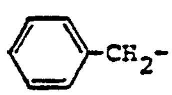 | 7,1s (5H); 2,7d (2H) |
| 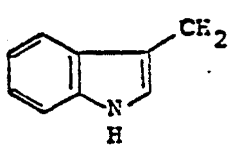 | 7,8-6,4m (9H) |
| $HO-CH_2-$ | 3,7d (2H) |

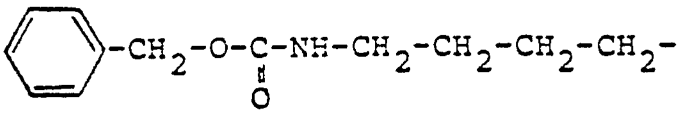

7,1s (5H); 5,0s (2H); 2.4m (2H); 1,8-1,3m (6H)

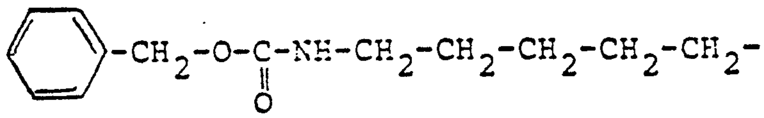

7,1s (5H); 5,0s (2H); 2,4m (2H); 1,8-1,3m (8H)

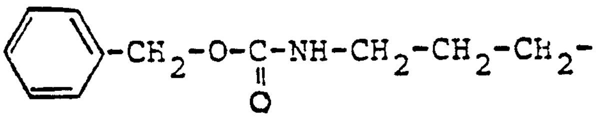

7,1s (5H); 5,0s (2H); 2,4m (2H); 1,8-1,3m (4H)

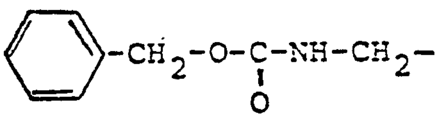

7,1s (5H); 5,0s (2H); 2.3m (2H)

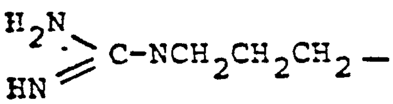

8,3-7,6m (2H); 2,9-1,6m (6H)

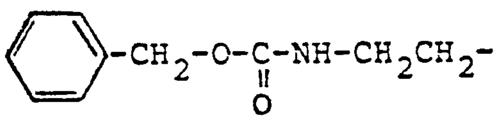

7,1s (5H); 5,0s (2H); 2,4m (2H); 1,7-1,4m (2H)

f) N-(1-Carboxy-3-phenylpropyl)-L-alanyl-1,2,3,4-tetrahydroisochinolin-3-carbonsäure-tert.-butylester

305 mg Alanyl-1,2,3,4-tetrahydroisochinolin-3-carbonsäurebutylester (Ala-Tic-OBut) und 445 mg 4-Phenyl-2-oxo-buttersäure werden in 4 ml Methanol gelöst und mit wässriger 1 N NaOH auf pH 7,5 gebracht. Man fügt 200 mg Natriumcyanoborhydrid hinzu und läßt 36 Std. reagieren. Man engt zur Trockne ein und gewinnt die Substanz durch Säulenchromatographie an Kieselgel (Laufmittelsystem: Chloroform/Methanol/Wasser/Eisessig 20 + 15 + 2 + 1).
Ausbeute: 382 mg; Schmp. ab 120° Zers.

g) N-(1-Carboxy-3-phenylpropyl)-L-alanyl-1,2,3,4-tetrahydroisochinolin-3-carbonsäure

350 mg N-(1-Carboxy-3-phenylpropyl)-Ala-Tic-OBut werden in 3 ml wasserfreier Trifluoressigsäure gelöst und 30 Min. bei Raumtemperatur belassen. Man engt i.Vak. ein und verreibt das hinterbleibende Öl mit kaltem Diisopropyläther und Petroläther.
Ausbeute 276 mg amorphe Substanz
NMR: 7.20 u. 7,10 (s); 4,3 (s, breit); 3,0-3,9 (m, breit); 1,23 u. 1,15 (d)

Beispiel 2

N-(1-Carbethoxy-3-phenyl-propyl)-L-alanyl-1,2,3,4-tetrahydroisochinolin-3-carbonsäure

Man löst 4,8 g H-Ala-Tic-O $Bu^t$ • Tos-OH in 40 ml absol. Ethanol, stellt mit KOH in Ethanol auf pH 7 und gibt 6,2 g 2-Oxo-4-phenylbuttersäure-ethylester zu und rührt in Anwesenheit von 9 g pulverisiertem Molekularsieb 4 Å unter langsamer Zugabe von einer Lösungvon 2 g Natriumcyanoborhydrid in 15 ml absol. Ethanol. Nach etwa 20 h wird filtriert und das Lösungsmittel im Vakuum abdestilliert. Der Rückstand wird zwischen Essigester und Wasser verteilt, die Essigesterphase abgetrennt und im Vakuum zur Trockne gebracht. Der Rückstand wird an Silicagel im Laufmittel Essigester/Cyclohexan (1:2 bis 1:4) chromatographiert.

Das Produkt wird in 20 ml Trifluoressigsäure gelöst und 30 Min. bei Raumtemperatur gerührt. Man destilliert die Trifluoressigsäure im Vakuum ab, destilliert mit Toluol nach und chromatographiert über Kieselgel in Methylenchlorid/Methanol (10 :1).
Ausbeute: 2,2 g

Beispiel 3

N-(1-Carboxy-5-aminopentyl)-L-alanyl-1,2,3,4-tetrahydroisochinolin-3-carbonsäure

1.2 g N-(1-Carboxy-5-Boc-aminopentyl)-L-alanyl-1,2,3,4-tetrahydroisochinolin-3-carbonsäure wurden mit 10 ml eiskalter Trifluoressigsäure übergossen und eine Stunde bei Raumtemperatur gerührt. Anschließend wird im Vakuum eingedampft. Der Rückstand wird in Wasser gelöst und einer Gefriertrocknung unterworfen.
Ausbeute: 0.95 g

Beispiel 4

N-(1-Carboxy-3-phenyl-propyl)-L-alanyl-1,2,3,4-tetrahydroisochinolin-3-carbonsäure

880 mg N-(1-Carbethoxy-3-phenyl-propyl)-L-alanyl-1,2,3,4-tetrahydroisochinolin-3-carbonsäure werden in 20 ml Dioxan / Wasser (9 : 1) gelöst und bei Raumtemperatur mit 4.5 ml 1 n Natriumhydroxid-Lösung verseift. Nach einer Stunde wird die Lösung mit der Äquivalenten Menge 1 n Salzsäure angesäuert und das Dioxan weitgehend abgedampft. Der Rückstand wird mehrfach mit Essigester extrahiert und die organische Phase nach Abziehen des Lösungsmittels über einen Kationenaustauscher (DOWEX 50) chromatographiert.
Ausbeute: 630 mg

Beispiel 5

N-(1-Carbethoxy-3-amino-propyl)-L-alanyl-1,2,3,4-tetrahydroisochinolin-3-carbonsäure

1.1 g N-(1-Carbethoxy-3-benzoxycarbonylamino-propyl)-L-alanyl-1,2,3,4-tetrahydroisochinolin-3-carbonsäure werden in 150 ml Methanol gelöst und in Gegenwart von 100 mg Palladium - Aktivkohle (10 %ig) bei 40° C unter Normaldruck hydriert. Nach Beendigung der Reaktion wird vom Katalysator abfiltriert und das Lösungsmittel im Vakuum abgezogen.
Ausbeute: 0.79 g

Beispiel 6

N-(1-Carboxy-3-phenyl-3-thiapropyl)-alanyl-1,2,3,4-tetrahydroisochinolin-3-carbonsäure

a.) 2-(2-Oxo-propionyl)-1,2,3,4-tetrahydroisochinolin-3-carbonsäure-tert.-butylester

In eine auf -50 °C gekühlte Lösung von 700 mg Tic-OBut und 630 mg Dicyclohexylcarbodiimid in 15 ml wasserfreiem Chloroform wird eine gekühlte und frisch destillierte Lösung von 270 mg Brenztraubensäure in 5 ml Chloroform rasch eingerührt. Man beläßt 16 Std. im Tiefkühlschrank (-20 °C) und filtriert den ausgefallenen DC-Harnstoff ab. Die Lösung wird mit $KHSO_4$-und anschließend mit $KHCO_3$-Lösung gewaschen, über $Na_2SO_4$ getrocknet und i.Vak. eingeengt. Die Verbindung (Öl) enthält etwas Dicyclohexylharnstoff; sie wurde zur Analyse über Kieselgel im System $CHCl_3/CH_3OH$ 15:1 chromatographiert.

Analog der Kondensation Brenztraubensäure mit Tic-OBut werden folgende Kondensationsprodukte aus Tic, Idc und der entsprechenden α-Ketocarbonsäure dargestellt.

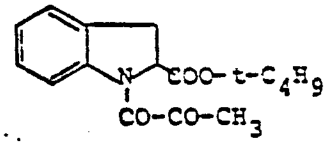

7,3-6,5m (4H); 2,4s (3H); 1,4s (9H)

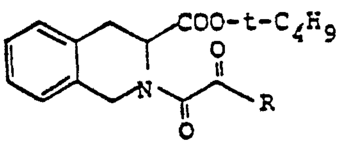

bei nachstehenden Verbindungen werden einheitlich folgende Signale für das Grundgerüst beobachtet 7,2s (4H); 5,1-4,3m (3H); 3,9-3,0m (2H); 1,4s (9H)

| | |
|---|---|
| 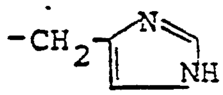 | 13,1s (1H); 7,5s (1H); 6,8s (1H); 3,9-3,0m (4H) |
| $-CH_2-CH(CH_3)_2$ | 2,4-1,0 m+d (9H) |
| 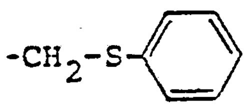 | 7,2-7,0m (9H); 2,5m (2H) |
| $-CH_2F$ | 4,4s (2H) |
| 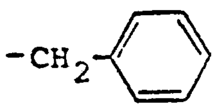 | 7,15s (5H); 3,0s (2H) |
| 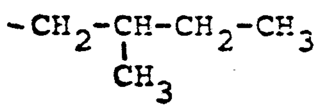 | 2.4-1,9m (3H); 1,5-1,0m (8H) |
| 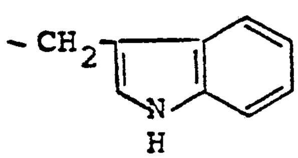 | 7,8-6,4m (9H); 3,9-2,9m (4H) |

b.) N-(1-Carboxy-3-phenyl-3-thiapropyl)-alanyl-1,2,3,4-tetrahydroisochinolin-3-carbonsäure-tert.-butylester

1,52 g Pyruvyl-Tic-OBut und 394 mg S-Phenyl-cystein werden in 5 ml Methanol gelöst und mit 1N NaOH (wässrig) auf pH 7.0 gebracht. Man fügt 400 mg Natriumcyanoborhydrid zu und beläßt 24 Stdn. bei Raumtemperatur. Man engt i.Vak. ein, nimmt in Chloroform auf, trocknet über Natriumsulfat und engt ein. Das Produkt wird durch Kieselgelchromatographie (System $CHCl_3/CH_3OH/HAcO/H_2O$ 50/20/5/1) rein erhalten.
NMR: 7,3-7,0m (9H); 5,1-4,3m (3H); 3,9-3,0m (4H); 2,5m (2H)

c.) N-(1-Carboxy-3-phenyl-3-thiapropyl)-alanyl-1,2,3,4-tetrahydroisochinolin-3-carbonsäure

1 g Butylester aus Beispiel 6b wird in 5 ml Trifluoressigsäure gelöst. Man engt nach 30 Min. i.Vak. ein, digeriert den Rückstand mit Diisopropyläther und trocknet über Kaliumhydroxyd.
Ausbeute 0,95 g Trifluoracetat (hygroskopisch)
NMR: 7,4-7,1m (9H); 5,2-4,4m (3H); 3,9-3,0m (4H); 2,5m (2H); 1,25m (3H)

Beispiel 7

N-(1-Carbethoxy-2-benzylsulfinylethyl)-L-alanyl-1,2,3,4-tetrahydroisochinolin-3-carbonsäure

Zu einer unter Eiskühlung gerührten Mischung von 0,64 g Natriumperjodat und 20 ml Wasser werden 1,3 g N-(1-Carbethoxy-3-benzylthio-ethyl)-L-alanyl-L-1,2,3,4-tetrahydroisochinolin-3-carbonsäure gelöst in 20 ml Methanol gegeben. Man läßt die Reaktionsmischung für 24 Stunden bei 0 °C rühren und filtriert anschließend vom Natriumjodat ab. Das Filtrat wird mehrfach mit Methylenchlorid extrahiert. Nach Entfernen des Lösungsmittels erhält man 1,1 g des Sulfoxids. Im NMR-Spektrum werden folgende Signale beobachtet: 7,3-7.0m (9H); 5,1-4,3m (3H); 4,1s (2H); 3,9-3,0m (4H); 2,6m (2H); 1,2d (3H)

Beispiel 8

N-(1-Carbethoxy-3-phenylsulfonyl-propyl)-L-alanyl-1,2,3,4-tetrahydroisochinolin-3-carbonsäure

260 mg N-(1-Carbethoxy-3-phenylsulfonyl-propyl)L-alanyl-1,2,3,4-tetrahydroisochinolin-3-carbonsäure und 20 mg Natriumwolframat Dihydrat werden in 50 ml Wasser gegeben und tropfenweise mit 1 ml Perhydrol (30 %ig) versetzt. Die Mischung wird für 30 min auf 80 C erwärmt und anschließend eine Stunde bei Raumtemperatur gerührt. Das überschüssige Peroxid wird sodann mit Palladium auf Bariumsulfat zerstört und nach Beendigung der Sauerstoffentwicklung wird die Lösung vom Palladium Katalysator abfiltriert und eingeengt. Das Rohprodukt wird durch Ionenaustauschchromatographie über DOWEX 50 $H^+$-Form gereinigt.
Ausbeute: 220 mg
NMR: 7,7m (9H); 5,1-4,3m (3H); 3,9-3,0m (4H); 2,8m (2H); 1,5m (2H); 1,2d (3H)

Beispiel 9

N-(2-Amino-1-carboxypropyl)-L-alanyl-1,2,3,4-tetrahydroisochinolin-3-carbonsäure

2.2 g $N^{\beta}$-Boc-$\alpha,\beta$-Diaminobuttersäure, hergestellt nach Coll. czech. chem. Commun. 31, 2955 (1966) analog der $N^{\gamma}$-Boc-Verbindung, werden mit 2.4 g Pyruvyl-Tic-OBut, hergestellt nach Beispiel 6 a), in der in Beispiel 6 b) beschriebenen Weise umgesetzt. Die Boc-Schutzgruppe wird anschließend auf die in Beispiel 3 beschriebene Weise abgespalten.
Ausbeute: 2.6 g

Beispiel 10

N-(1-Carboxy-3-phenylpropyl)-L-alanyl-1,2,3,4-dekahydroisochinolin-3-carbonsäure

a. L-Dekahydroisochinolin-3-carbonsäure (Dic)

250 g L-alanyl-1,2,3,4-tetrahydroisochinolin-3-carbonsäure werden in 2 l 90-proz. Essigsäure suspendiert. Man gibt 10 g Rhodium auf Kohle zu und hydriert 24 Stunden bei 60-80°C und 120 bar. Die filtrierte Lösung wird eingeengt, der Rückstand in 200 ml Essigester aufgenommen und unter kräftigem Rühren in 2 l Diisopropylether eingetropft. Man dekantiert vom harzigen Rückstand, engt die Lösung im Vakuum ein und wiederholt dieselbe Prozedur mit etwa 1/3 der vorher verwendeten Lösungsmittelmenge. Die harzigen Niederschläge werden vereinigt und mit heißem Essigester behandelt, wobei nur ein Teil in Lösung geht. Man läßt unter kräftigem Rühren in 3 l Diethylether-Diisopropylester 1:1 einlaufen, wobei ein flockiger Niederschlag entsteht, der abfiltriert, mit Ether gewaschen und getrocknet wird. Ausbeute: 234 g; dünnschichtchromatographisch nicht einheitlich (Diastereoisomerengemisch). Nach UV-Spektrum und NMR kein Aromat mehr vorhanden, Elementaranalyse korrekt.

Das Material wird ohne weitere Reinigung für die nächste Stufe eingesetzt.

b. N-Benzyloxycarbonyl-L-dekahydroisochinolin-3-carbonsäure (Z-Dic-OH)

58 g L-Dekahydroisochinolin-3-carbonsäure werden in 315 ml 1N NaOH gelöst. Unter kräftigem Rühren läßt man bei 0-5°C gleichzeitig 48 ml Benzyloxycarbonylchlorid und 370 ml 1N NaOH innerhalb einer Stunde zutropfen. Dabei fällt ein dicker Niederschlag aus. Man rührt nach beendeter Zugabe noch 2 Stdn. weiter, extrahiert dann mit Ether und filtriert den Niederschlag ab (=Natriumsalz eines Diastereoisomeren "A"; Ausbeute 43 g). Das Filtrat wird mit konz. HCl auf pH 1,5 - 2 gebracht, wobei sich ein Öl absetzt. Es wird in Essigester aufgenommen. Die Wasserphase wird mit Essigester extrahiert, die vereinigten Essigesterlösungen werden mit Wasser gewaschen, über Natriumsulfat getrocknet und auf ein kleines Volumen (ca. 200 ml) eingeengt. Bei Zugabe von 15-18 ml Cyclohexylamin (CA) fällt ein Niederschlag aus, der nach Kühlen mit Eiswasser abfiltriert und mit wenig kaltem Essigester gewaschen und dann getrocknet wird. Ausbeute: 48 g, (CA-Salz des Isomerengemisches B).
Das Natriumsalz von "A" wird wie folgt in das CA-Salz übergeführt: Man suspendiert die Verbindung in 500 ml Wasser, überschichtet mit 200 ml Essigester und säuert mit konz. HCl bis pH 1,5 - 2 an. Nach Ausschütteln trennt man die Essigesterphase ab, wäscht mit etwas Wasser und trocknet über Natriumsulfat. Man engt wie oben ein und versetzt mit 16 ml Cyclohexylamin. Das CA-Salz wird wie oben isoliert.
Zur Reinigung wird das CA-Salz von "A" aus der achtfachen Menge Essigester umkristallisiert. Schmelzpunkt: 197 - 198°C, $[\alpha]_D$ = 7,5° (c = 1, in Methanol). Elementaranalyse korrekt.

Das CA-Salz von "B" wird aus der vierfachen Menge Essigester umkristallisiert. Schmp.: 190° (sintert ab 187°) 1 $[\alpha]_D$ = + 14,6° (c=1, in Methanol) Elementaranalyse korrekt.

Die freien Säuren werden in bekannter Weise durch Suspendieren der CA-Salze in Wasser/Essigester und Ansäuern mit Citronensäure erhalten. Die Essigesterphasen werden mit wenig Wasser gewaschen und eingeengt. Man erhält Z-Dic-OH als öligen Rückstand.

c. Benzyloxycarbonyl-L-dekahydroisochinolin-3-carbonsäure-tert.-butylester (Z-Dic-OBu$^t$).

41 g Z-Dic-OH ("A") werden in 350 ml Methylenchlorid gelöst. Man gibt 52 ml t-Butanol und 1,3 g 4-Dimethylaminopyridin zu, kühlt auf 0°C und tropft unter Rühren die Lösung von 29 g Dicyclohexylcarbodiimid (DCC) in 60 ml Methylenchlorid zu. Man rührt anschließend noch 20 min. bei 0°C und 5 h bei Raumtemperatur und filtriert den ausgefallenen Dicyclohexylharnstoff ab. Das Filtrat wird im Vakuum eingedampft, der Rückstand in Essigester aufgenommen, die Lösung nacheinander mit $KHSO_4/K_2SO_4$-Lösung, Natriumhydrogencarbonat (nicht-umgesetztes Na-Salz von Z-Dic-OH fällt aus und wird abfiltriert) und Wasser gewaschen, getrocknet und im Vakuum zur Trockene gebracht. Öliger Rückstand. Ausbeute: 38,5 g $[\alpha]_D$ = -17,3° (c=1, in Methanol). Zur Gewinnung der analogen Verbindung von "B" geht man in derselben Weise vor. Ausbeute 38,1 g $[\alpha]_D$= + 6,7° (c=1, in Methanol).

d. L-Dekahydroisochinolin-3-carbonsäure-tert.-butylester-tosylat (H-Dic-OBu$^t$•TosOH).

27 g der nach Beispiel 10c hergestellten Verbindung von "A" werden in 250 ml Methanol unter Zutropfen von 4N TosOH in Methanol an Pd/Kohle bei pH 4,5 (pH-Stat) katalytisch hydriert. Dann engt man die filtrierte Lösung im Vakuum ein, digeriert den kristallinen Rückstand mit Ether und trocknet. Ausbeute: 22,6 g Zur Analyse wird aus Essigester umkristallisiert. Schmp.: 159-160°C; $[\alpha]_D$ = -6,5° (C=1, in Methanol). Elementaranalyse korrekt. Die Verbindung ist dünnschichtchromatographisch einheitlich.

Analog verfährt man mit der nach Beispiel 10c hergestellten Verbindung des Isomerengemisches "B". Dabei wird nach Umkristallisieren aus Essigester eine Substanz vom Schmp. 162-164°C, $[\alpha]_D$ = +4,4° (c=1, in Methanol) erhalten. Elementaranalyse korrekt.

e.) N-Benzyloxycarbonyl-L-alanyl-L-dekahydroisochinolin-3-carbonsäure-tert.-butylester (Z-Ala-Dic-OBU$^t$)

12,4 g H-Dic-OBu$^t$. TosOH, hergestellt nach Beispiel 10d mit Verbindung "A", werden in 250 ml Methylenchlorid gelöst. Man gibt 6,7 g Z-Ala-OH und 4,05 g 1-Hydroxybenzotriazol (HOBt) zu, versetzt mit 4,2 ml N-Ethylmorpholin und tropft unter Rühren bei 0° bis 4°C die Lösung von 6,6 g Dicyclohexylcarbodiimid (DCC) in 30 ml Methylenchlorid zu. Nach 5 Stunden filtriert man, engt das Filtrat zur Trockene ein, nimmt den Rückstand in Essigester auf und wäscht unterhalb 5°C mit 10-prozentiger $KHSO_4/K_2SO_4$ (1 : 2),

1M Natriumhydrogencarbonat und Wasser, trocknet über Natriumsulfat und destilliert das Lösungsmittel ab. Harz, leicht löslich in allen organischen Lösungsmitteln. Ausbeute: 15,2 g. Analog verfährt man mit dem Isomerengemisch "B", das auf dieser Stufe mittels präparativer HPLC an Silicagel im Laufmittel Chloroform/Cyclohexan (9:1) aufgetrennt werden kann.

f.) L-Alanyl-L-dekahydroisochinolin-3-carbonsäure-tert.-butylester-tosylat (H-Ala-Dic-OBu$^t$•TosOH)

8,5 g der Z-Verbindung werden analog Beispiel 10d katalytisch hydriert. Der nach dem Abdestillieren des Lösungsmittels zunächst harzige Rückstand kristallisiert nach einiger Zeit oder beim Verreiben mit Essigester und wird zur Analyse aus Essigester umkristallisiert. Schmp.: 151-153° (Zers.), $[\alpha]_D$ = -28,8° (c = 1, in Methanol). Elementaranalyse korrekt. Analog wird mit dem Isomerengemisch "B" verfahren. Man erhält ein nichtkristallisierendes Harz; korrekte Elementaranalyse.

Analog H-Ala-Dic-OBu$^t$ werden Alanyl-oktahydroindol-2-2-carbonsäure-t-butylester (H-Ala-Oic-OBu$^t$) und die folgenden Verbindungen dargestellt und bevorzugt als Tosylate isoliert

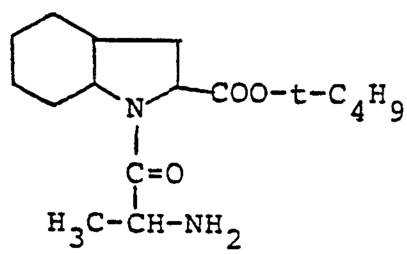

(NMR-Daten auf Base bezogen): 4,9 t (1H); 4,5-3,0 m (2H); 1,4 s (9H); 1,2 d (3H); 2,0-1,0 m (11H), (H-Ala-Oic-OBu$^t$)

Die Ausgangsaminocarbonsäure Oktahydroindol-2-carbonsäure wird wie folgt hergestellt:

45 g Indol-2-carbonsäure werden in 500 ml 4-prozNatronlauge gelöst. Man setzt 20 g Raney-Nickel zu und hydriert 24 Stunden bei 40°C. Man filtriert, säuert mit konz. HCl an, saugt von Ungelöstem ab und extrahiert die wässrige Phase mehrfach mit Butanol. Die organische Phase wird eingeengt und chromatographiert.
($CHCl_3$/Methanol/Eisessig 50:20:5).
Ausbeute: 22 g
Schmp.: 260°C.

Analog werden folgende Verbindungen hergestellt. Die Verbindungen zeigen die folgenden NMR-Signale:

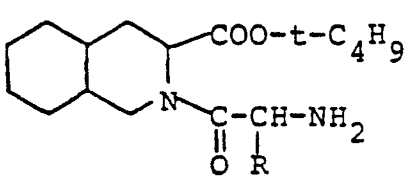

5,1-4,3 m (2H); 3,9-3,0 m (2H); 1,4 s (9H); 2,0-1,0m (12H)

für R : H

3,9-3,0 m (4H)

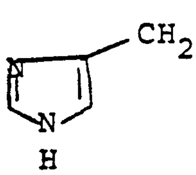

13,1 s(1H); 7,5 s (1H); 6,8 s (1H); 2,8 m (2H)

| | |
|---|---|
| 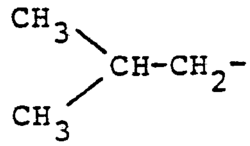 | 1,0-2,0 m (15H); 0,9 d (6H) |
| $-CH_2F$ | 5,1-4,3 m (4H) |
| 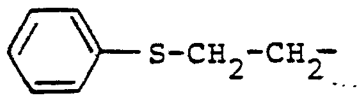 | 7,2 s (5H); 2,7-2,0 m (4H) |
| 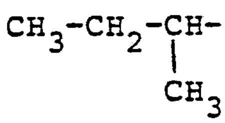 | 1,0-2,0 m (21H) |
| 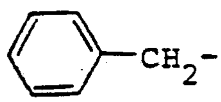 | 7,1 s (5H); 2,7 d (2H) |
| 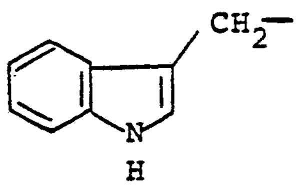 | 7,8-6,4 m (5H); 2,8 m (2H) |
| $HO-CH_2-$ | 3,7 d (2H) |
| 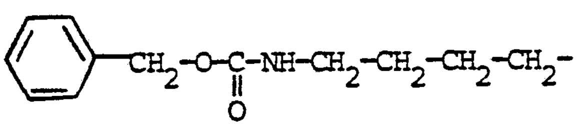 | 7,1 s (5H); 5,0 s (2H); 2,4 m (2H); 2,0-1,0 m (18H) |
| 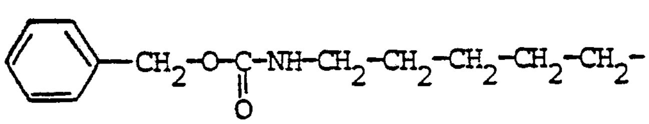 | 7,1 s (5H); 5,0 s (2H); 2,4 m (2H); 2,0-1,0 m (20H) |
| 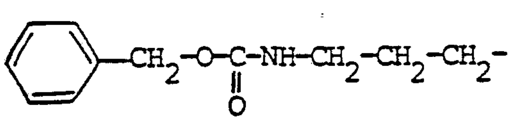 | 7,1 s(5H); 5,0s (2H); 2,4 m (2H); 2,0-1,0 m (16H) |

| | |
|---|---|
| 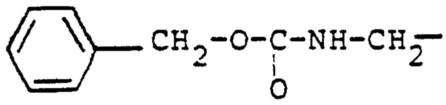 | 7,1 s (5H); 5,0 s (2H); 2,3 m (2H) |
| 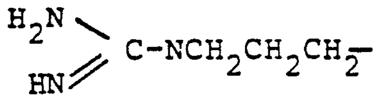 | 8,3-7,6 m (2H); 2,9-1,0 m (18H) |
| 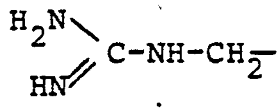 | 8,3-7,6 m (2H); 2,9-2,5 m (2H) |
| 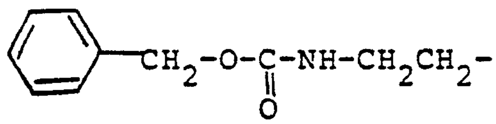 | 7,1 s (5H); 5,0 s (2H); 2,4 m (2H); 2,0-1,0 m(14H) |
| 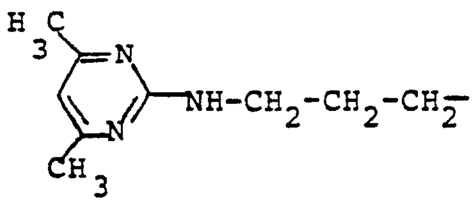 | 7,4 s(1H); 2,5 m (2H); 2,3 s (6H); 2,0-1,0 m (16H) |
| 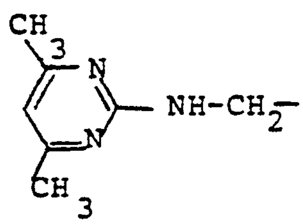 | 7,4 s(1H); 2,5 m (2H); 2,3 s (6H) |
| 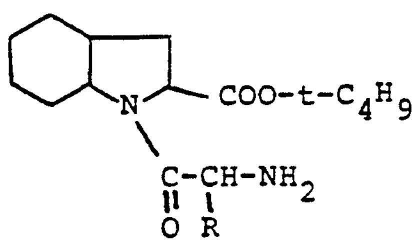 | 4,9 m (1H); 4,5-3,0 m (2H); 1,4 s (9H); 2,0-1,0 m (11H) |
| für R : H | 3,9-3,0 m (4H) |
| 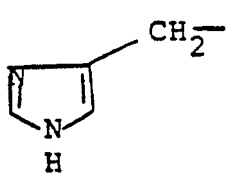 | 13,1 s (1H); 7,5 s (1H); 6,8 s (1H); 2,8 m (2H) |

| | |
|---|---|
| 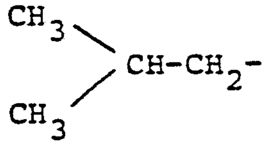 | 1,0-2,0 m (14H); 0,9 d (6H) |
| $-CH_2F$ | 5,1-4,3 m (4H) |
| 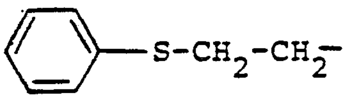 | 7,2-7,0 m (5H); 2,7-2,0 m(4H) |
| 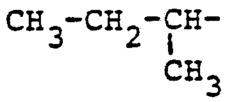 | 1,0-2,0 m (20H) |
| 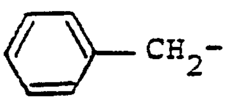 | 7,1 s (5H); 2,7 d (2H) |
| 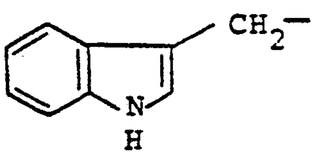 | 7,8-6,4 m (5H); 2,8 m (2H) |
| $HO-CH_2-$ | 3,7 d (2H) |
| 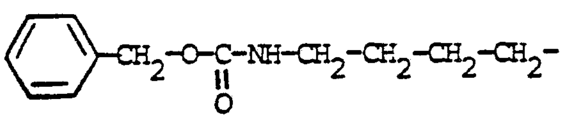 | 7,1 s (5H); 5,0 s (2H); 2,4 m (2H); 2,0-1,0 m (17H) |
| 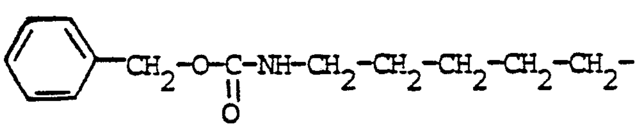 | 7,1 s (5H); 5,0 s (2H); 2,4 m (2H); 2,0-1,0 m (19H) |
| 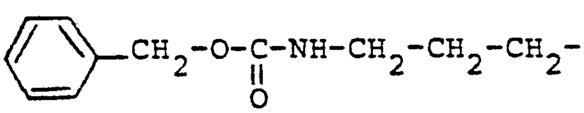 | 7,1 s (5H); 5,0 s (2H); 2,4 m (2H); 2,0-1,0 m (15H) |

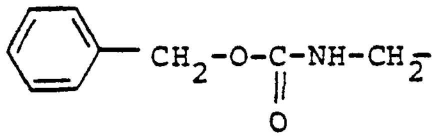

7,1 s (5H); 5,0 s (2H); 2,3 m (2H)

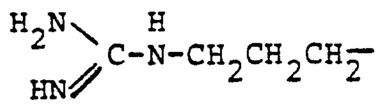

8,3-7,6 m (2H); 2,9-1,0 m (17H)

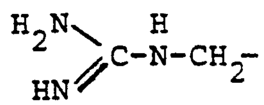

8,3-7,6 m (2H); 2,9-2,5 m (2H)

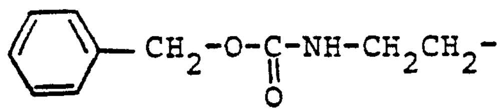

7,1 s (5H); 5,0 s (2H); 2,4 m (2H); 2,0-1,0 m (13H)

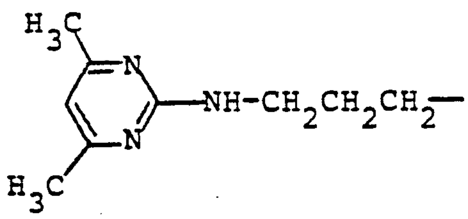

7,4 s(1H); 2,5 m(2H); 2,3 s (6H); 2,0-1,0 m (15H)

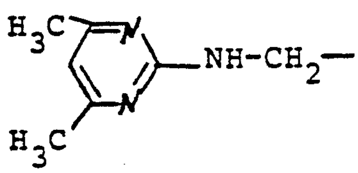

7,4 s(1H); 2,5 m (2H); 2,3 s (6H)

g. N-(1-Carboxy-3-phenylpropyl)-L-alanyl-L-dekahydroisochinolin-3-carbonsäure-tert.-butylester

305 mg Alanyl-dekahydroisochinolin-3-carbonsäure-t-butyl ester (Ala-Dic-OBut) und 445 mg 4-Phenyl-2-oxo-buttersäure werden in 4 ml Methanol gelöst und mit wässriger 1n NaOH auf pH 7,5 gebracht. Man fügt 200 mg Natriumcyanoborhydrid hinzu und läßt 36 Stunden reagieren. Man engt zur Trockne ein und gewinnt die Substanz durch Säulenchromatographie an Kieselgel (Laufmittelsystem: Chloroform/Methanol/Wasser/Eisessig 20 + 15 + 2 + 1).
Ausbeute: 376 mg; Schmp.: ab 123° Zers.

h. N-(1-Carboxy-3-phenylpropyl)-L-alanyl-L-dekahydroisochinolin-3-carbonsäure

350 mg N-(1-Carboxy-3-phenylpropyl)-Ala-Dic-OBut werden in 3 ml wasserfreier Trifluoressigsäure gelöst und 30 Minuten bei Raumtemperatur belassen. Man engt im Vakuum ein und verreibt das hinterbleibende Öl mit kaltem Diisopropyläther und Petroläther. Ausbeute: 226 mg amorphe Substanz
NMR: 7,10 (s); 3,0-3,9 (m, breit);
1,23 und 1,15 (d); 2,0-1,0 (m, breit)

Beispiel 11

N-(1-Carbethoxy-3-phenyl-propyl)-L-alanyl-L-dekahydroisochinolin-3-carbonsäure

Man löst 4,83 g H-Ala-Dic-$OBu^t$ • TosOH in 40 ml absol. Ethanol, stellt mit KOH in Ethanol auf pH 7 und gibt 6,2 g 2-Oxo-4-phenylbuttersäure-ethylester zu und rührt in Anwesenheit von 9 g pulverisiertem Molekularsieb 4 Å unter langsamer Zugabe von einer Lösung von 2 g Natriumcyanoborhydrid in 15 ml absol. Ethanol. Nach etwa 20 h wird filtriert und das Lösungsmittel im Vakuum abdestilliert. Der Rückstand wird zwischen Essigester und Wasser verteilt, die Essigesterphase abgetrennt und im Vakuum zur Trockene gebracht. Der Rückstand wird an Silicagel im Laufmittel Essigester/Cyclohexan (1:2 bis 1:4) chromatographiert.

Das Produkt wird 30 min. in 20 ml Trifluoressigsäure gelöst. Man destilliert die Trifluoressigsäure im Vakuum ab, destilliert mit Toluol nach und chromatographiert über Silicagel in Methylenchlorid/Methanol (10:1). Ausbeute: 1,8 g.

## Beispiel 12

N-(1-Carboxy-3-phenyl-3-thiapropyl)-alanyl-L-dekahydroisochinolin-3-carbonsäure

a.) 2-(2-Oxo-propionyl)-L-dekahydroisochinolin-3-carbonsäure-tert.-butylester

In eine auf -50°C gekühlte Lösung von 700 mg Dic-OBut und 630 mg Dicyclohexylcarbondiimid in 15 ml wasserfreiem Chloroform wird eine gekühlte und frisch destillierte Lösung von 270 mg Brenztraubensäure in 5 ml Chloroform rasch eingerührt. Man beläßt 16 Stunden im Tiefkühlschrank (-20°C) und filtriert den ausgefallenen DC-Harnstoff ab. Die Lösung wird mit $KHSO_4$-und anschließend mit $KHCO_3$-Lösung gewaschen, über $Na_2SO_4$ getrocknet und im Vakuum eingeengt. Die Verbindung (Öl) enthält etwas Dicyclohexylharnstoff; sie wurde zur Analyse über Kieselgel im System $CHCl_3/CH_3OH$ 15:1 chromatographiert.

Analog der Kondensation Brenztraubensäure mit Dic-OBut werden folgende Kondensationsprodukte mit der entsprechenden α-Ketocarbonsäure dargestellt.

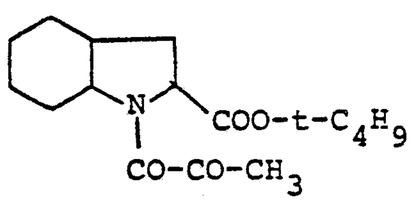

4,9 m (1H); 4,2-3,5 m (1H); 3,0-1,2 m (11H); 2,4 s (3H); 1,4 s (9H)

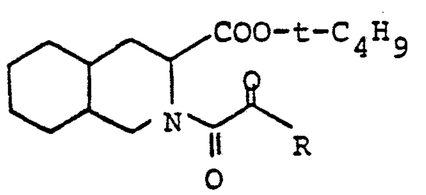

bei nachstehenen Verbindungen werden einheitlich folgende Signale für das Grundgerüst beobachtet: 5,1-4,8m (1H); 3,9-3,0m (2H); 1,4 s (9H); 2,0-1,0m (12H)

| | |
|---|---|
| 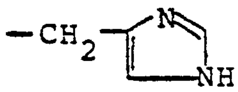 | 13,1 s (1H); 7,5 s (1H); 6,8s (1H); 3,9-3,0 m (4H) |
| $-CH_2-CH(CH_3)_2$ | 2,4-1,0 m (21H) |
| 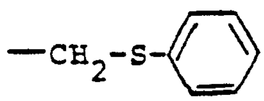 | 7,2-7,0 m (5H); 2,5 m (2H) |
| $-CH_2F$ | 4,4 s (2H) |
| 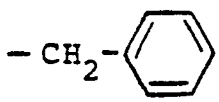 | 7,15 (5H); 3,0 s (2H) |
| 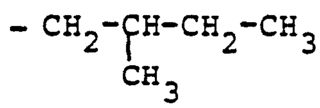 | 2,4-1,0 m (23H) |
| 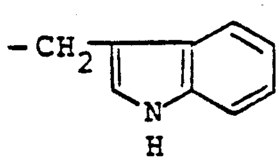 | 7,8-6,4 m (5H); 3,9-2,9 m(4H) |

b.) N-(1-Carboxy-3-phenyl-3-thiapropyl)-alanyl-L-dekahydroisochinolin-3-carbonsäure-tert.-butylester

1,52 g Pyruvyl-Dic-OBut und 394 mg S-Phenyl-cystein werden in 5 ml Methanol gelöst und mit 1n NaOH (wässrig) auf pH 7,0 gebracht. Man fügt 400 mg Natriumcyanoborhydrid zu und beläßt 24 Stunden bei Raumtemperatur. Man engt im Vakuum ein, nimmt in Chloroform auf, trocknet über Natriumsulfat und engt ein. Das Produkt wird durch Kieselgelchromatographie (System $CHCl_3/CH_3OH/HAcO/H_2O$ 50/20/5/1) rein erhalten.
NMR: 7,3 m (5H); 5,1-4,8 m (1H); 3,9-3,0 m (4H); 2,5 m (2H)

c.) N-(1-Carboxy-3-phenyl-3-thiapropyl)-alanyl-L-dekahydroisochinolin-3-carbonsäure

1 g Butylester aus Beispiel 12b wird in 5 ml Trifluoressigsäure gelöst. Man engt nach 30 Minuten im Vakuum ein, digeriert den Rückstand mit Diisopropyläther und trocknet über Kaliumhydroxyd.
Ausbeute: 0,95 g Trifluoracetat (hygroskopisch)
NMR: 7,3 m (5H); 5,2-4,8 m (1H); 3,9-3,0 m (4H); 2,5 m (2H); 1,25 m (3H)

Beispiel 13

N-(1-Carboxy-3-phenyl-propyl)-alanyl-L-dekahydroisochinolin-3-carbonsäure

880 mg N-(1-Carbethoxy-3-phenyl-propyl)-L-alanyl-L-dekahydroisochinolin-3-carbonsäure werden in 20 ml Dioxan/Wasser (9:1) gelöst und bei Raumtemperatur mit 4,5 ml 1n Natriumhydroxid-Lösung verseift. Nach einer Stunde wird die Lösung mit der äquivalenten Menge 1 n Salzsäure angesäuert und das Dioxan weitgehend abgedampft. Der Rückstand wird mehrfach mit Essigester extrahiert und die organische Phase nach Abziehen des Lösungsmittels über einen Kationenaustauscher (DOWEX 50) chromatographiert.
Ausbeute: 630 mg.

Beispiel 14

N-(1-Carboxy-5-aminopentyl)-L-alanyl-L-dekahydroisochinolin-3-carbonsäure

1,2 g N-(1-Carboxy-5-Boc-aminopentyl)-L-alanyl-L-dekahydroisochinolin-3-carbonsäure wurden mit 10 ml eiskalter Trifluoressigsäure übergossen und eine Stunde bei Raumtemperatur gerührt. Anschließend wird im Vakuum eingedampft. Der Rückstand wird in Wasser gelöst und einer Gefrier trocknung unterworfen.
Ausbeute: 0,90 g.

Beispiel 15

N-(1-Carbethoxy-3-amino-propyl)-L-alanyl-L-dekahydroisochinolin-3-carbonsäure

1,1 g N-(1-Carbethoxy-3-benzoxycarbonylamino-propyl)-L-alanyl-L-dekahydroisochinolin-3-carbonsäure werden in 150 ml Methanol gelöst und in Gegenwart von 100 mg Palladium - Aktivkohle (10 %ig) bei 40°C unter Normaldruck hydriert. Nach Beendigung der Reaktion wird vom Katalysator abfiltriert und das Lösungsmittel im Vakuum abgezogen.
Ausbeute: 0,79 g.
NMR: 5,1-4,8 m (1H); 3,9-2,5 m (6H); 2,0-1,0 m (14H); 1,2 d (3H).

Beispiel 16

N-(1-Carbethoxy-2-benzylsulfinylethyl)-L-alanyl-L-dekahydroisochinolin-3-carbonsäure

Zu einer unter Eiskühlung gerührten Mischung von 0,64 g Natriumperjodat und 20 ml Wasser werden 1,3 g N-(1-Carbethoxy-2-benzyl-thio-ethyl)-L-alanyl-L-dekahydroisochinolin-3-carbonsäure gelöst in 20 ml Methanol gegeben. Man läßt die Reaktionsmischung für 24 Stunden bei 0°C rühren und filtriert anschließend vom Natriumjodat ab. Das Filtrat wird mehrfach mit Methylenchlorid extrahiert. Nach Entfernen des Lösungsmittels erhält man 1,1 g des Sulfoxids. Im NMR-Spektrum werden folgende Signale beobachtet:
7,3 m (5H); 5,1-4,8 m (1H); 4,1 s (1H); 3,9-3,0 m (4H); 2,6 m (2H); 1,2 d (3H).

Beispiel 17

N-(1-Carbethoxy-3-phenylsulfonyl-propyl)-L-alanyl-L-dekahydroisochinolin-3-carbonsäure

260 mg N-(1-Carbethoxy-3-phenylthio-propyl)-L-alanyl-L-dekahydroisochinolin-3-carbonsäure und 20 mg Natriumwolframat Dihydrat werden in 50 ml Wasser gegeben und tropfenweise mit 1 ml Perhydrol (30 %ig) versetzt. Die Mischung wird für 30 min auf 80°C erwärmt und anschließend eine Stunden bei Raumtemperatur gerührt. Das überschüssige Peroxid wird sodann mit Palladium auf Bariumsulfat zerstört und nach Beendigung der Sauerstoffentwicklung wird die Lösung vom Palladium Katalysator abfiltriert und eingeengt. Das Rohprodukt wird durch Ionenaustauschchromatographie über DOWEX 50 $H^+$-Form gereinigt.
Ausbeute: 220 mg
NMR: 7,7 m (5H); 5,1-4,8 m (1H); 3,9-3,0 m (4H); 2,8 m (2H) 1,5 m (2H); 1,2 d (3H)

Beispiel 18

N-(2-Amino-1-carboxypropyl)-L-alanyl-L-dekahydroisochinolin-3-carbonsäure

2,2 g $N^{\beta}$-Boc-$\alpha,\beta$-Diaminobuttersäure, hergestellt analog Coll. Czech chem. Commun. 31, 2955 (1966) werden mit 2,4 g Pyruvyl-Dic-OBut, hergestellt nach Beispiel 12a, in der Beispiel 12b beschriebenen Weise umgesetzt. Die Boc-Schutzgruppe wird anschließend nach der in Beispiel 14 beschriebenen Weise abgespalten.
Ausbeute: 2,6 g.
NMR: 5,1-4,8 m (1H); 3,9-3,0 m (5H); 2,0-1,0 m (15H) 1,2 d (3H).

Beispiel 19

L-N-(1-Carbethoxy-5-aminopentyl)-L-alanyl-L-dekahydroisochinolin-3-carbonsäure

a) 35 g $N^{\epsilon}$-Benzyloxycarbonyl-L-lysin-ethylester-hydrochlorid und 30,6 g $\alpha$-Brompropionsäure werden in einer Mischung aus 350 ml Dioxan, 50 ml Ethanol und 75 ml 4N NaOH gelöst. Man rührt über Nacht bei Raumtemperatur unter Konstanthalten des pH bei 8.8-9 am Autotitrator, bringt dann mit wenig HCl auf pH 7-8 und destilliert das Lösungsmittel im Vakuum ab. Der Rückstand wird in möglichst wenig Wasser gelöst und die Lösung mit Salzsäure auf pH 5-6 gebracht. Man kühlt mit Eis und filtriert den Niederschlag nach kurzem Stehen ab, wäscht mit wenig eiskaltem Wasser und Ether und trocknet im Vakuum. Man kann die wässrige Lösung auch über schwach basischem Ionenaustauscher in das Zwitterion überführen und reinigen. Das Filtrat wird dann einfach lyophilisiert. Ausbeute: 12,9 g (52 %).

b) Man löst die Verbindung in 100 ml Dimethylformamid und versetzt nacheinander bei Raumtemperatur mit 23 g H-Dic-OBzl; TosOH (in üblicher Weise aus der Aminosäure durch Kochen in Benzylalkohol-Toluol-Toluolsulfonsäure unter Rückfluß und Wasserabscheidung hergestellt), 6,5 ml N-Ethylmorpholin, 6,8 g 1-Hydroxybenzotriazol und 11 g Dicyclohexylcarbodiimid. Nach 4 h Rühren wird vom Harnstoff abfiltriert, das Lösungsmittel im Vakuum abdestilliert und der Rückstand in Cyclohexan-Essigester (4:1) an Silicagel chromatographiert. Dabei tritt Auftrennung der Diastereoisomeren ein. Die einheitlichen Fraktionen, welche die Titelverbindung enthalten, werden gesammtelt, das Lösungsmittel wird im Vakuum abdestilliert.

c) Der Rückstand wird in Methanol aufgenommen, an Pd/Kohle katalytisch hydriert und ggfs. nach Abfiltrieren des Katalysators durch Einstellen des pH auf 3 in Hydrochlorid überführt. Abdestillieren des Lösungsmittels und Trocknen des Rückstandes im Vakuum.

Beispiel 20

L-N-(1-Carbethoxy-3-phenylpropyl)-alanyl-L-oktahydroindolin-2-carbonsäure

a) 55 g 2-Brom-4-phenyl-n-buttersäureäthylester, 14,5 g L-Alanin-t-butylester und 45 ml Triethylamin werden 24 h in Tetrahydrofuran aufbewahrt. Man destilliert das Lösungsmittel im Vakuum ab, verteilt den Rückstand zwischen Wasser und Essigester, trennt die Essigesterschicht ab, trocknet über Natriumsulfat und destilliert das Lösungsmittel im Vakuum ab. Der Rückstand wird in Cyclohexan-Essigester (4:1) über Silicagel chromatographiert, wobei neben der Reinigung auch Diastereoisomerentrennung eintritt. Nach Einengen der entsprechenden Fraktionen wird der t-Butylester durch 20 min. Aufbewahren der Verbindung in Trifluoressigsäure abgespalten. Man destilliert die Trifluoressigsäure im Vakuum ab und destilliert mit Toluol im Vakuum nach.

b) Nun setzt man analog Beispiel 19b in Dimethylformamid mit 11 g Oktahydroindol-2-carbonsäure-benzylester-tosylat, 3,2 ml N-Ethylmorpholin, 3,3 g 1-Hydroxybenzotriazol und 5,5 g Dicyclohexylcarbodiimid um, arbeitet wie dort beschrieben auf und trennt die Diastereomeren durch Chromatographie an Silicagel im System Cyclohexan:Essigester (4:1) auf. Von der chromatographisch einheitlichen Verbindung wird der Benzylester durch katalytische Hydrierung abgespalten.

Beispiel 21

L-N-[1-Carbethoxy-4-(4,6-dimethyl-pyrimidyl-2-amino)-butyl]-L-alanyl-L-dekahydroisochinolin-3-carbonsäure

a) 15,3 g α-Brompropionsäure und 41,1 g H-Dic-OBut • TosOH werden in 300 ml Tetrahydrofuran in Anwesenheit von 12,8 ml N-Ethylmorpholin und 1,35 g 1-Hydroxybenzotriazol mittels 22 g Dicyclohexylcarbodiimid kondensiert. Nach 4 h wird der Harnstoff abfiltriert und das Lösungsmittel im Vakuum abdestilliert. Man nimmt den Rückstand in Essigester auf, wäscht die Lösung mit Natriumhydrogencarbonat-und Citronensäurelösung und mit Wasser, trocknet die Essigesterlösung über Natriumsulfat und destilliert das Lösungsmittel im Vakuum ab.

b) 3,75 g α-Brompropionyl-Dic-OBut werden in 20 ml Tetrahydrofuran mit 2,5 g $N^{\delta}$-(4,6-Dimethylpyrimidyl-2-L-ornithin-ethylester unter Zusatz von 1 ml Diisopropylethylamin bei Raumtemperatur umgesetzt. Nach 24 h wird das Lösungsmittel im Vakuum abdestilliert, der Rückstand in Essigester aufgenommen, die Lösung mit Wasser gewaschen, über Natriumsulfat getrocknet und im Vakuum eingeengt. Der Rückstand wird analog Beispiel (20b) unter Diastereoisomerentrennung chromatographiert. Der t-Butylester wird durch Lösen der Verbindung in Dioxan/HCl gespalten. Nach 10-15 min destilliert man das Lösungsmittel ab und trocknet den Rückstand scharf im Vakuum über KOH. Die Verbindung liegt als Hydrochlorid vor.

Beispiel 22

N-(1-S-Carbethoxy -3-phenyl-propyl)-S-alanyl-cis-octahydroindol-2-S-carbonsäure

a) N-(1-S-Carbethoxy -3-phenyl-propyl)-alanin-benzylester

Durch Extraktion mit Essigester wird aus einer wässr. Sodalösung von 70 g Alanin-benzylester-Toluolsulfonat die freie Base gewonnen. Man versetzt die über wasserfreiem Natriumsulfat getrocknete organische Phase mit 200 ml Dimethylacetamid (DMA) und engt bei RT im Wasserstrahlvakuum ein. Nach Zugabe von 90 g α-Brom-phenyl-buttersäureethylester und 40 ml N-Äthylmorpholin wird die Lösung 4 Tage bei RT belassen. Die Reaktion wird dünnschichtchromatographisch auf Kieselgelplatten (System: Cyclohexan/Essigester 2:1) verfolgt. Man engt im Vakuum bis fast zur Trockne ein, stellt mit 2n methanolischer Salzsäure auf pH 3 und setzt 10 ml Wasser zu. Man extrahiert überschüssigen Bromester und lipophile Produkte in Petroläther, engt die methanolische Phase ein, fügt 3 % Sodalösung (250 ml) zu und extrahiert die Reaktionsprodukte in ein Essigester/Äther-Gemisch (1:1). Nach Trocknung über festem Natriumsulfat wird eingeengt und mit Hilfe einer Kieselgelchromatographie (System Cyclohexan/Essigester 4:1) eine Reinigung und Diastereomerentrennung vorgenommen. Die S,S-Verbindung weist einen niedrigeren $R_f$-Wert auf und fällt in überwiegender Menge an (48%). Die ölige Substanz wird sofort weiter verarbeitet.

b) N-(1-S-Carbethoxy -3-phenyl-propyl)-5-alanin

37 g des Benzylesters werden in 250 ml Ethanol aufgenommen und mit 1 g Pd/Kohle (10 % Pd) bei Normaldruck in 2 Stunden hydrogenolytisch entbenzyliert. Man filtriert den Katalysator ab, engt im Vakuum fast zur Trockene ein und fällt durch Zusatz von Petroläther.
Ausbeute: 33 g; amorph;
NMR: 1,18 (d, 3H); 1,18 (t, 3H); 1,5-2,1 (m, 2H); 2,4-2,8 (m, 2H); 3,0-3,4 (2 m, je 1 H); 4,07 (q, 2H); 4,3-5,5 (b, 2H); 7,17 (s, 5H). DMSO-$d_6$

c) N-(1-S-Carbethoxy -3-phenyl-propyl)-S-alanyl-cis-octahydroindol-2-R,S-carbonsäurebenzylester

5 g N-(1-S-Carbethoxy -3-phenyl-propyl)-S-alanin werden in 20 ml DMF gelöst. Man fügt 2,45 g 1-Hydroxybenzotriazol, 5,1 g cis-Octahydroindol-R,S-carbonsäurebenzylester-hydrochlorid, 2,5 ml N-Ethylmorpholin sowie 4 g Dicyclohexylcarbodiimid zu und rührt 3 Stunden bei Raumtemperatur. Man verdünnt mit 30 ml Essigester und saugt vom Harnstoff ab. Nach Einengen im Vakuum wird in 100 ml Äther

aufgenommen und mit wässr. Bicarbonatlösung 2 x extrahiert. Die über Natriumsulfat getrocknete und zur Trockene eingeengte organische Phase wird an Kieselgel chromatographiert. Man eluiert mit Essigester/Petroläther 4:3 und erhält 2 Fraktionen, aus denen durch Einengen im Vakuum farblose Öle erhalten werden.

| Elementaranalyse: | C | H | N | |
|---|---|---|---|---|
| ber. für $C_{31}H_{40}N_2O_5$ | 71.5 | 7.7 | 5.4 | Ausb. |
| Frakt. 1 | 71.3 | 7.8 | 5.3 | 3.3 g |
| Frakt. 2 | 71.6 | 7.6 | 5.6 | 3.9 g |

d) N-(1-S-Carbethoxy -3-phenyl-propyl)-S-alanyl-cis-octahydroindol-2-S-carbonsäure

3 g des Benzylesters (Fraktion 2) werden in Ethanol gelöst und mit 200 mg 10 % Pd/Kohle bei Normaldruck hydrogenolytisch entbenzyliert. Nach beendeter Wasserstoffaufnahme (etwa 1 Stunde) wird vom Katalsator abfiltriert und die Lösung im Vakuum eingeengt. Man versetzt mit etwas Pentan, erwärmt auf etwa 45°C und legt ein kräftiges Vakuum an. Es bildet sich ein amorpher, fester Schaum. Ausb.: 2,4 g.
NMR ($CDCl_3$): 1,20 (d, 3H); 1,23 (t, 3H); über Multiplett 1,0-2,9 (11H); 3,0-4,5 (m, 3H); 4,12 (q, 2H); 4,65 (b, 2H); 7,13 (s, 5H).

Beispiel 23

N-(1-Carbethoxy -3-phenyl-propyl)-alanyl-L-octahydroindol-2-carbonsäurebenzylester

a)N-tert.Butyloxycarbonyl-alanyl, octahydroindol-2-carbonsäure-benzylester (Boc-Ala-Oic-OBzl)

19 g Boc-Ala-OH werden in 100 ml DMF gelöst und mit 13 ml N-Ethylmorpholin, 13,5 g HOBt sowie 29,6 g Octahydroindol-2-carbonsäurebenzylester-hydrochlorid versetzt. Man kühlt im Eisbad, fügt 21 g Dicyclohexylcarbodiimid zu und läßt 15 Stunden bei Raumtemperatur rühren. Der ausgefallene Harnstoff wird abgesaugt, das Filtrat im Vakuum eingeengt und in Essigester aufgenommen. Man extrahiert je 3 x mit wässriger $KHSO_4$-, $KHCO_3$-und gesättigter NaCl-Lösung und engt die organische Phase im Vakuum ein. Ausbeute: 38.5 g.
NMR: 1,26 (d, 3H); 1,40 (s, 9H); 1,1-2,4 (m, 12H); 3,2-3,9 (m, 2H); 5,28 (s, 2H); 7,31 (s, 5H).

b) Alanyl-octahydroindol-2-carbonsäurebenzylester-trifluoracetat

21,5 g Boc-Ala-Oic-OBzl werden in 50 ml Trifluoressigsäure gelöst. Man engt im Vakuum ein, digeriert den Rückstand mehrfach mit Diisopropyläther und trocknet im Vakuum. Ausbeute: 21 g. Im NMR fehlt das Protonensignal für die tert. Butylgruppe vollständig.

c) N-(1-Carbethoxy -3-phenyl-propyl)-alanyl-octahydroidol-2-carbonsäurebenzylester

11,1 g Ala-Oic-OBzl.TFA, 7 g α-Brom-phenylbuttersäureethylester und 3,3 ml N-Ethylmorpholin werden in 20 ml Dimethylacetamid 4 Tage bei Raumtemperatur gerührt. Man engt im Vakuum weitgehend ein, löst den Rückstand in Methanol, stellt mit wässr. 2n HCl auf pH 2 und extrahiert lipophile Verbindungen mit Petroläther. Die methanolische Phase wird eingeengt, mit 5 % Sodalösung versetzt und das Reaktionsprodukt in Essigester extrahiert. Chromatographie über Kieselgel (System Essigester/Petroläther 5:3) liefert die Titelverbindung als farbloses Oel.

Die nachstehend aufgeführten Beispiele sind nach einer der folgenden Methoden A - H hergestellt, wobei die bezeichneten Methoden wie folgt charakterisiert werden:

Methode A: Reduktive Aminierung eines Kondensationsproduktes aus Tic oder Dic-Ot$C_4H_9$-Ester oder Benzylester bzw. Oic-Ot$C_4H_9$-Ester oder Benzylester und einer entsprechenden $\alpha$-Ketocarbonsäure, dargestellt nach Beispiel (6a oder 12a), mit einem entsprechenden Aminosäurederivat nach Beispiel (6b), Abspaltung der Schutzgruppen nach Beispiel (6c bzw. 12 c oder 22d) und gegebenenfalls Verseifung nach Beispiel (13).

Methode B: Reduktive Aminierung eines Dipeptids-t-Butylesters, dargestellt nach Beispiel (1e bzw. 10e,f), mit einem entsprechenden $\alpha$-Ketocarbonsäurederivat nach Beispiel (1f, 10g, 11) und ggfs. Abspaltung der Schutzgruppen nach Beispiel (10h bzw. 11) und/oder ggfs.nach Beispiel (13).

Methode C: Reduktive Aminierung nach Methode A und mit einer $\omega$-geschützten bifunktionellen Aminosäure, Abspaltung einer Schutzgruppe aus dem eingesetzten Aminosäure-derivat nach Beispiel 3 bzw. 14.

Methode D: Oxidation der zugrunde liegenden Schwefel-Verbindung zum Sulfoxid nach Beispiel 7 bzw. 16.

Methode E: Oxidation der Zugrunde liegenden Schwefel-Verbindung zum Sulfon nach Beispiel (8) bzw. (17).

Methode F: Kondensation einer entsprechenden N-Alkylierten $\alpha$-Aminosäure (dargestellt nach Beispiel 19a) mit einem Aminosäureester (z.B. Dic-OBu$^t$ oder Dic-OBzl bzw. Oic-OBu$^t$ oder Oic-OBzl) nach Beispiel 19 b oder 22c und Abspaltung der Schutzgruppe nach Beispiel 19 c oder 22 d.

Methode G: Umsetzung eines N-acylierten Aminosäureesters, der im Acylteil in $\alpha$-Position zur Amidfunktion eine nucleofuge Gruppe trägt (wie z.B. Halogen, Arylsulfonyl oder Alkylsulfonyl), dargestellt nach Beispiel 21a, mit einer entsprechenden Aminosäure oder einem entsprechenden Aminosäureester nach Beispiel 21b und Abspaltung der Schutzgruppen.

Method H: Umsetzung eines Dipeptid-benzylesters, dargestellt nach Beispiel 23a, 23b, mit einer entsprechend substituierten $\alpha$-Arylsulfonyl-oder $\alpha$-Alkylsulfonyl-oder $\alpha$-Halogen-carbonsäure bzw. Carbonsäureester, dargestellt nach Beispiel 23c und Spaltung des Benzylesters zu Verbindungen der Formel I nach der in Beispiel 22d angegebenen Weise.

Falls nicht anders angegeben, gelten für die nachstehenden Verbindungen folgende NMR-Daten ( $\delta$ - Werte in ppm; TMS als Standard):

A = Benzolring:
für n = 1 7.3s (4H), 5.1-4.3m (3H) 3.9-3.0m (4H)
n = 0 7.2-6.5m (4H); 4.9t (1H) 3.6 - 3.0m (4H)
A = Cyclohexanring:
für n = 1 5.1-4.3m (2H); 3.9-3.0m (3H); 2.0-1.0m (12H)
n = 0 4.9m (1H); 4.5-3.0m (3H), 2.0-1.0m (11H0

Im Falle der Ethylester treten noch folgende Signale auf:
4.2 q 7 Hz (2H)
1.2 t 7 Hz (3H)

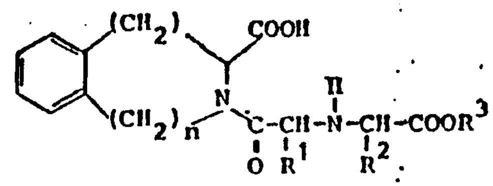

| Nr. | n | $R^1$ | $R^2$ | $R^3$ | Methode | NMR |
|---|---|---|---|---|---|---|
| 1 | 1 | $CH_3$ | $CH_2-\overset{H}{N}-CHO$ | $C_2H_5$ | A | 8.3 2s (1H); 2.9m (2H); 1.2d (3H) |
| 2 | 1 | $CH_3$ | $CH_2-\overset{H}{N}-COCH_3$ | $C_2H_5$ | A | 2.9m (2H); 2.1s (3H); 1.2d (3H) |
| 3 | 1 | $CH_3$ | 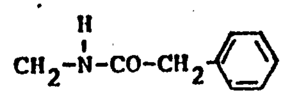 | $C_2H_5$ | A | 3.9 - 3.0m (6H); 2.9m (2H); 1.2d (3H) |
| 4 | 1 | H | 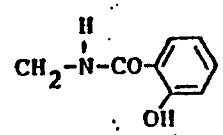 | $C_2H_5$ | A | ~~7.9~~ 7.2 - ~~7.0~~ 6.8m (8H); 3.8 - 3.0m (5H); 2.9m (2H) |
| 5 | 1 | H | 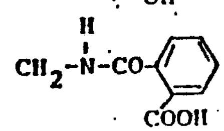 | $C_2H_5$ | A | 7.9 - 7.0m (8H); 3.9 - 3.0m (5H); 2.9m (2H) |
| 6 | 1 | $CH_3$ | 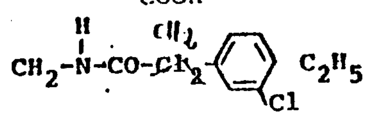 | $C_2H_5$ | A | 7.65 - 7.1m (8H); 3.9 - 3.0m (6H) 2.9m (2H); 1.2d (3H) |
| 7 | 1 | $CH_3$ | 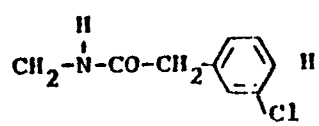 | H | A | 7.65 - 7.1m (8H); 3.9 - 3.0m (6H) 2.9m (2H); 1.2d (3H) |

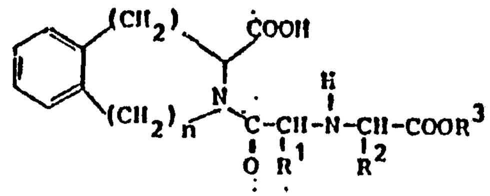

| | n | $R^1$ | $R^2$ | $R^3$ | Methode, | NMR |
|---|---|---|---|---|---|---|
| 8 | 1 | $CH_3$ | 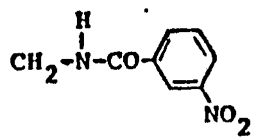 | $C_2H_5$ | A | 8.2 - 7.1m (8H); 2.9m (2H); 1.2d (3H) |
| 9 | 1 | $CH_3$ | 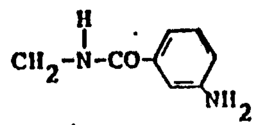 | $C_2H_5$ | A | 7.2 - 6.8m(8H); 2.9m (2H); 1.2d (3H) |
| 10 | 1 | 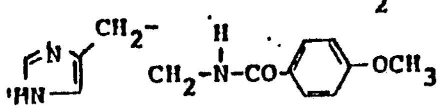 | | $C_2H_5$ | A | 13.0s (1H); 7.5 - 6.8m (10H); 3.9s (3H) 2.9 - 2.6m (4H) |
| 11 | 1 | $CH_3$ | 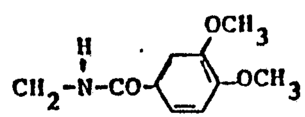 | $C_2H_5$ | A | 7.5 - 6.9m (7H); 3.9s (6H); 2.9m (2H); 1.2d (3H) |
| 12 | 1 | $CH_3$ | 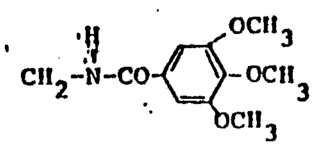 | $C_2H_5$ | A | 7.3 - 6.8m (6H); 3.9s (9H); 2.9m (2H) 1.2d (3H) |
| 13 | 1 | $CH_3$ | 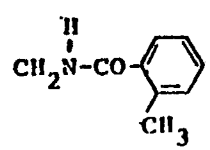 | $C_2H_5$ | A | 7.4 - 7.0m (8H); 2.9m (2H) 2.4s (3H); 1.2d (3H) |

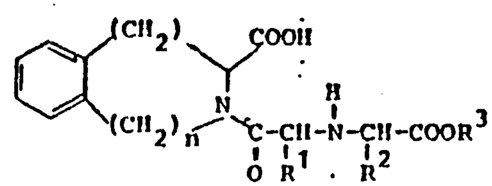

| | n | $R^1$ | $R^2$ | $R^3$ | Methode | NMR |
|---|---|---|---|---|---|---|
| 14 | 1 | $CH_3$ | 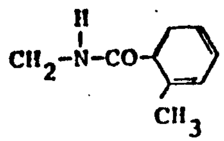 | H | A | 7.4 - 7.0m (8H); 2.9m (2H); 2.4s (3H) 1.2d (3H) |
| 15 | 1 | $CH_3$ | $CH_2-CH_2-N(CH_3)_2$ | $C_2H_5$ | A | 2.4m (2H); 2.2s (6H); 1.7m (2H); 1.2d (3H) |
| 16 | 1 | $CH_3$ | 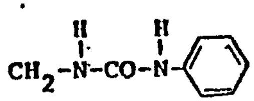 | $C_2H_5$ | A | 7.3 - 7.0m (9H); 2.9m (2H) 1.2d (3H) |
| 17 | 1 | $CH_3$ | 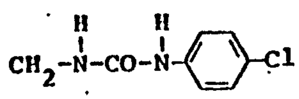 | $C_2H_5$ | A | 8.0 - 7.0m (8H); 2.9m (2H); 1.2d (3H) |
| 18 | 1 | $CH_3$ | 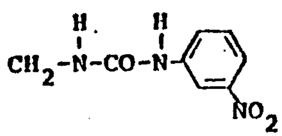 | $C_2H_5$ | A | 8.3 - 7.1m (8H); 2.9m (2H); 1.2d (3H) |
| 19 | 1 | $(CH_3)_2CH-CH_2$ | 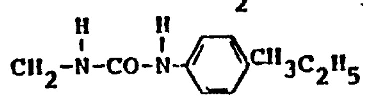 | $C_2H_5$ | A | 7.4 - 7.0m (8H); 2.9m (2H); 2.3s (3H) 1.0d (6H) |

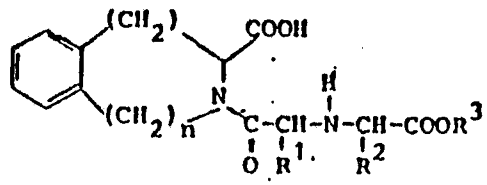

| | n | $R^1$ | $R^2$ | $R^3$ | Methode | NMR |
|---|---|---|---|---|---|---|
| 20 | 1 | $CH_3$ | 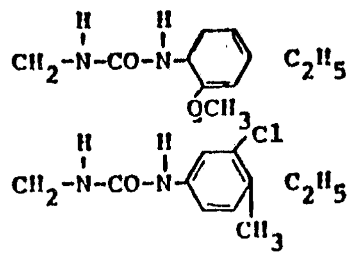 | $C_2H_5$ | A | 7.3 - 6.7m (8H); 3.8s (3H); 2.9m (2H); 1.2d (3H) |
| 21 | 1 | $CH_3$ | | $C_2H_5$ | A | 7.6 - 6.9m (7H); 2.9m(2H); 2.4s (3H); 1.2d (3H) |
| 22 | 1 | 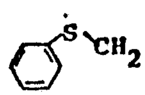 | $CH_2-\overset{H}{N}-CO-\overset{H}{N}-CH_3$ | $C_2H_5$ | A | 7.3 - 7.0m (9H); 3.0 - 2.6 m+s (7H) |
| 23 | 1 | 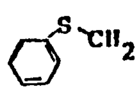 | $CH_2-\overset{H}{N}-CO-\overset{H}{N}-CH_3$ | H | A | 7.3 - 7.0m (9H); 3.0 - 2.6 m+s (7H) |
| 24 | 1 | $CH_2F$ | $CH_2-\overset{H}{N}-CO-\overset{H}{N}-C_4H_9$ | $C_2H_5$ | A | 5.1 - 4.3m (5H); 2.9m (2H); 1.0t (3H) |

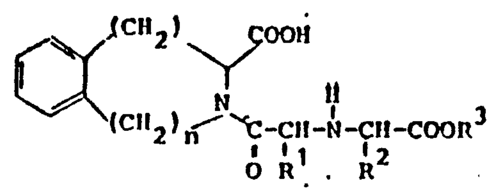

| | n | $R^1$ | $R^2$ | $R^3$ | Methode | NMR |
|---|---|---|---|---|---|---|
| 25 | 1 | $CH_3$ | 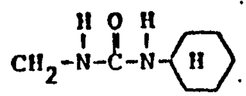 | $C_2H_5$ | A | 3.2 - 2.9m (3H); 2.4 - 1.0m (10H); 1.2d (3H) |
| 26 | 1 | $CH_3$ | 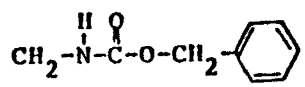 | $C_2H_5$ | A | 7.1s (5H): 5.0s (2H); 2.9m (2H); 1.2d (3H) |
| 27 | 1 | $CH_3$ | 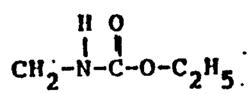 | $C_2H_5$ | A | 4.2q (4H); 2.9m (2H); 1.2 t+d (9H) |
| 28 | 1 | $CH_3$ | 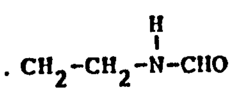 | $C_2H_5$ | A | 8.2 2s (1H); 2.9m (2H); 1.5m (2H); 1.2d (3H) |
| 29 | 1 | H | 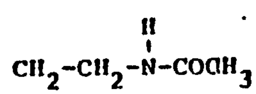 | $C_2H_5$ | A | 3.9 - 3.0m (5H); 2.9m (2H) 2.1s (3H); 1.5m (2H) |
| 30 | 1 | $CH_3$ | 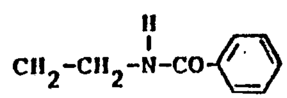 | $C_2H_5$ | A | 7.6 - 7.0m (9H); 2.9m (2H) 1.5m (2H); 1.2d (3H) |

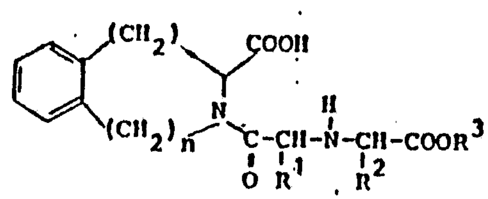

| | n | $R^1$ | $R^2$ | $R^3$ | Methode | NMR |
|---|---|---|---|---|---|---|
| 31 | 1 | $CH_3$ | 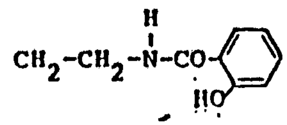 | $C_2H_5$ | A | 7.2 - 6.8m (8H); 2.9m (2H). 1.5m (2H); 1.2d (3H) |
| 32 | 1 | 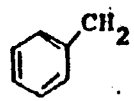 | 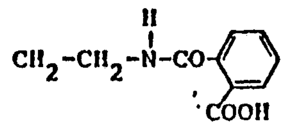 | $C_2H_5$ | A | 7.9 - 7.0m (13H); 2.9 - 2.7m (4H); 1.5m (2H) |
| 33 | 1 | $CH_3$ | 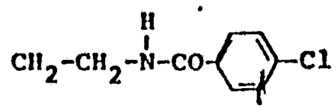 | $C_2H_5$ | A | 8.0 - 7.1m (8H); 2.9m (2H); 1.5m (2H); 1.2d (3H) |
| 34 | 1 | $CH_3$ | 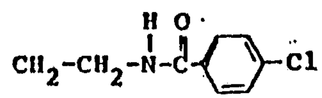 | H | A | 8.0 - 7.1m (8H); 2.9m (2H) 1.5m (2H); 1.2d (3H) |
| 35 | 1 | $CH_3$ | 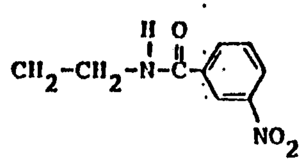 | $C_2H_5$ | A | 8.3 - 7.0m (8H); 2.9m (2H) 1.5m (2H); 1.2d (3H) |

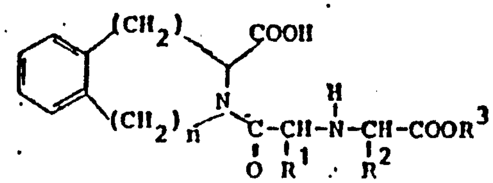

| | n | $R^1$ | $R^2$ | $R^3$ | Methode | NMR |
|---|---|---|---|---|---|---|
| 36 | 1 | $CH_3$ | 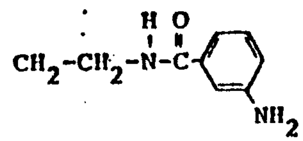 | $C_2H_5$ | A | 7.2 - 6.8m (8H); 2.9m (2H); 1.5m (2H) 1.2d (3H) |
| 37 | 1 | $CH_3$ | 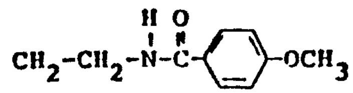 | $C_2H_5$ | A | 7.5 - 6.8m (8H); 3.8s (3H); 2.9m (2H); 1.5m (2H); 1.2d (3H) |
| 38 | 1 | $CH_3$ | 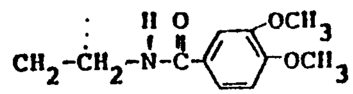 | $C_2H_5$ | A | 7.5 - 6.9m (7H); 3.9s (6H); 2.9m (2H); 1.5m (2H); 1.2d (3H) |
| 39 | 1 | $CH_3-CH_2-\underset{CH_3}{CH}-CH_2$ | 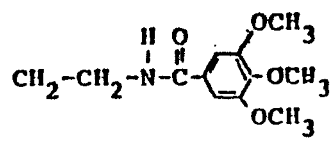 | $C_2H_5$ | A | 7.2 - 6.8m (6H); 3.9s (9H); 2.9m (2H); 1.5 - 1.0m (13H) |
| 40 | 1 | $CH_3$ | 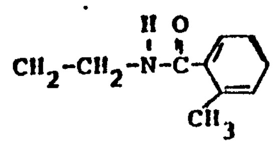 | $C_2H_5$ | A | 7.4 - 7.0m (8H); 2.9m (2H); 2.3s (3H); 1.5m (2H); 1.2d (3H) |

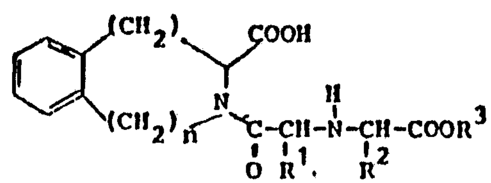

| | n | $R^1$ | $R^2$ | $R^3$ | Methode | NMR |
|---|---|---|---|---|---|---|
| 41 | 1 | $CH_3$ | 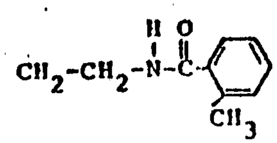 | H | A | 7.4 - 7.0m (8H); 2.9m (2H); 2.3s (3H); 1.5m (2H); 1.2d (3H) |
| 42 | 1 | $CH_3$ | $CH_2-CH_2-CH_2-N(C_2H_5)_2$ | $C_2H_5$ | A | 2.6 - 2.4m (6H); 1.5m (4H); 1.2d (3H 0.9t (6H) |
| 43 | 1 | $CH_3$ | 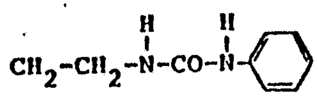 | $C_2H_5$ | A | 7.5 - 7.0m (9H); 2.9m (2H); 1.5m (2H); 1.2d (3H) |
| 44 | 1 | $CH_3$ | 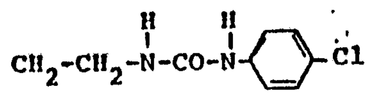 | $C_2H_5$ | A | 7.5 - 7.0m (8H); 2.9m (2H); 1.5m (2H 1.2d (3 H) |
| 45 | 1 | $CH_3$ | 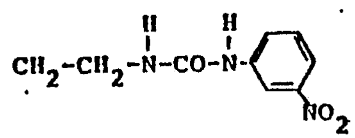 | $C_2H_5$ | A | 8.3 - 7.1m (8H); 2.9m (2H); 1.5m (2H 1.2d (3H) |
| 46 | 1 | $CH_3$ | 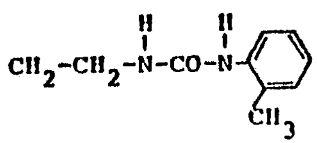 | $C_2H_5$ | A | 7.4 - 7.0m (8H); 2.9m (2H); 2.3s (3H 1.5m (2H); 1.2d (3H) |

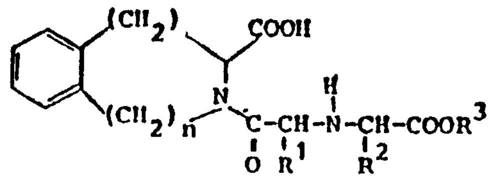

| | n | $R_1$ | $R_2$ | $R_3$ | Methode | NMR |
|---|---|---|---|---|---|---|
| 47 | 1 | $CH_3$ | 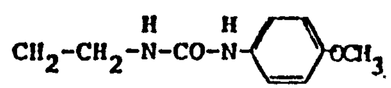 | $C_2H_5$ | A | 7,2-6,5m (8H); 3,9s (3H); 2,9m (2H); 1,5m (2H); 1,2d (3H) |
| 48 | 1 | $CH_3$ | 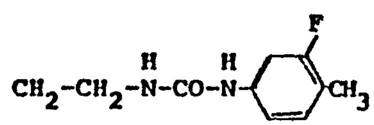 | $C_2H_5$ | A | 7,3-6,9m (7H); 2,9m (2H); 2,3s (3H); 1,5m (2H); 1,2d (3H) |

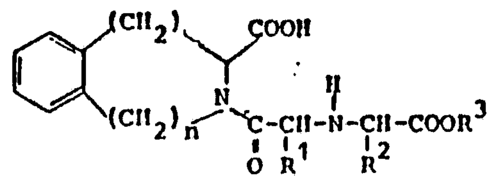

| | n | $R_1$ | $R_2$ | $R_3$ | Methode | NMR |
|---|---|---|---|---|---|---|
| 49 | 1 | $(CH_3)_2CH-CH_2$ | $CH_2-CH_2-N(H)-CO-N(H)-CH_3$ | $C_2H_5$ | A | 3,0-2,6m (5H); 1,7-1,3m (5H); 1,0d (6H) |
| 50 | 1 | $(CH_3)_2CH-CH_2$ | $CH_2-CH_2-N(H)-CO-N(H)-C_4H_9$ | $C_2H_5$ | A | 3,0-2,6m (4H); 1,7-1,2m (9H); 0,9 d+t (9H) |
| 51 | 1 | $(CH_3)_2CH-CH_2$ | $CH_2-CH_2-N(H)-CO-N(H)-C_4H_9$ | H | A | 3,0-2,6m (4H); 1,7-1,2m (9H); 0,9 d+t (9H) |
| 52 | 1 | $CH_2F$ | $CH_2-CH_2-N(H)-CO-N(H)-$ 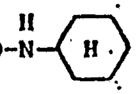 | $C_2H_5$ | A | 5,1-4,3m (5H); 2,9m (3H); 2,0-1,0m (12H) |
| 53 | 1 | $CH_3$ | $CH_2-CH_2-N(H)-CO-O-CH_2-$ 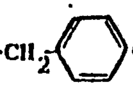 | $C_2H_5$ | A | 7,1s (5H); 5,0s (2H); 2,9m (2H); 1,5m (2H); 1,2 d (3H) |

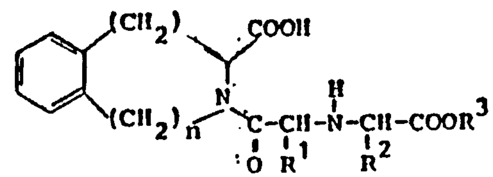

| | n | $R_1$ | $R_2$ | $R_3$ | Methode | NMR |
|---|---|---|---|---|---|---|
| 54 | 1 | $CH_3$ | $CH_2-CH_2-NH-CO-O-C_2H_5$ | $C_2H_5$ | A | 4,2q (4H); 2,9m (2H); 1,5m (2H); 1,2 q+t (9H) |
| 55 | 1 | $CH_3$ | $CH_2-CH_2-NH-CH_3$ | $C_2H_5$ | A | 2,4m (2H); 2,3s (3H); 1,5 m (2H); 1,2d (3H) |
| 56 | 1 | $CH_3$ | 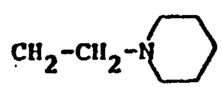 | $C_2H_5$ | A | 2,4m (6H); 1,8-1,2m (8H); 1,2d (3H) |
| 57 | 1 | $CH_3$ | 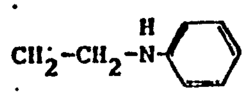 | $C_2H_5$ | A | 7,2-6,5m (9H); 2,5m (2H); 1,5m (2H) 1,2d (3H) |
| 58 | 1 | $(CH_3)_2CH-CH_2$ | 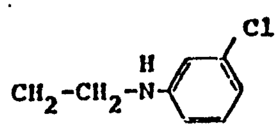 | $C_2H_5$ | A | 7,2-6,6m (8H); 2,4m (2H); 1,5m(5H) 1,0d (6H) |

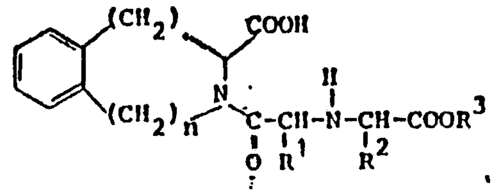

| | n | $R_1$ | $R_2$ | $R_3$ | Methode | NMR |
|---|---|---|---|---|---|---|
| 59 | 1 | 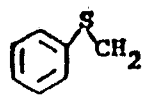 | 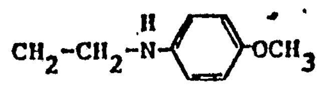 | $C_2H_5$ | A | 7,3-6,4m (13H); 3,8s (3H); 2,5-2,2m (4H); 1,5m (2H) |
| 60 | 1 | $CH_2F$ | 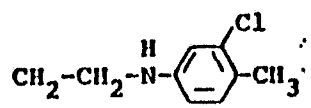 | $C_2H_5$ | A | 7,2-6,6m (7H); 5,1-4,3m (5H); 2,4m (2H); 2,3s (3H); 1,5m (2H) |
| 61 | 1 | $CH_3$ | 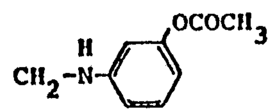 | $C_2H_5$ | A | 7,3-6,5m (8H); 2,5m (2H); 2,1s (3H); 1,2d (3H) |
| 62 | 1 | $CH_3$ | 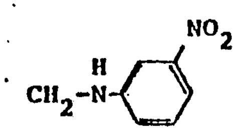 | $C_2H_5$ | A | 7,7-7,0m (8H); 2,5m (2H); 1,2d (3H) |
| 63 | 1 | $CH_3$ | 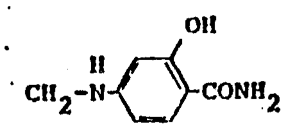 | $C_2H_5$ | A | 7,6-6,7m (7H); 2,4 m (2H); 1,2d (3H) |

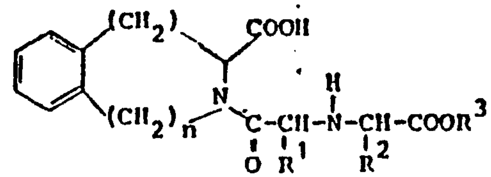

| | n | $R_1$ | $R_2$ | $R_3$ | Methode | NMR |
|---|---|---|---|---|---|---|
| 64 | 1 | $CH_3$ | 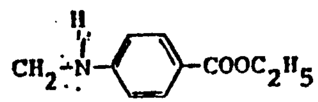 | $C_2H_5$ | A | 7,6-6,8m (8H) ; 4,2q (4H) ; 2,5m (2H) 1,2d + t (9H) |
| 65 | 1 | $CH_3$ | 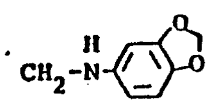 | $C_2H_5$ | A | 7,2-6,2m (7H) ; 5,0s (2H) ; 2,5m (2H) 1,2d (3H) |
| 66 | 1 | $CH_3$ | 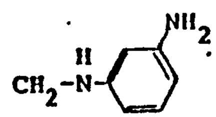 | $C_2H_5$ | A | 7,2-6,7m (8H) ; 2,5m (2H) ; 1,2d (3H) |
| 67 | 1 | $CH_3$ | $-CH_2-\overset{H}{N}-CH_2-CH_2-COOC_2H_5$ | $C_2H_5$ | A | 4,2q (4H) ; 2,6-2,3m (6H) ; 1,2 d + t (9H) |
| 68 | 1 | $CH_3$ | $CH_2-CH_2-CH_2-NH_2$ | H | C | 2,3m (2H) ; 1,5m (4H) ; 1,2d (3H) |

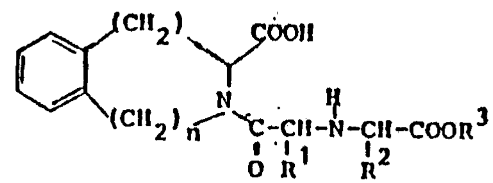

| | n | $R^1$ | $R^2$ | $R^3$ | Methode | NMR |
|---|---|---|---|---|---|---|
| 69 | 1 | $CH_3$ | $CH_2-N(H)-CH_2-CH_2-N(C_2H_5)_2$ | $C_2H_5$ | A | 2.6 - 2.4m (10H); 1.2d+t (9H) |
| 70 | 1 | $CH_3$ | $CH_2-$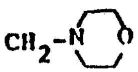 | $C_2H_5$ | A | 3.9 - 3.0m (12H); 2.3m (2H); 1.2d (3H) |
| 71 | 1 | $CH_3$ | $CH_2-$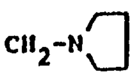 | $C_2H_5$ | A | 2.6 - 2.3m (6H); 1.6 - 1.4m (4H); 1.2d (3H) |
| 72 | 1 | $CH_3$ | $CH_2-N(H)-CH_2-CH_2-CONH_2$ | $C_2H_5$ | A | 2.6 - 2.2m (6H); 1.2d (3H) |
| 73 | 1 | $CH_3$ | $CH_2-CH_2-CH_2-N(CH_3)_2$ | $C_2H_5$ | A | 2.4m (2H); 2.2s (6H); 1.5m (4H) 1.2d (3H) |
| 74 | 1 | $CH_3$ | $CH_2-N(H)-CH_2-CONH_2$ | $C_2H_5$ | A | 3.9 - 3.0m(6H); 2.4m (2H); 1.2d (3H) |
| 75 | 1 | $CH_3$ | $(CH_2)_5-NH_2$ | H | C | 2.3m (2H); 1.6 - 1.2m (8H); 1.2d (3H) |

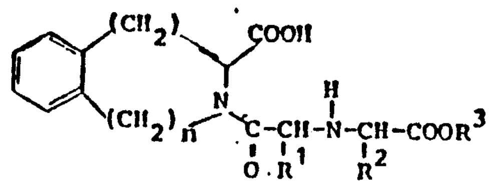

| | n | $R^1$ | $R^2$ | $R^3$ | Methode | NMR |
|---|---|---|---|---|---|---|
| 76 | 1 | $H_2N-(CH_2)_4$ | $(CH_2)_4-NH_2$ | H | C | 2.4m (4H); 1.6 - 1.2m (12H) |
| 77 | 1 | $CH_3$ | $CH_2-CH_2-NH-CH_3$ | $C_2H_5$ | A | 2.4m (2H); 2.2s (3H); 1.5m (2H); 1.2d (3H) |
| 78 | 1 | $CH_3$ | $CH_2-NH-CH_3$ | $C_2H_5$ | A | 2.4m (2H); 2.1s (3H); 1.2d (3H) |
| 79 | 0 | $CH_3$ | 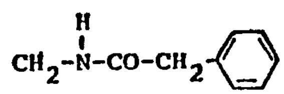 | $C_2H_5$ | A | 7.2 - 6.5m (9H); 3.8 - 3.0m (6H); 2.9m (2H); 1.2d (3H) |
| 80 | 0 | $CH_3$ | 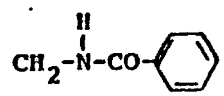 | $C_2H_5$ | A | 7.8 - 6.5m (9H); 2.9m (2H); 1.2d (3H) |
| 81 | 0 | $CH_3$ | 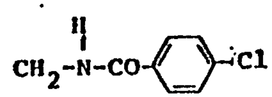 | $C_2H_5$ | A | 7.8 - 6.5m (8H); 2.9m (2H); 1.2d (3H) |
| 82 | 0 | $CH_3$ | 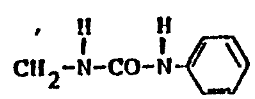 | $C_2H_5$ | A | 7.4 - 6.5m (9H); 2.9m (2H); 1.2d (3H) |

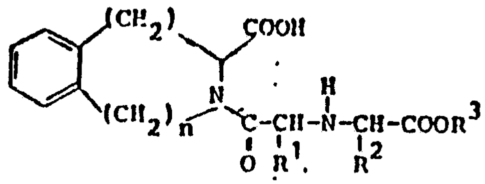

| | n | $R^1$ | $R^2$ | $R^3$ | Methode | NMR |
|---|---|---|---|---|---|---|
| 83 | O | $CH_3$ | 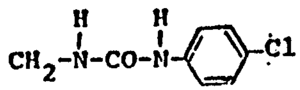 | $C_2H_5$ | A | 7.7 - 6.5m (8H); 2.9m (2H); 1.2d (3H) |
| 84 | O | $CH_3$ | $CH_2-NH-CO-NH-CH_3$ | $C_2H_5$ | A | 3.0 - 2.6m + s (5H); 1.2d (3H) |
| 85 | O | $CH_3$ | 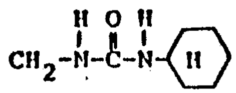 | $C_2H_5$ | A | 3.2 - 2.9m (3H); 2.4 - 1.2m (10H) 1.2d (3H) |
| 86 | O | $CH_3$ | 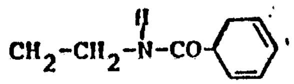 | $C_2H_5$ | A | 7.6 - 6.5m (9H); 2.9m (2H); 1.5m (2H); 1.2d (3H) |
| 87 | O | $CH_3$ | 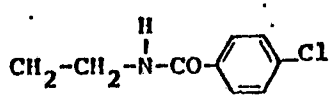 | $C_2H_5$ | A | 7.6 - 6.5m (8H); 2.9m (2H); 1.5m (2H); 1.2d (3H) |
| 88 | O | $CH_3$ | 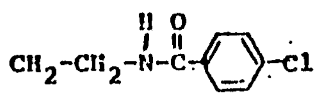 | H | A | 7.6 - 6.5m (8H); 2.9m (2H); 1.5m (2H); 1.2d (3H) |
| 89 | O | $CH_3$ | $CH_2-CH_2-CH_2-N(C_2H_5)_2$ | $C_2H_5$ | A | 2.6 - 2.4m (6H); 1.5m (4H); 1.2d (3H); 1.0 t (6H) |

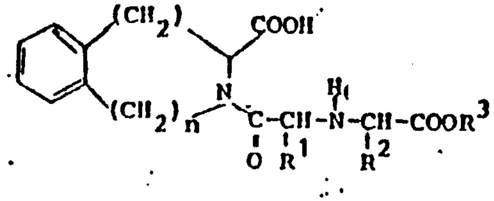

| | n | $R^1$ | $R^2$ | $R^3$ | Methode | NMR |
|---|---|---|---|---|---|---|
| 90 | 0 | $CH_3$ | 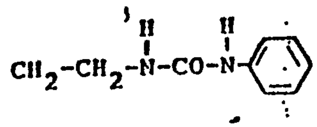 | $C_2H_5$ | A | 7.4 - 6.5m (9H); 2.9m (2H); 1.5m (2H); 1.2d (3H) |
| 91 | 0 | $CH_3$ | 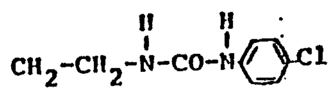 | $C_2H_5$ | A | 7.5 - 6.5m (8H); 2.9m (2H); 1.5m (2H); 1.2d (3H) |
| 92 | 0 | $CH_3$ | 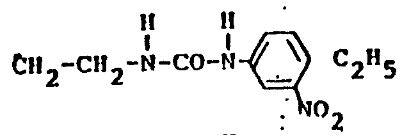 | $C_2H_5$ | A | 7.8 - 6.5m (8H); 2.9m (2H); 1.5m (2H); 1.2d (3H) |
| 93 | 0 | $CH_3$- | $CH_2-CH_2-NH-CO-NH-CH_3$ | $C_2H_5$ | A | 3.0 - 2.6m + s (5H); 1.5m (2H); 1.2d (3H) |
| 94 | 0 | $CH_3$ | 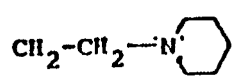 | $C_2H_5$ | A | 2.6 - 2.3m (6H); 1.8 - 1.2m (8H 1.2d (3H) |
| 95 | 0 | $CH_3$ | 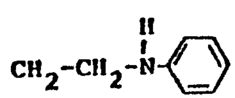 | $C_2H_5$ | A | 7.2 - 6.5m (9H); 2.4m (2H); 1.5m (2H); 1.2d (3H) |

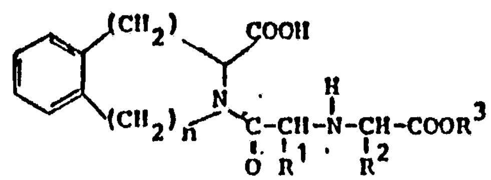

| | n | $R^1$ | $R^2$ | $R^3$ | Methode | NMR |
|---|---|---|---|---|---|---|
| 96 | O | $CH_3$ | 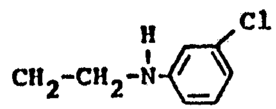 | $C_2H_5$ | A | 7.6 - 6.5m (8H); 2.4m (2H); 1.5m (2H); 1.2d (3H) |
| 97 | O | $CH_3$ | 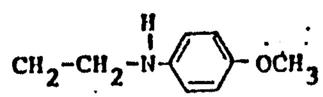 | $C_2H_5$ | A | 7.2 - 6.4m (8H); 3.9s (3H); 2.4m (2H); 1.5m (2H); 1.2d (3H) |
| 98 | O | $CH_3$ | $(CH_2)_5-NH_2$ | H | C | 2.4m (2H); 1.8 - 1.2m (8H); 1.2d (3H) |
| 99 | O | $CH_3$ | $(CH_2)_4-NH_2$ | H | C | 2.4m (2H); 1.8 - 1.2m (6H); 1.2d (3H) |

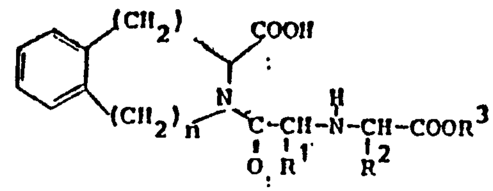

| | n | $R^1$ | $R^2$ | $R^3$ | Methode | NMR |
|---|---|---|---|---|---|---|
| 100 | 1 | $CH_3$ | 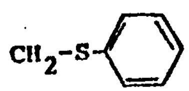 | $C_2H_5$ | A | 7,4-7,0m (9H); 2,7m (2H); 1,2d (3H) |
| 101 | 1 | $CH_3$ | 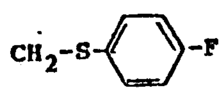 | $C_2H_5$ | A | 7,3-6,8m (8H); 2,7m (2H); 1,2d (3H) |
| 102 | 1 | $CH_3$ | $CH_2-SH$ | $C_2H_5$ | A | 2,4m (2H); 1,2d (3H) |

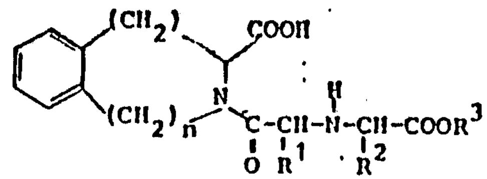

| | n | $R^1$ | $R^2$ | $R^3$ | Methode | NMR |
|---|---|---|---|---|---|---|
| 103 | 1 | $CH_3$ | 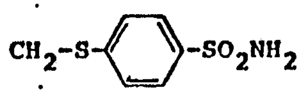 | $C_2H_5$ | A | 8,1-7,1m (8H); 6,5bs (2H); 2,7m (2H); 1,3d (3H) |
| 104 | 1 | $(CH_3)_2CH-CH_2$ | 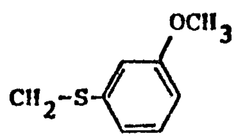 | $C_2H_5$ | A | 7,2-6,7m (8H); 3,8s (3H); 2,7m (2H); 1,6-1,2m (3H); 1,0d (6H) |
| 105 | 1 | 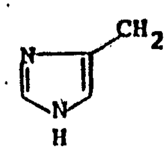 | 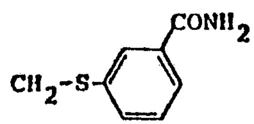 | $C_2H_5$ | A | 13,0s (1H); 7,8-6,8m(10H); 6,0bs (2H); 2,9-2,6m (4H) |
| 106 | 1 | 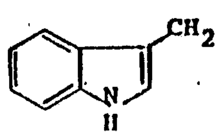 | 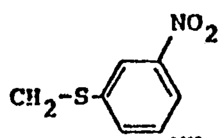 | $C_2H_5$ | A | 8,2-6,4m (14H); 2,9-2,6m (4H) |
| 107 | 1 | 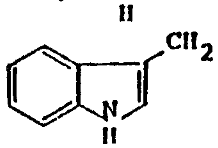 | 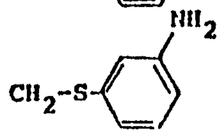 | $C_2H_5$ | A | 7,8-6,4m (14H); 2,9-2,6m (4H) |

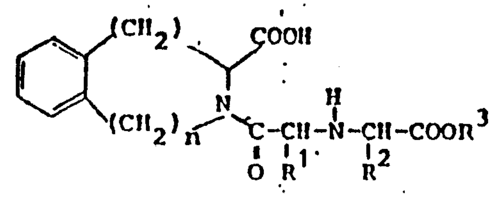

| | n | $R^1$ | $R^2$ | $R^3$ | Methode | NMR |
|---|---|---|---|---|---|---|
| 108 | 1 | $(CH_3)_2CH-CH_2$ | 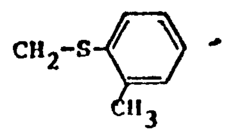 | $C_2H_5$ | A | 7,3-7,0m (8H); 2,7m (2H); 2,3s (3H); 1,9-1,4m (3H); 1,0d (6H) |
| 109 | 1 | $CH_3$ | 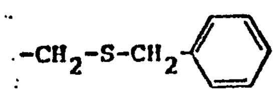 | $C_2H_5$ | A | 7,3-7,0m (9H); 3,9-3,0m+s (6H); 2,4m (2H); 1,2d (3H) |
| 110 | 1 | 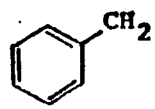 | $-CH_2-S-CH(CH_3)_2$ | $C_2H_5$ | A | 7,3-6,9m (9H); 2,6-2,3m (5H); 0,9d (6H) |
| 111 | 1 | $CH_3$ | $-CH_2-S-CH_2-CH_2-N(CH_3)_2$ | $C_2H_5$ | A | 2,6-2,2m+s (12H); 1,2d (3H) |
| 112 | 1 | $CH_2F$ | $-CH_2-S-CH_2-CH_2-CONH_2$ | $C_2H_5$ | A | 5,1-4,3m (5H); 2,5-2,2m (6H) |

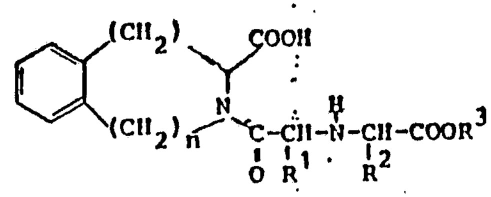

| | n | $R^1$ | $R^2$ | $R^3$ | Methode | NMR |
|---|---|---|---|---|---|---|
| 113 | 1 | $CH_3$ | $-CH_2-S-CH_2-CH_2-COOC_2H_5$ | $C_2H_5$ | A | 4,2q (4H); 2,5-2,2m (6H); 1,2d+t (9H) |
| 114 | 1 | $CH_3$ | $-CH_2-S-CH_2-CH_2-OC_2H_5$ | $C_2H_5$ | A | 3,9-3,0q+m (8H); 2,4-2,2m (4H); 1,2 d+t (6H) |
| 115 | 1 | $CH_3$ | $-CH_2-CH_2-S-$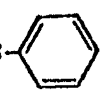 | $C_2H_5$ | A | 7,3-7,0m (9H); 2,7m (2H); 1,5m (2H); 1,2d (3H) |
| 116 | 1 | $CH_3$ | $-CH_2-CH_2-S-$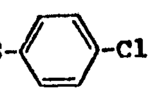$-Cl$ | $C_2H_5$ | A | 7,5-7,0m (8H); 2,7m (2H); 1,5m (2H); 1,2d (3H) |
| 117 | 1 | $CH_3$ | $-CH_2-CH_2-S-$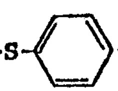$-COOH$ | $C_2H_5$ | A | 7,9-6,9m (8H); 2,7m (2H); 1,5m (2H); 1,2d (3H) |

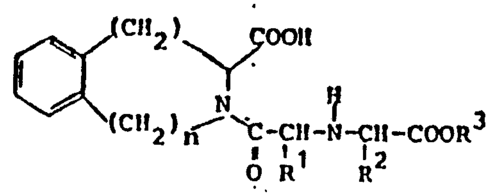

| | n | $R^1$ | $R^2$ | $R^3$ | Methode | NMR |
|---|---|---|---|---|---|---|
| 118 | 1 | $CH_3$ | 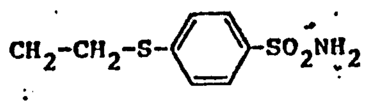 | $C_2H_5$ | A | 8,0-6,9m (8H); 6,5bq (2H); 2,6m (2H); 1,5m (2H); 1,2d (3H) |
| 119 | 1 | $CH_3$ | 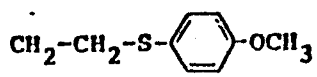 | $C_2H_5$ | A | 7,3-6,4m (8H); 3,8s (3H); 2,7m (2H); 1,5m (2H); 1,2d (3H) |
| 120 | 1 | $CH_3$ | 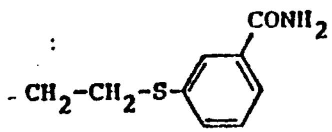 | $C_2H_5$ | A | 7,8-6,9m (8H); 6,0bs (2H); 2,6m (2H); 1,5m (2H); 1,2d (3H) |
| 121 | 1 | $CH_3$ | 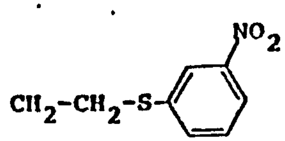 | $C_2H_5$ | A | 8,1-6,9m (8H); 2,7m (2H); 1,5m (2H); 1,2d (3H) |
| 122 | 1 | $H_2N-(CH_2)_4$ | 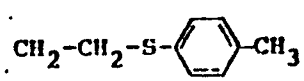 | $C_2H_5$ | B | 7,3-6,8m (8H); 2,7-2,3m+s (7H); 1,7-1,3m (8H) |

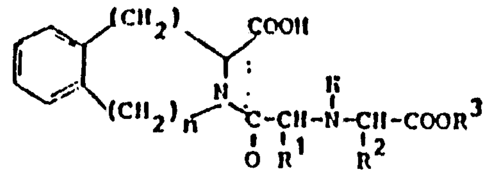

| | n | $R^1$ | $R^2$ | $R^3$ | Methode | NMR |
|---|---|---|---|---|---|---|
| 123 | 1 | $CH_3$ | 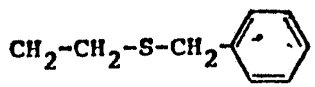 | $C_2H_5$ | A | 7,4-7,0m (9H); 3,9-3,0 m+s (6H); 2,4m (2H); 1,5m (2H), 1,2d (3H) |
| 124 | 1 | $CH_3$ | $CH_2-CH_2-S-CH(CH_3)_2$ | $C_2H_5$ | A | 2,6-2,3m (3H); 1,5m (2H); 1,2d (3H); 1,0d (6H) |
| 125 | 1 | 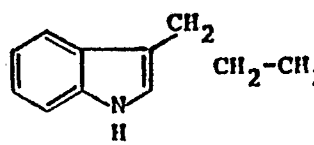 | $CH_2-CH_2-S-CH_2-CH_2-N(CH_3)_2$ | $C_2H_5$ | A | 7,8-6,5m (10H); 2,8-2,3m (14H); 1,5m (2H) |
| 126 | 1 | $CH_3$ | $CH_2-CH_2-S-CH_2-CH_2-CONH_2$ | $C_2H_5$ | A | 6,0 bs (2H); 2,5-2,2m (6H); 1,5m (2H); 1,2d (3H) |
| 127 | 1 | $CH_3$ | 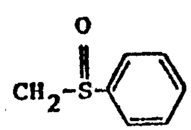 | $C_2H_5$ | D | 7,5-7,0m (9H); 2,6m (2H); 1,2d (3H) |

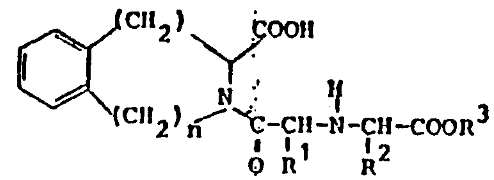

| | n | $R^1$ | $R^2$ | $R^3$ | Methode | NMR |
|---|---|---|---|---|---|---|
| 128 | 1 | $CH_3$ | 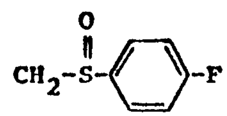 | $C_2H_5$ | D | 7,4-6,9m (8H); 2,6m (2H); 1,2d (3H) |
| 129 | 1 | $CH_3$ | 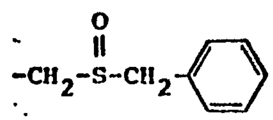 | $C_2H_5$ | D | 7,3-7,0m (9H); 4,1s (2H); 2,6m (2H); 1,2d (3H) |
| 130 | 1 | $CH_3$ | 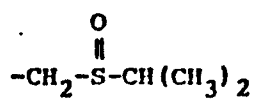 | $C_2H_5$ | D | 2,8-2,5m (3H); 1,2d (3H); 1,0d (6H) |
| 131 | 1 | $CH_3$ | 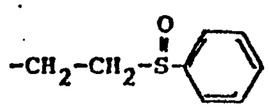 | $C_2H_5$ | D | 7,4-7,0m (9H); 2,6m (2H); 1,5m (2H); 1,2d (3H) |
| 132 | 1 | $CH_3$ | 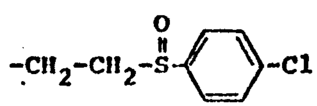 | $C_2H_5$ | D | 7,5-6,9m (8H); 2,6m (2H); 1,5m (2H); 1,2d (3H) |

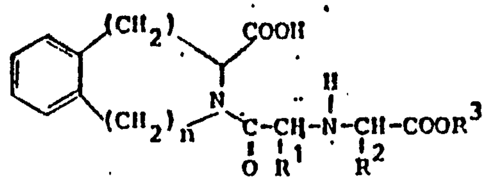

| | n | $R^1$ | $R^2$ | $R^3$ | Methode | NMR |
|---|---|---|---|---|---|---|
| 133 | 1 | $CH_3$ | 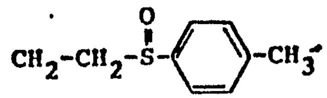 | $C_2H_5$ | D | 7,6-6,9m (8H); 2,6m (2H); 2,3s (3H); 1,5m (2H); 1,2d (3H) |
| 134 | 1 | $CH_3$ | 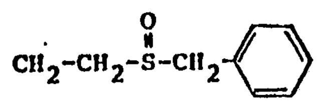 | $C_2H_5$ | D | 7,3-7,0m (9H); 4,1s (2H); 2,7m (2H); 1,5m (2H); 1,2d (3H) |
| 135 | 1 | $CH_3$ | 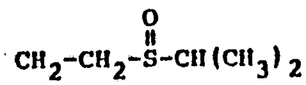 | $C_2H_5$ | D | 2,8-2,5m (3H); 1,5m (2H); 1,2d (3H); 0,9d (6H) |
| 136 | 1 | $CH_3$ | 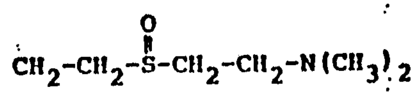 | $C_2H_5$ | D | 2,8-2,2m+s (12H); 1,5m (2H); 1,2d (3H) |
| 137 | 1 | $CH_2$ | 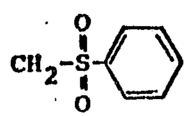 | $C_2H_5$ | E | 7,8-7,1m (9H); 2,8m (2H); 1,2d (3H) |

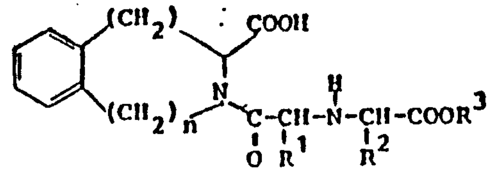

| | n | $R^1$ | $R^2$ | $R^3$ | Methode | NMR |
|---|---|---|---|---|---|---|
| 138 | 1 | $CH_3$ | 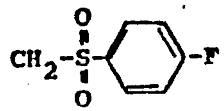 | $C_2H_5$ | E | 7,7-6,9m (8H); 2,8m (2H); 1,2d (3H) |
| 139 | 1 | $CH_3$ | 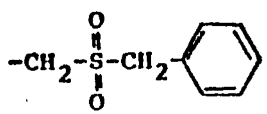 | $C_2H_5$ | E | 7,6-7,0m (9H); 5,0-4,3m (5H); 2,8m (2H); 1,2d (3H) |
| 140 | 1 | $CH_3$ | 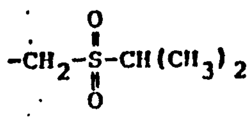 | $C_2H_5$ | E | 3,0-2,7m (3H); 1,2 d+t (9H) |
| 141 | 1 | $CH_3$ | 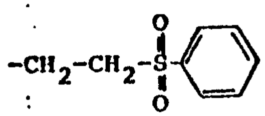 | $C_2H_5$ | E | 7,7-7,2m (9H); 2,8m (2H); 1,5m (2H); 1,2d (3H) |
| 142 | 1 | $CH_3$ | 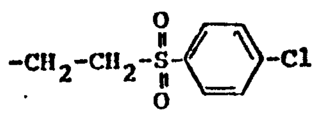 | $C_2H_5$ | E | 8,0-7,1m (8H); 2,8m (2H); 1,5m (2H); 1,2d (3H) |

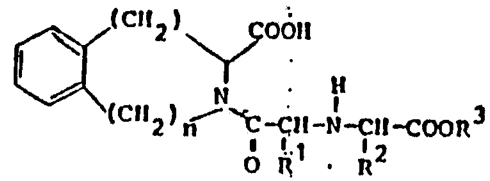

| | n | $R^1$ | $R^2$ | $R^3$ | Methode | NMR |
|---|---|---|---|---|---|---|
| 143 | 1 | $CH_3$ | 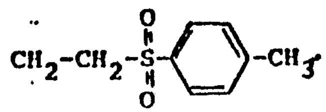 | $C_2H_5$ | E | 7,6-6,9m (8H); 2,8m (2H); 2,3s (3H); 1,5m (2H); 1,2d (3H) |
| 144 | 1 | $CH_3$ | 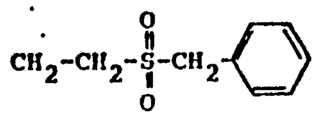 | $C_2H_5$ | E | 7,2-6,9m (9H); 4,2s (2H); 2,8m (2H); 1,5m (2H); 1,2d (3H) |
| 145 | 1 | $CH_3$ | $CH_2-CH_2-SO_2-CH(CH_3)_2$ | $C_2H_5$ | E | 3,0-2,7m (3H); 1,5m (2H); 1,1 d+t (9H) |
| 146 | 1 | $CH_3$ | $CH_2-CH_2-SO_2-CH_2-CH_2-N(CH_3)_2$ | $C_2H_5$ | E | 3,0-2,2 m+s (12H); 1,5m (2H); 1,2d (3H) |
| 147 | 0 | $CH_3$ | 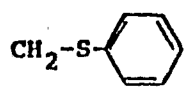 | $C_2H_5$ | A | 7,4-6,5m (9H); 2,5m (2H); 1,2d (3H) |

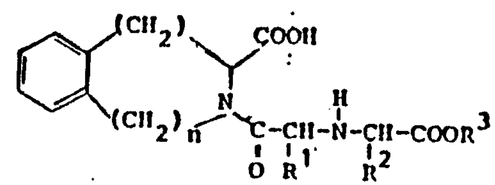

| | n | $R^1$ | $R^2$ | $R^3$ | Methode | NMR |
|---|---|---|---|---|---|---|
| 148 | 0 | $CH_3$ | 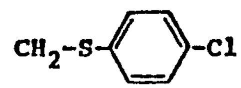 | $C_2H_5$ | A | 7,4-6,4m (8H); 2,5m (2H); 1,2d (3H) |
| 149 | 0 | $CH_3$ | 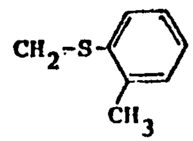 | $C_2H_5$ | A | 7,5-6,5m (8H); 2,5m (2H); 2,3s (3H); 1,2d (3H) |
| 150 | 0 | $CH_3$ | 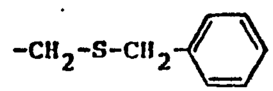 | $C_2H_5$ | A | 7,3-6,5m (9H); 3,6-3,0 m+s (6H); 2,4m (2H); 1,2d (3H) |
| 151 | 0 | $CH_3$ | $-CH_2-S-CH-(CH_3)_2$ | $C_2H_5$ | A | 2,7-2,3m (3H); 1,2d (3H); 0,9d (6H) |
| 152 | 0 | $CH_3$ | 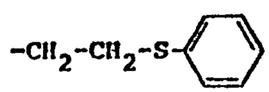 | $C_2H_5$ | A | 7,3-6,5m (9H), 2,7m (2H); 1,5m (2H); 1,2d (3H) |

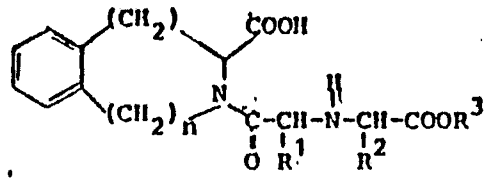

| | n | $R^1$ | $R^2$ | $R^3$ | Methode | NMR |
|---|---|---|---|---|---|---|
| 153 | O | $CH_3$ | 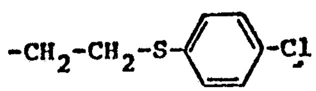 | $C_2H_5$ | A | 7,5-6,5m (8H); 2,7m (2H); 1,5m (2H); 1,2d (3H) |
| 154 | O | $CH_3$ | 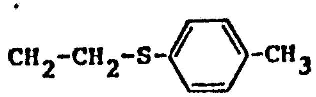 | $C_2H_5$ | A | 7,3-6,5m (8H); 2,7-2,3m+s (5H); 1,5m (2H); 1,2d (3H) |
| 155 | O | $CH_3$ | 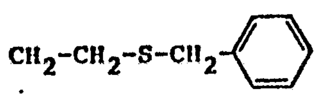 | $C_2H_5$ | A | 3,9-3,0m+s (6H); 2,4m (2H); 1,5m (2H); 1,2d (3H) |
| 156 | O | $CH_3$ | $CH_2-CH_2-S-CH(CH_3)_2$ | $C_2H_5$ | A | 2,6-2,3m (3H); 1,5m (2H); 1,2d (3H); 1,0d (6H) |
| 157 | O | $CH_3$ | 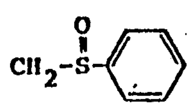 | $C_2H_5$ | D | 7,5-6,5m (9H); 2,6m (2H); 1,2d (3H) |

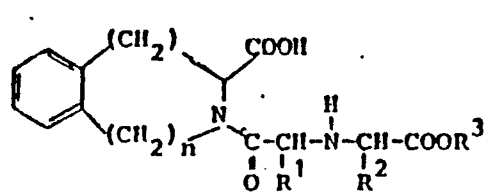

| | n | $R^1$ | $R^2$ | $R^3$ | Methode | NMR |
|---|---|---|---|---|---|---|
| 158 | 0 | $CH_3$ | 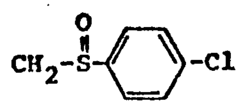 | $C_2H_5$ | D | 7,4-6,5m (8H); 2,6m (2H); 1,2d (3H) |
| 159 | 0 | $CH_3$ | 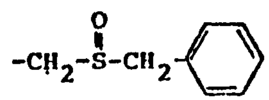 | $C_2H_5$ | D | 7,3-6,5m (9H); 4,1s (2H); 2,6m (2H); 1,2d (3H) |
| 160 | 0 | $CH_3$ | 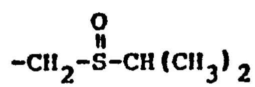 | $C_2H_5$ | D | 2,8-2,5m (3H); 1,2d (3H); 1,0d (6H) |
| 161 | 0 | $CH_3$ | 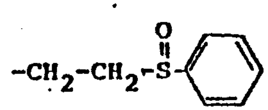 | $C_2H_5$ | D | 7,4-6,5m (9H); 2,6m (2H); 1,5m (2H); 1,2d (3H) |
| 162 | 0 | $CH_3$ | 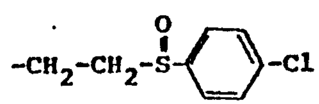 | $C_2H_5$ | D | 7,5-6,5m (8H); 2,6m (2H); 1,5m (2H); 1,2d (3H) |

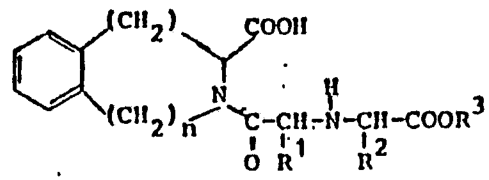

| | n | $R^1$ | $R^2$ | $R^3$ | Methode | NMR |
|---|---|---|---|---|---|---|
| 163 | 0 | $CH_3$ | 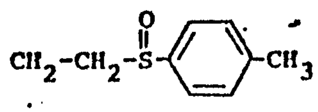 | $C_2H_5$ | D | 7.6-6.5m (8H); 2.6m (2H); 2.3s (3H); 1.5m (2H); 1.2d (3H) |
| 164 | 0 | $CH_3$ | 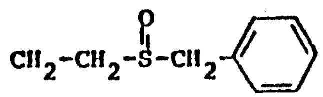 | $C_2H_5$ | D | 7.3-6.5m (9H); 4.1s (2H); 2.7m (2H); 1.5m (2H); 1.2d (3H) |
| 165 | 0 | $CH_3$ | 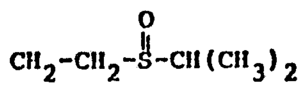 | $C_2H_5$ | D | 2.8-2.5m (3H); 1.5m (2H); 1.2d (3H); 1.1d (6H) |
| 166 | 0 | $CH_3$ | 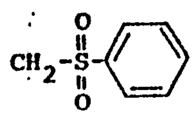 | $C_2H_5$ | E | 7.8-6.5m (9H);, 2.8m (2H); 1.2d (3H) |
| 167 | 0 | $CH_3$ | 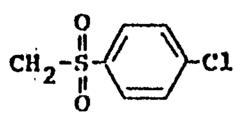 | $C_2H_5$ | E | 7.7-6.5m (8H); 2.8m (2H); 1.2d (3H) |

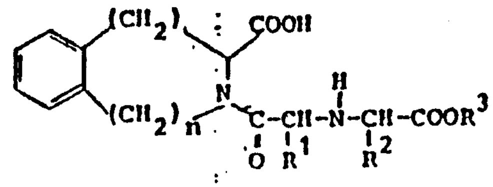

| | n | $R^1$ | $R^2$ | $R^3$ | Methode | NMR |
|---|---|---|---|---|---|---|
| 168 | O | $CH_3$ | 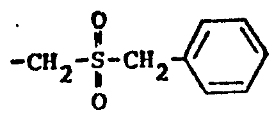 | $C_2H_5$ | E | 7.6-6.5m (9H); 4.3s (2H); 2.8m (2H); 1.2d (3H) |
| 169 | O | $CH_3$ | 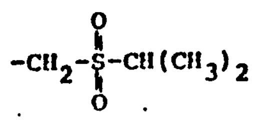 | $C_2H_5$ | E | 3.0-2.7m (3H); 1.2 d+t (9H) |
| 170 | O | $CH_3$ | 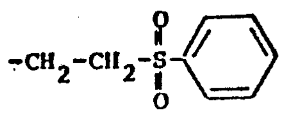 | $C_2H_5$ | E | 7.7-6.5m (9H); 2.8m (2H); 1.5m (2H); 1.2d (3H) |
| 171 | O | $CH_3$ | 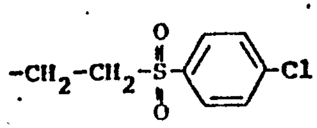 | $C_2H_5$ | E | 8.0-6.5m (8H); 2.8m (2H); 1.5m (2H); 1.2d (3H) |
| 172 | O | $CH_3$ | 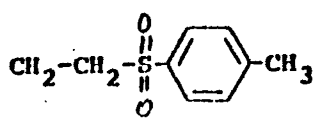 | $C_2H_5$ | E | 7.7-6.5m (8H); 2.8m (2H); 2.3s (3H); 1.5m (2H); 1.2d (3H) |

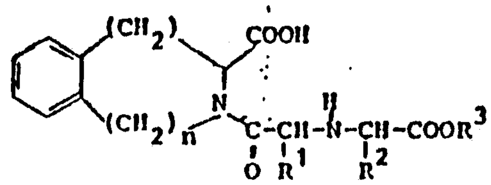

| | n | $R^1$ | $R^2$ | $R^3$ | Methode | NMR |
|---|---|---|---|---|---|---|
| 173 | 0 | $CH_3$ | 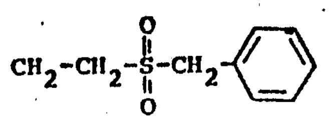 | $C_2H_5$ | E | 7.2-6.5m (9H); 4.2s (2H); 2.8m (2H); 1.5m (2H); 1.2d (3H) |
| 174 | 0 | $CH_3$ | 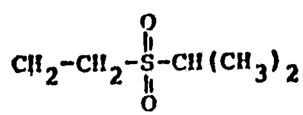 | $C_2H_5$ | E | 3.0-2.7m (3H); 1.5m (2H); 1.1 d+t (9H) |
| 175 | 1 | $CH_3$ | $-CH_2-OH$ | $C_2H_5$ | A | 3.9-3.0m (6H); 1.2d (3H) |
| 176 | 1 | $CH_3$ | 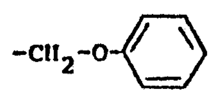 | $C_2H_5$ | A | 7.3-6.7m (9H); 3.9-3.0m (6H) 1.2d (3H) |
| 177 | 1 | $CH_3$ | 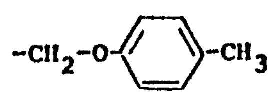 | $C_2H_5$ | A | 7.3-6.6m (8H); 3.9-3.0m (6H); 2.3s (3H); 1.2d (3H) |

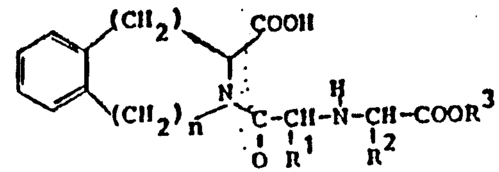

| | n | $R^1$ | $R^2$ | $R^3$ | Methode | NMR |
|---|---|---|---|---|---|---|
| 178 | 1 | $CH_3$ | 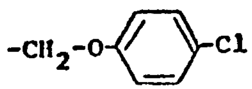 | $C_2H_5$ | A | 7.3-6.7m (8H); 3.9-3.0m (6H); 1.2d (3H) |
| 179 | 1 | $H_2N-(CH_2)_4$ | 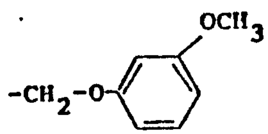 | $C_2H_5$ | B | 7.2-6.4m (8H); 3.9-3.0 m+s (9H); 2.4m (2H); 1.7-1.3m (6H) |
| 180 | 1 | $H_2N-(CH_2)_3$ | 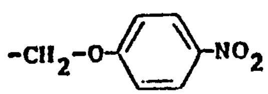 | $C_2H_5$ | B | 8.2-6.8m (8H); 3.9-3.0m (6H); 2.4m (2H); 1.7-1.3m (4H) |
| 181 | 1 | $(CH_3)_2-CH-CH_2-$ | 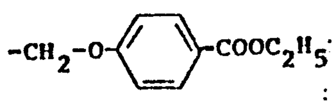 | $C_2H_5$ | A | 7.6-6.8m (8H); 4.2q (4H); 3.9-3.0 m (6H); 1.9-1.3m (3H); 1.2t (6H) 1.0d (6H) |
| 182 | 1 | $CH_3$ | 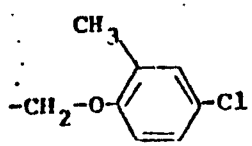 | $C_2H_5$ | A | 7.2-6.8m (7H); 3.9-3.0m (6H); 2.3s (3H); 1.2d (3H) |

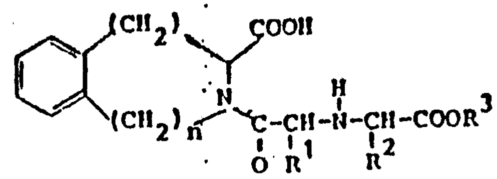

| | n | R1 | R2 | R3 | Methode | NMR |
|---|---|---|---|---|---|---|
| 183 | 1 | $CH_3$ | 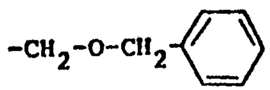 | $C_2H_5$ | A | 7.2-6.9m (9H); 4.0s (2H); 3.9-3.0m (6H); 1.3d (3H) |
| 184 | 1 | $CH_3$ | 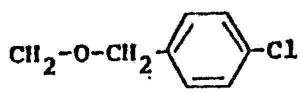 | $C_2H_5$ | A | 7.4-6.9m (8H); 4.0s (2H); 3.9-3.0m (6H); 1.2d (3H) |
| 185 | 1 | $H_2N-(CH_2)_4$ | 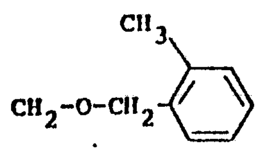 | $C_2H_5$ | B | 7.4-7.0m (8H); 4.0s (2H); 3.9-3.0 m (6H); 2.5-2.2 d+t (5H); 1.7-1.3m (6H) |
| 186 | 1 | 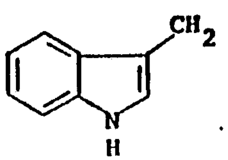 | 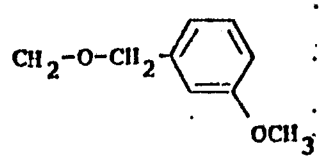 | $C_2H_5$ | A | 7.8-6.4m(14H); 4.0s (2H); 3.8s (3H) 3.9-3.0m (6H); 2.7m (2H) |
| 187 | 1 | H | 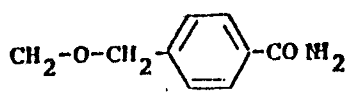 | $C_2H_5$ | A | 7.8-7.0m (8H); 6.0 bs (2H); 4.0 s (2H); 3.9-3.0m (7H) |

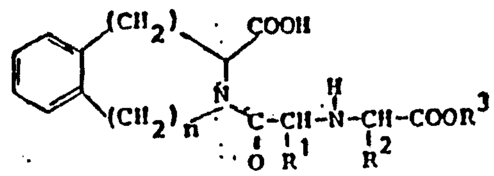

| | n | $R^1$ | $R^2$ | $R^3$ | Methode | NMR |
|---|---|---|---|---|---|---|
| 188 | 1 | $HS-CH_2$ | 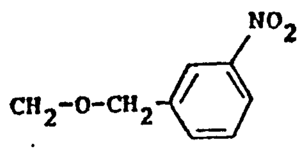 | $C_2H_5$ | B | 8.2-7.0m (8H); 4.0s (2H); 3.9-3.0m (6H); 2.3m (2H) |
| 189 | 1 | $HS-CH_2$ | 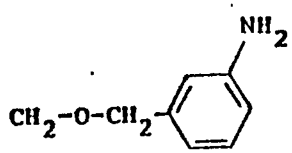 | $C_2H_5$ | B | 7.2-6.4m (8H); 4.0s (2H); 3.9-3.0m (6H); 2.3m(2H) |
| 190 | 1 | $CH_3$ | $CH_2-O-CH(CH_3)_2$ | $C_2H_5$ | A | 3.9-3.0m (7H); 1.2d (3H); 0.9d (6H) |
| 191 | 1 | $CH_3$ | $CH_2-O-CH_2-CH_2-N(CH_3)_2$ | $C_2H_5$ | A | 3.9-3.0m (8H); 2.4-2.1 m+s(8H); 1.2d (3H) |
| 192 | 1 | $CH_3$ | 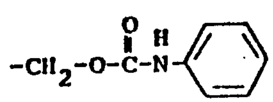 | $C_2H_5$ | A | 7.6-6.9m (9H); 4.0m (2H); 1.2d (3H) |

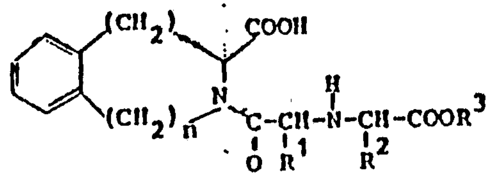

| | n | $R^1$ | $R^2$ | $R^3$ | Methode | NMR |
|---|---|---|---|---|---|---|
| 193 | 1 | $CH_3$ | $CH_2-COOH$ | H | A | 2,1m (2H); 1,2d (3H) |
| 194 | 1 | $CH_3$ | $CH_2-CONH_2$ | H | F | 6,5bs (2H); 2,1m (2H); 1,2d (3H) |
| 195 | 1 | $CH_3$ | $CH_2-CON(CH_3)_2$ | H | F | 3,9-3,0m+2s (10H); 2,1m (2H); 1,2d (3H) |
| 196 | 1 | 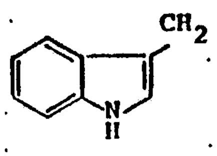 | $CH_2$-CON 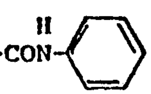 | H | F | 7,8-6,4m (15H); 2,8-2,0m (4H) |
| 197 | 1 | $CH_3$ | $CH_2$-CON 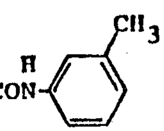 | H | F | 7,3-6,9m (8H); 2,3s (3H); 2,2m (2H); 1,2d (3H) |

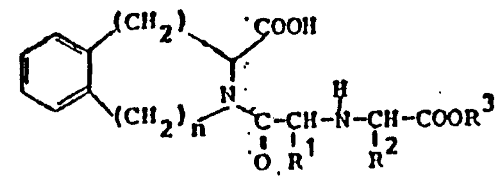

| | n | $R^1$ | $R^2$ | $R^3$ | Methode | NMR |
|---|---|---|---|---|---|---|
| 198 | 1 | $CH_3$ | 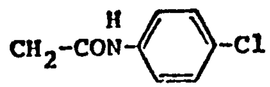 | H | F | 7,7-6,9m (8H); 2,3m (2H); 1,2d (3H) |
| 199 | 1 | 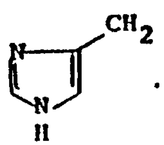 | 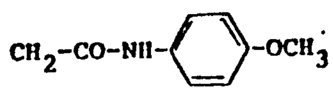 | H | F | 13,0s (1H); 7,5-6,3m (10H); 3,9s (3H); 2,7m (2H); 2,1m (2H) |
| 200 | 1 | $CH_3$ | 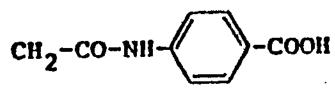 | H | F | 7,7-6,6m (8H); 2,1m (2H); 1,2d (3H) |
| 201 | 1 | $CH_3$ | 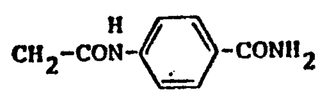 | H | F | 7,7-6,6m (8H); 2,1m (2H); 1,2d (3H) |
| 202 | 1 | $CH_3$ | 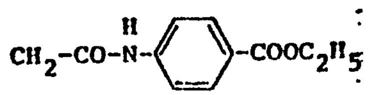 | H | F | 7,7-6,6m (8H); 4,2q (2H); 2,1m (2H); 1,2d+t (6H) |

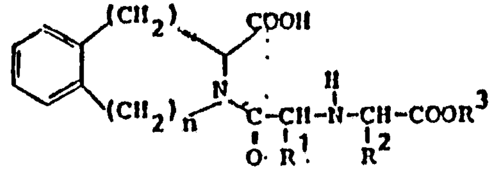

| | n | $R^1$ | $R^2$ | $R^3$ | Methode | NMR |
|---|---|---|---|---|---|---|
| 203 | 1 | $CH_3$ | 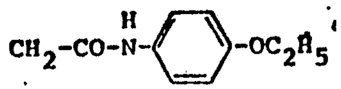 | H | F | 7,2-6,2m (8H); 3,5q (2H); 2,1m (2H); 1,2d+t (6H) |
| 204 | 1 | $CH_3$ | 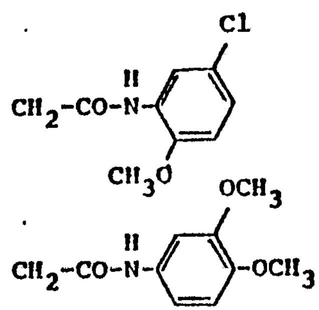 | H | F | 7,2-6,4m (7H); 3,9s (3H); 2,1m (2H); 1,2d (3H) |
| 205 | 1 | $CH_3$ | | H | F | 7,2-6,4m (7H); 3,8s (6H); 2,1m (2H); 1,2d (3H) |
| 206 | 1 | $CH_3$ | 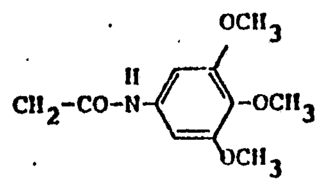 | H | F | 7,2-6,2m (6H); 3,9s (9H); 2,1m (2H); 1,2d (3H) |
| 207 | 1 | $CH_3$ | $CH_2-COOC_2H_5$ | H | F | 4,2q (2H); 2,0m (2H); 1,2d+t (6H) |

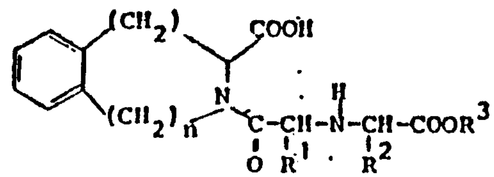

| | n | $R^1$ | $R^2$ | $R^3$ | Methode | NMR |
|---|---|---|---|---|---|---|
| 208 | 1 | $CH_3$ | $CH_2-CO-O-CH(CH_3)_2$ | H | F | 4,8septett (1H); 2,1m (2H); 1,2d (3H); 0,9d (6H) |
| 209 | 1 | $CH_3$ | $CH_2-CO-O-CH_2-$ 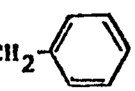 | H | F | 7,3-7,0m (9H); 5,3s (2H); 2,1m (2H); 1,2d (3H) |
| 210 | 1 | $CH_3$ | $CH_2-CH_2-COOH$ | $C_2H_5$ | A | 2,1m (2H); 1,5m (2H); 1,2d (3H) |
| 211 | 1 | $CH_3$ | $CH_2-CH_2-CONH_2$ | $C_2H_5$ | A | 2,1m (2H); 1,5m (2H); 1,2d (3H) |
| 212 | 1 | $CH_3$ | $CH_2-CH_2-CON(CH_3)_2$ | $C_2H_5$ | A | 3,02s (6H); 1,5m (2H); 1,2d (3H) |

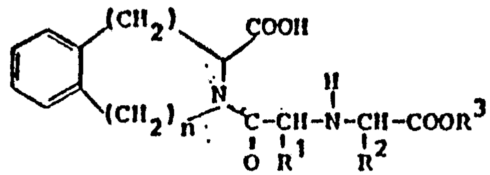

| | n | $R^1$ | $R^2$ | $R^3$ | Methode | NMR |
|---|---|---|---|---|---|---|
| 213 | 1 | $CH_3$ | 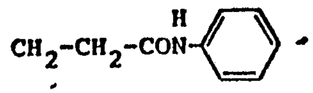 | $C_2H_5$ | A | 7,4-7,0m (9H); 2,1m (2H); 1,5m (2H); 1,2d (3H) |
| 214 | 1 | $CH_3$ | 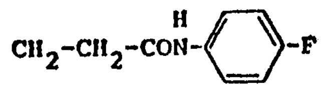 | $C_2H_5$ | A | 7,3-6,9m (8H); 2,1m (2H); 1,5m (2H); 1,2d (3H) |
| 215 | 1 | $H_2N-(CH_2)_4$ | 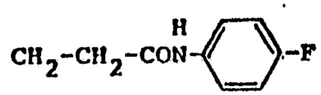 | $C_2H_5$ | B | 7,3-6,8m (8H); 2,4-2,1m (4H); 1,7-1,3m (8H) |
| 216 | 1 | $CH_3$ | 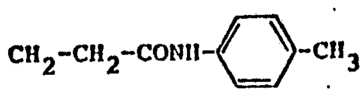 | $C_2H_5$ | A | 7,2-6,4m (8H); 3,9s (3H); 2,1m (2H); 1,5m (2H); 1,2d (3H) |
| 217 | 1 | $CH_3$ | 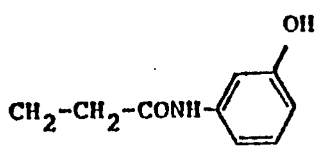 | H | A | 7,3-6,3m (8H); 2,1m (2H); 1,5m (2H); 1,2d (3H) |

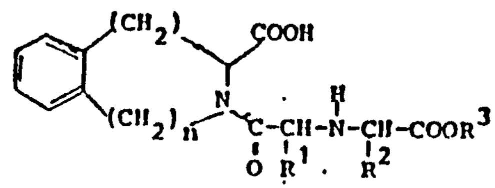

| | n | $R^1$ | $R^2$ | $R^3$ | Methode | NMR |
|---|---|---|---|---|---|---|
| 218 | 1 | $CH_3$ | 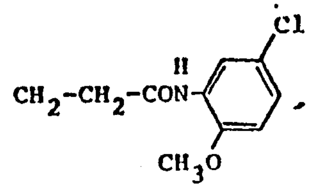 | H | A | 7,3-6,4m (7H); 3,9s (3H); 2,1m (2H); 1,5m (2H); 1,2d (2H) |
| 219 | 1 | $CH_3$ | 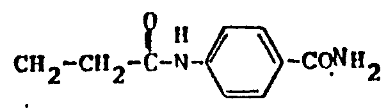 | H | A | 7,7-7,0m (8H); 2,1m (2H); 1,5m (2H); 1,2d (3H) |
| 220 | 1 | $CH_3$ | 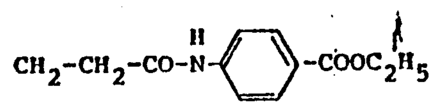 | H | A | 7,6-7,0m (8H); 4,2q (2H); 2,1m (2H); 1,5m (2H); 1,2 d+t (6H) |
| 221 | 1 | $CH_3$ | 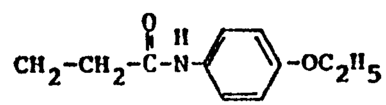 | H | A | 7,3-6,4m (8H); 3,9-3,0 m+q (6H); 2,1m (2H); 1,5m (2H); 1,2 d+t (6H) |
| 222 | 1 | $CH_3$ | 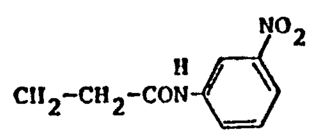 | H | A | 8,2-6,9m (8H); 2,1m (2H); 1,5m (2H); 1,2d (3H) |

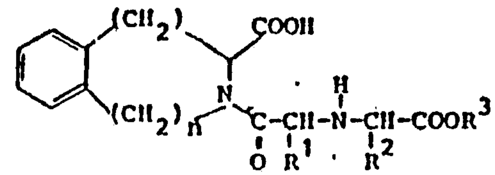

| | n | $R^1$ | $R^2$ | $R^3$ | Methode | NMR |
|---|---|---|---|---|---|---|
| 223 | 1 | $CH_3$ | 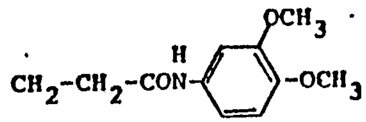 | $C_2H_5$ | A | 7,3-6,3m (7H); 3,8s (6H); 2,1m (2H); 1,5m (2H); 1,2d (3H) |
| 224 | 1 | $CH_3$ | 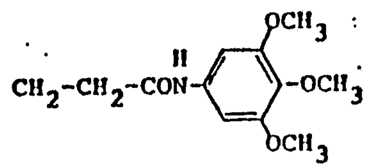 | $C_2H_5$ | A | 7,3-6,2m (6H); 3,9s (9H); 2,1m (2H); 1,5m (2H); 1,2d (3H) |
| 225 | 1 | $CH_3$ | $CH_2-CH_2-CON(H)-CH(CH_3)_2$ | H | A | 2,8-2,2m (3H); 1,5m (2H); 1,2d (3H); 1,0d (6H) |
| 226 | 1 | $CH_3$ | 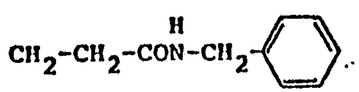 | H | A | 7,4-7,0m (9H); 5,1-4,3 m+s (5H); 2,1m (2H); 1,5m (2H); 1,2d (3H) |
| 227 | 1 | $CH_3$ | 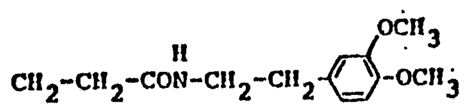 | $C_2H_5$ | A | 7,3-6,2m (7H); 3,9s (6H); 2,9-2,1m (6H), 1,5m (2H); 1,2d (3H) |

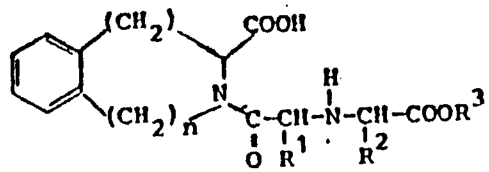

| | n | $R^1$ | $R^2$ | $R^3$ | Methode | NMR |
|---|---|---|---|---|---|---|
| 228 | O | $CH_3$ | $CH_2-OH$ | $C_2H_5$ | A | 3,6-3,0m (6H); 1,2d (3H) |
| 229 | O | $CH_3$ | 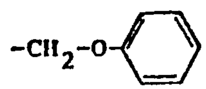 | $C_2H_5$ | A | 7,3-6,5m (9H); 3,9-3,0m (6H); 1,2d (3H) |
| 230 | O | $CH_3$ | 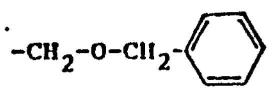 | $C_2H_5$ | A | 7,2-6,5m (9H); 4,0s (2H); 3,9-3,0m (6H); 1,2d (3H) |
| 231 | O | $CH_3$ | 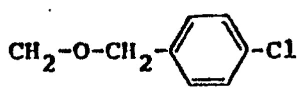 | $C_2H_5$ | A | 7,4-6,5m (8H); 4,0s (2H); 3,9-3,0m (6H); 1,2d (3H) |
| 232 | O | $CH_3$ | $CH_2-O-CH(CH_3)_2$ | $C_2H_5$ | A | 3,9-3,0m (7H); 1,2d (3H); 0,9d (6H) |

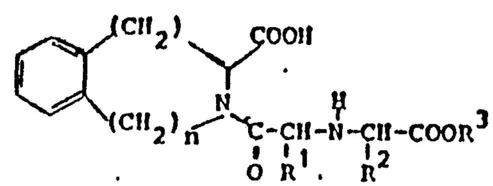

| | n | $R^1$ | $R^2$ | $R^3$ | Methode | NMR |
|---|---|---|---|---|---|---|
| 233 | O | $CH_3$ | $CH_2-O-CH_2-CH_2-N(CH_3)_2$ | $C_2H_5$ | A | 3,9-3,0m (8H); 2,4-2,1 m+s (8H); 1,2d (2H) |
| 234 | O | $CH_3$ | 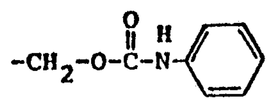 | $C_2H_5$ | A | 7,6-6,5m (9H); 4,0m (2H); 1,2d (3H) |
| 235 | O | $CH_3$ | $CH_2-COOH$ | H | A | 2,1m (2H); 1,2d (3H) |
| 236 | O | $CH_3$ | $CH_2-CONH_2$ | H | F | 6,5bs (2H); 2,1m (2H); 1,2d (3H) |
| 237 | O | $CH_3$ | $CH_2-CON(CH_3)_2$ | H | F | 3,9-3,0 m+2s (10H); 2,1m (2H); 1,2d (3H) |

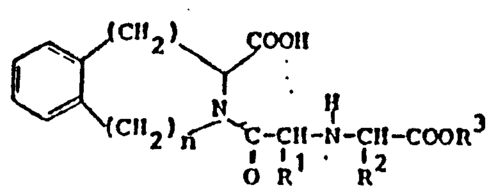

| | n | $R^1$ | $R^2$ | $R^3$ | Methode | NMR |
|---|---|---|---|---|---|---|
| 238 | O | $CH_3$ | 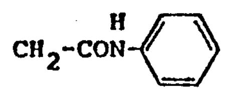 | H | F | 7,3-6,5m (9H); 2,1m (2H); 1,2d (3H) |
| 239 | O | $CH_3$ | 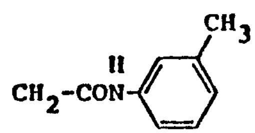 | H | F | 7,3-6,5m (8H); 2,3s (3H); 2,2m (2H); 1,2d (3H) |
| 240 | O | $CH_3$ | 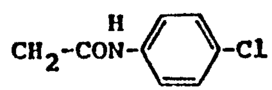 | H | F | 7,7-6,5m (8H); 2,3m (2H); 1,2d (3H) |
| 241 | O | $CH_3$ | $CH_2-COOC_2H_5$ | H | F | 4,2q (2H); 2,0m (2H); 1,2 d+t (6H) |
| 242 | O | $CH_3$ | $CH_2-CH_2-COOH$ | $C_2H_5$ | A | 2,1m (2H); 1,5m (2H); 1,2d (3H) |

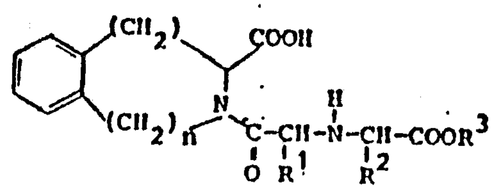

| | n | $R^1$ | $R^2$ | $R^3$ | Methode | NMR |
|---|---|---|---|---|---|---|
| 243 | O | $CH_3$ | $CH_2-CH_2-CONH_2$ | $C_2H_5$ | A | 2,1m (2H); 1,5m (2H); 1,2d (3H) |
| 244 | O | $CH_3$ | $CH_2-CH_2-CON(CH_3)_2$ | $C_2H_5$ | A | 3,0 2s (6H); 1,5m (2H); 1,2d (3H) |
| 245 | O | $CH_3$ | 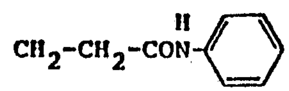 | $C_2H_5$ | A | 7,4-6,5m (9H); 2,1m (2H); 1,5m (2H); 1,2d (3H) |
| 246 | O | $CH_3$ | 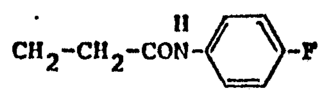 | H | A | 7,3-6,5m (8H); 2,1m (2H); 1,5m (2H); 1,2d (3H) |
| 247 | O | $CH_3$ | 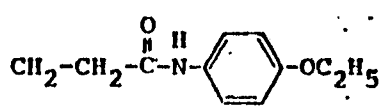 | $C_2H_5$ | A | 7.3-6.4m (8H); 3.6q (3H); 2.1m (2H); 1.5m (2H); 1.2 d+t (6H) |

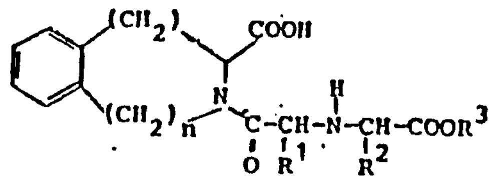

| | n | $R^1$ | $R^2$ | $R^3$ | Methode | NMR |
|---|---|---|---|---|---|---|
| 248 | O | $CH_3$ | $CH_2-CH_2-CON(H)-CH(CH_3)_2$ | $C_2H_5$ | A | 2,8-2,2m (3H); 1,5m (2H); 1,2d (3H); 1,0d (6H) |
| 249 | O | $CH_3$ | $CH_2-CH_2-CON(H)-CH_2-$ 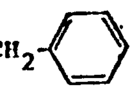 | $C_2H_5$ | A | 7,4-6,5m (9H); 5,1-4,3 m+s (5H); 2,1m (2H); 1,5m (2H); 1,2d (3H) |
| 250 | O | $CH_3$ | $CH_2-CH_2-CON(H)-CH_2-CH_2-$ 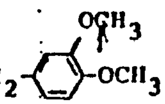 | $C_2H_5$ | A | 7,3-6,2m (7H); 3,9s (6H); 2,9-2,1m (6H); 1.5m (2H); 1,2d (3H) |

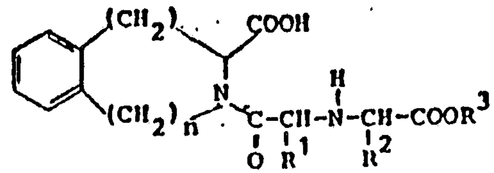

| | n | $R^1$ | $R^2$ | $R^3$ | Methode | NMR |
|---|---|---|---|---|---|---|
| 251 | 1 | $CH_3$ | 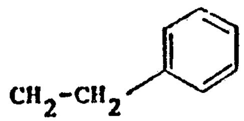 | $C_2H_5$ | B | 7.2bs (9H); 2.6m (2H); 1.7m (2H); 1.2d (3H) |
| 252 | 1 | $CH_3$ | 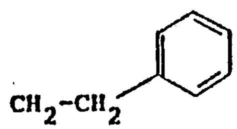 | H | B | 7.2bs(9H); 2.6m (2H); 1.7m (2H) |
| 253 | 1 | $CH_3$ | 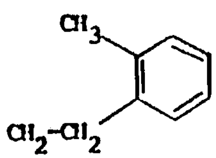 | $C_2H_5$ | B | 7.4-7.0m (8H); 2.6m (2H); 2.3 s (3H); 1.7m (2H); 1.2d (3H) |

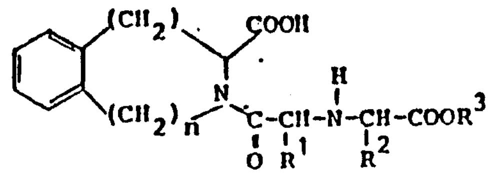

| | n | $R^1$ | $R^2$ | $R^3$ | Methode | NMR |
|---|---|---|---|---|---|---|
| 254 | 1 | $HO-CH_2$ | 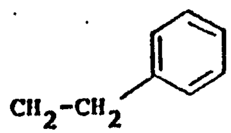 | $C_2H_5$ | B | 7.2bs (9H); 2.8-2.5m (4H); 1.7m (2H) |
| 255 | 1 | $H_2N-(CH_2)_4-$ | 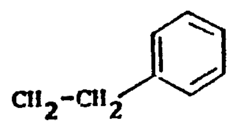 | H | B | 7.2bs (9H); 2.8-2.3m (4H); 1.8-1.3m (8H) |
| 256 | 1 | $H_2N(CH_2)_3-$ | 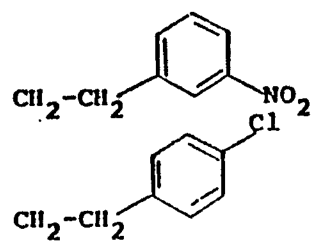 | H | B | 8.2-7.1m (8H); 2.8-2.3m (4H) 1.8-1.3m (6H) |
| 257 | 1 | $CH_3$ | | $C_2H_5$ | B | 7.5-6.9m (8H); 2.7m (2H); 1.7m (2H); 1.2d (3H) |
| 258 | 1 | $CH_3$ | 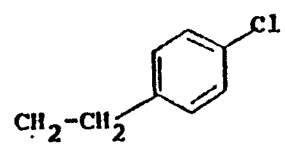 | H | B | 7.5-6.9m (8H); 2.7m (2H); 1.7m (2H); 1.2d (3H) |

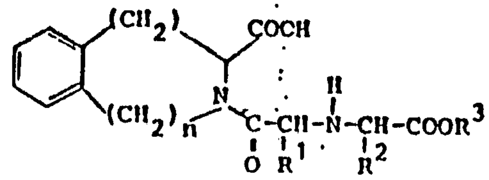

| | n | $R^1$ | $R^2$ | $R^3$ | Methode | NMR |
|---|---|---|---|---|---|---|
| 259 | 1 | $CH_3$ | 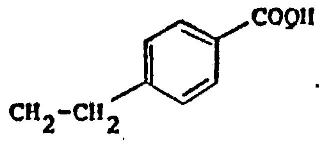 | H | B | 7.8-7.1m (8H); 2.7m (2H); 1.7m (2H); 1.2d (3H) |
| 260 | 1 | 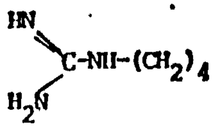 | 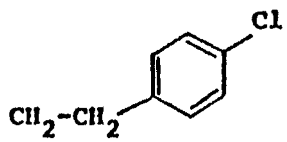 | $C_2H_5$ | B | 7.5-7.0m (8H); 2.8-2.6m (4H); 1.7-1.3m (6H) |
| 261 | 1 | $H_2N-(CH_2)_2$ | 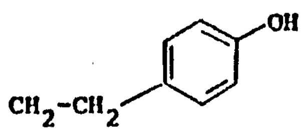 | H | B | 7.2-6.5m (8H); 2.8-2.3m (4H); 1.7-1.3m (4H) |
| 262 | 1 | $H_2N-(CH_2)_5$ | 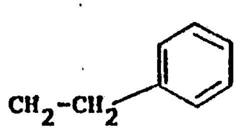 | H | B | 7.2bs (9H); 2.7-2.3m (4H); 1.7-1.2m (10H) |
| 263 | 1 | $H_2N-(CH_2)_4$ | 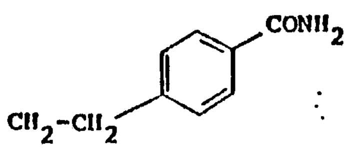 | H | B | 7.8-7.1m (8H); 2.7-2.3m (4H); 1.8-1.3m (8H) |

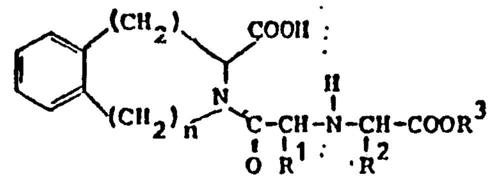

| | n | $R^1$ | $R^2$ | $R^3$ | Methode | NMR |
|---|---|---|---|---|---|---|
| 264 | 1 | $H_2N-CH_2$ | 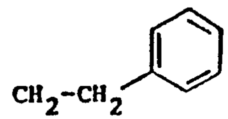 | H | B | 7.2bs (9H); 2.7-2.3m (4H); 1.7m (2H) |
| 265 | 1 | $CH_3$ | 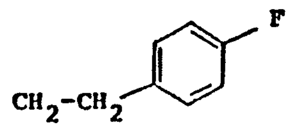 | $C_2H_5$ | B | 7.2-6.8m (8H); 2.7m (2H); 1.7m (2H); 1.2d (3H) |
| 266 | 1 | $CH_3$ | 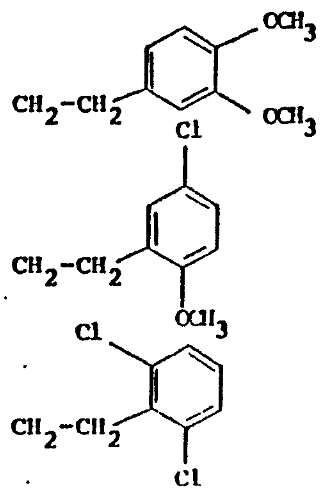 | $C_2H_5$ | B | 7.2-6.3m (7H); 3.9s (6H); 2.7m (2H) 1.7m (2H); 1.2d (3H) |
| 267 | 1 | $CH_3$ | | $C_2H_5$ | B | 7.3-6.4m (7H); 3.8s (3H); 2.6m (2H) 1.7m (2H); 1.2d (3H) |
| 268 | 1 | $CH_3$ | | $C_2H_5$ | B | 7.4-6.8m (7H); 2.7m (2H); 1.7m (2H) 1.2d (3H) |

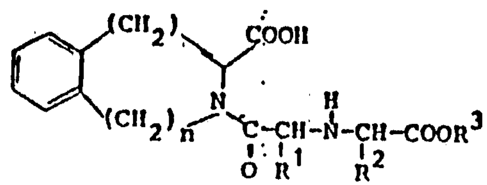

| | n | $R^1$ | $R^2$ | $R^3$ | Methode | NMR |
|---|---|---|---|---|---|---|
| 269 | 1 | 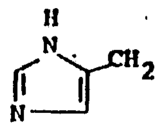 | 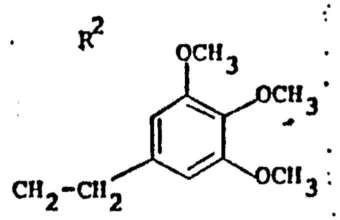 | $C_2H_5$ | B | 13.1s (1H); 7.5-6.2m (8H); 3.9s (9H); 2.8-2.3m (4H); 1.7m (2H) |
| 270 | 1 | $CH_3$ | 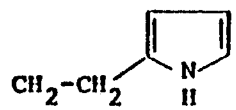 | $C_2H_5$ | B | 7.3-6.0m (7H); 2.8m (2H); 1.7m (2H); 1.2d (3H) |
| 271 | 1 | $CH_3$ | 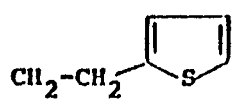 | $C_2H_5$ | B | 7.3-6.9m (7H); 2.9m (2H); 1.7m (2H) 1.2d (3H) |
| 272 | 1 | $H_2N-(CH_2)_4$ | 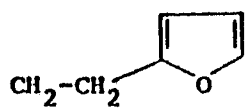 | H | B | 7.4-6.3m (7H); 2.8-2.3m (4H); 1.8-1.3m (8H) |
| 273 | 1 | $CH_3$ | 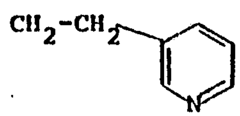 | $C_2H_5$ | B | 8.6-7.1m (8H); 2.9m (2H); 1.7m (2H); 1.2d (3H) |

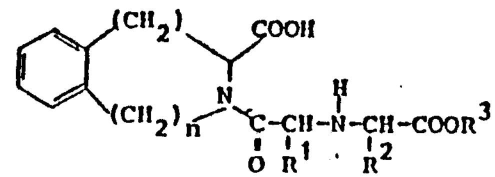

| | n | R[1] | R[2] | R[3] | Methode | NMR |
|---|---|---|---|---|---|---|
| 274 | 1 | $CH_3$ | 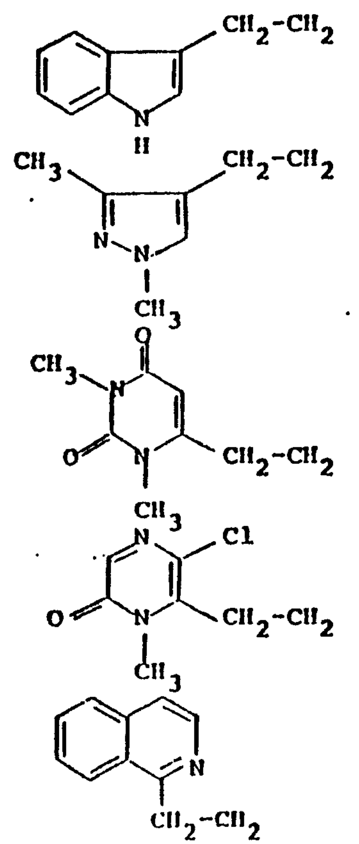 | $C_2H_5$ | B | 7.8-6.5m (10H); 2.9m (2H); 1.7m (2H); 1.2d (3H) |
| 275 | 1 | $CH_3$ | | $C_2H_5$ | B | 7.4-7.0m (5H); 3.9-3.0 m+s (7H); 2.8m (2H); 2.0s (3H); 1.7m (2H); 1.2d (3H) |
| 276 | 1 | $CH_3$ | | $C_2H_5$ | B | 5.2s (1H); 3.2 2s (6H); 2.9m (2H); 1.7m (2H); 1.2d (3H) |
| 277 | 1 | $CH_3$ | | $C_2H_5$ | B | 8.1s (1H); 3.2s (3H); 2.9m (2H); 1.7m (2H); 1.2d (3H) |
| 278 | 1 | $CH_3$ | | $C_2H_5$ | B | 8.5-7.5m (6H); 3.1m (2H); 1.8m (2H); 1.2d (3H); |

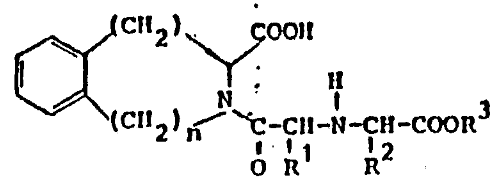

| | n | $R^1$ | $R^2$ | $R^3$ | Methode | NMR |
|---|---|---|---|---|---|---|
| 279 | 1 | $CH_3$ | 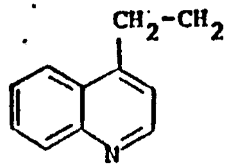 | $C_2H_5$ | B | 8.8-7.4m (6H); 3.0m (2H); 1.7m (2H); 1.2d (3H) |
| 280 | 1 | $CH_3$ | 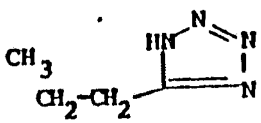 | $C_2H_5$ | B | 13.0s (1H); 2.9m (2H); 1.8m (2H); 1.2d (3H) |
| 281 | 1 | $CH_3$ | 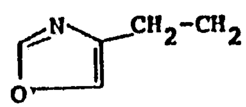 | $C_2H_5$ | B | 7.9-7.4 2s (2H); 2.8m (2H); 1.7m (2H); 1.2d (3H) |
| 282 | 1 | $CH_3$ | 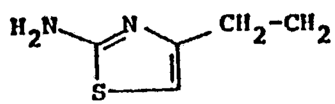 | $C_2H_5$ | B | 8.0s (1H); 2.8m (2H); 1.7m (2H); 1.2d (3H) |
| 283 | 1 | $CH_3$ | 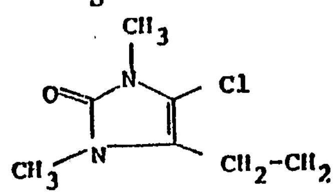 | $C_2H_5$ | B | 3.2 2s (6H); 3.1m (2H); 1.8m (2H) 1.2d (3H) |

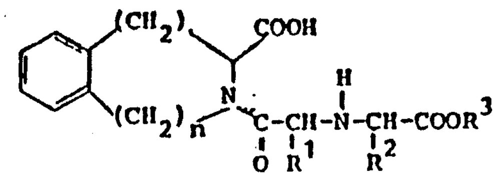

| | n. | $R^1$ | $R^2$ | $R^3$ | Methode | NMR |
|---|---|---|---|---|---|---|
| 284 | 1 | $CH_3$ | 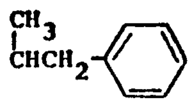 | $C_2H_5$ | B | 7.2bs (9H); 2.7m (2H); 2.2m (1H); 1.2d (3H); 1.0d (3H) |
| 285 | 1 | $H_2N-(CH_2)_4$ | 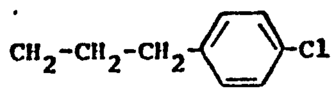 | H | B | 7.4-7.0m (8H); 2.7-2.3m (4H); 1.8-1.3m (10H) |
| 286 | 1 | $CH_3$ | 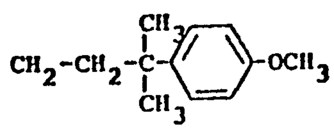 | $C_2H_5$ | B | 7.3-6.3m (8H); 3.9s (3H); 1.9-1.2m (4H); 1.2d (3H); 1.0s (6H) |
| 287 | 1 | 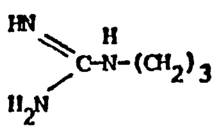 | 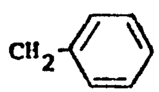 | H | B | 7.2bs (9H); 2.9-2.6m (4H); 1.7-1.3 m (4H); |
| 288 | 1 | H | 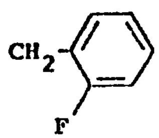 | H | B | 7.4-6.9m (8H); 3.9-3.0m (5H); 2.7m (2H) |

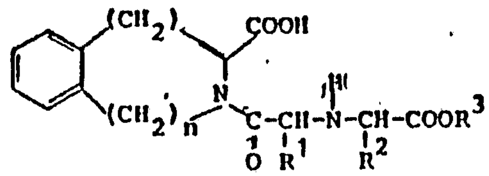

| | n | $R^1$ | $R^2$ | $R^3$ | Methode | NMR |
|---|---|---|---|---|---|---|
| 289 | 1 | H | 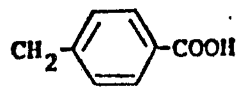 | H | B | 7.8-7.0m (8H); 3.9-3.0m (5H); 2.7m (2H) |
| 290 | 1 | H | 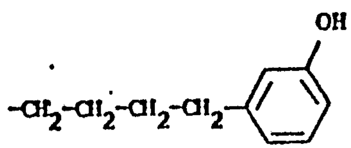 | H | B | 7.3-6.5m (8H); 3.9-3.0m (5H); 2.7m (2H); 1.8-1.3m (6H) |
| 291 | 1 | $CH_3$ | 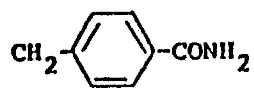 | H | B | 7.8-7.0m (8H); 2.8m (2H); 1.2d (3H) |
| 292 | 1 | $CH_3$ | 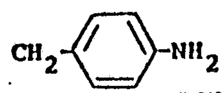 | H | B | 7.2-6.4m (8H); 2.7m (2H); 1.2d (3H) |
| 293 | 1 | $CH_3$ | 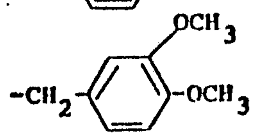 | H | B | 7.2-6.3m (7H); 3.9s (6H); 2.7m (2H); 1.2d (3H) |

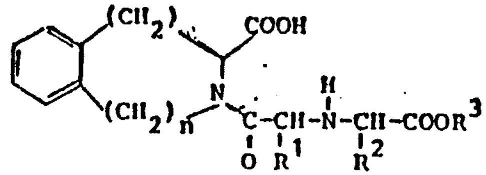

| | n | $R^1$ | $R^2$ | $R^3$ | Methode | NMR |
|---|---|---|---|---|---|---|
| 294 | 1 | $(CH_3)_2CH-CH_2$ | 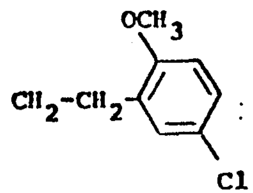 | $C_2H_5$ | B | 7.2-6.4m (7H); 3.8s (3H); 2.8m (2H); 2.0-1.5m (5H); 0.9d (6H) |
| 295 | 1 | $CH_3$ | 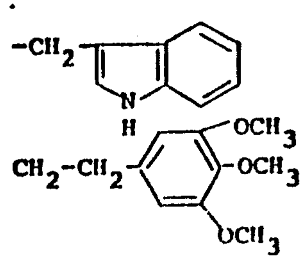 | $C_2H_5$ | B | 7.8-6.5m (10H); 2.9m (2H); 1.2d (3H) |
| 296 | 1 | $(CH_3)_2CH-CH_2$ | | H | B | 7.2-6.2m (6H); 3.9s (9H); 2.7m (2H); 1.9-1.3m (5H); 0.9d (6H) |
| 297 | 1 | $CH_3$ | 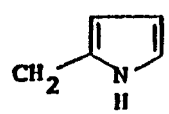 | H | B | 7.3-6.0m (7H); 2.8m (2H); 1.2d (3H) |
| 298 | 1 | $CH_3$ | 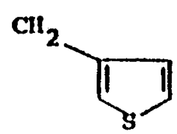 | H | B | 7.3-6.9m (7H); 2.9m (2H); 1.2d (3H) |

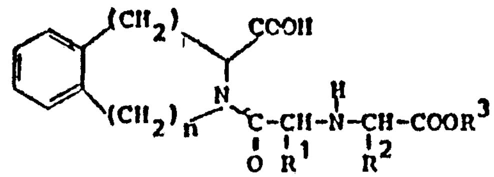

| | n | $R^1$ | $R^2$ | $R^3$ | Methode | NMR |
|---|---|---|---|---|---|---|
| 299 | 1 | $CH_3$ | 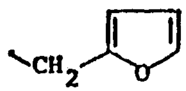 | H | B | 7.4-6.3m (7H); 2.8m (2H); 1.2d (3H) |
| 300 | 1 | $CH_3$ | 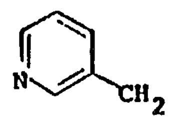 | H | B | 8.6-7.1m (8H); 2.9m (2H); 1.2d (3H) |
| 301 | 1 | $CH_3$ | 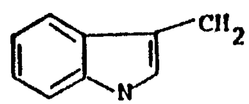 | H | B | 7.8-6.5m (10H); 2.9m (2H); 1.2d (3H) |
| 302 | 1 | $(CH_3)_2CH-CH_2$ | 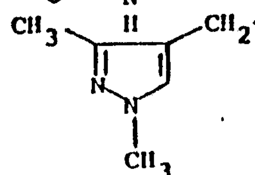 | $C_2H_5$ | B | 7.4-7.0m (5H); 3.9-3.0 m+s (7H); 2.8m (2H); 2.0s (3H); 1.8-1.3m (3H); 1.0d (6H) |
| 303 | 1 | H | 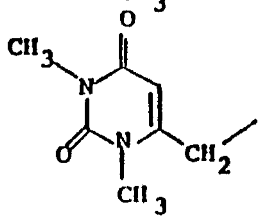 | $C_2H_5$ | B | 5.2s (1H); 3.2s (6H); 2.9m (2H) |

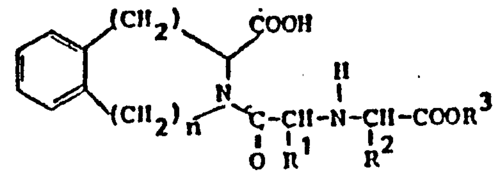

| | n | $R^1$ | $R^2$ | $R^3$ | Methode | NMR |
|---|---|---|---|---|---|---|
| 304 | 1 | $CH_3$ | 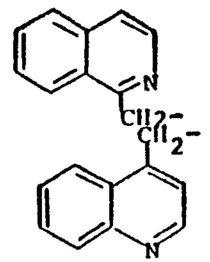 | H | B | 8.5-7.5m (6H); 3.1m (2H); 1.2d (3H) |
| 305 | 1 | $CH_3$ | | H | B | 8.8-7.4m (6H); 3.0m (2H); 1.2d (3H) |
| 306 | 1 | $CH_3$ | 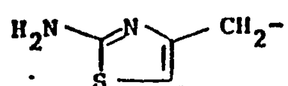 | H | B | 8.0s (1H); 2.8m (2H); 1.2d (3H) |
| 307 | 1 | $CH_3$ | 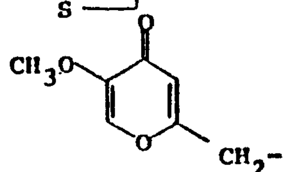 | H | B | 8.1s (1H); 6.4s (1H); 3.7s (3H); 2.9m (2H); 1.2d (3H) |
| 308 | 1 | $(CH_3)_2CH-CH_2$ | 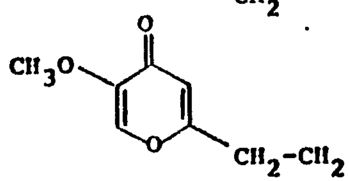 | H | B | 8.1s (1H); 6.4s (1H); 3.7s (3H); 2.9m (2H); 1.8-1.3m (5H); 1.0d (6H) |

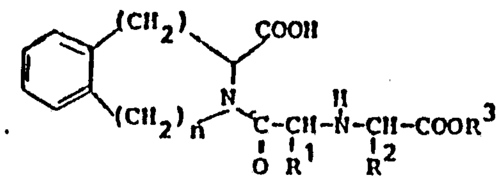

| | n | $R^1$ | $R^2$ | $R^3$ | Methode | NMR |
|---|---|---|---|---|---|---|
| 309 | 1 | $CH_3$ | 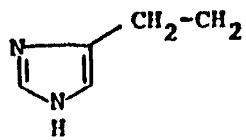 | $C_2H_5$ | B | 7.7-7.1m (6H); 2.9m (2H); 1.7m (2H) 1.2d (3H) |
| 310 | 1 | $CH_3$ | 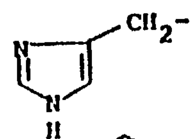 | $C_2H_5$ | B | 7.7-7.1m (6H); 2.9m (2H), 1.2d (3H) |
| 311 | 0 | $CH_3$ | 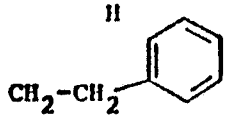 | $C_2H_5$ | B | 7.2-6.5m (9H); 2.7m (2H); 1.7m (2H); 1.2d (3H) |
| 312 | 0 | $CH_3$ | 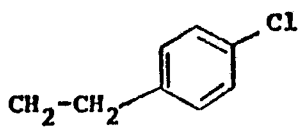 | H | B | 7.5-6.5m (8H); 2.7m (2H); 1.7m (2H); 1.2d (3H) |
| 313 | 0 | $CH_3$ | 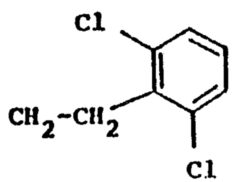 | H | B | 7.6-6.5m (7H); 2.8m (2H); 1.7m (2H); 1.2d (3H) |

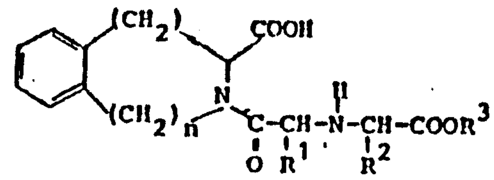

| | n | $R^1$ | $R^2$ | $R^3$ | Methode | NMR |
|---|---|---|---|---|---|---|
| 314 | O | $CH_3$ | 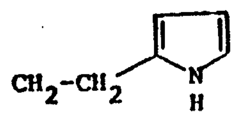 | H | B | 7.3-6.0m (7H); 2.8m (2H); 1.7m (2H); 1.2d (3H) |
| 315 | O | $CH_3$ | 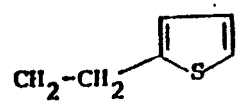 | H | B | 7.3-6.5m (7H); 2.9m (2H); 1.7m (2H); 1.2d (3H) |
| 316 | O | $CH_3$ | 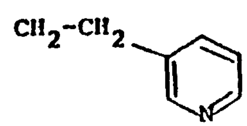 | H | B | 8.6-6.5m (8H); 2.9m (2H); 1.7m (2H); 1.2d (3H) |
| 317 | O | $CH_3$ | 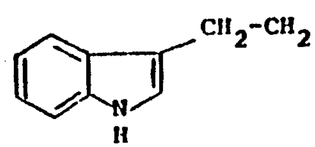 | $C_2H_5$ | B | 7.8-6.5m (10H); 2.9m (2H); 1.7m (2H); 1.2d (3H) |
| 318 | O | $CH_3$ | 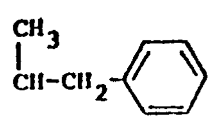 | $C_2H_5$ | B | 7.2-6.5m (9H); 2.9m (2H); 1.8m (1H); 1.2d (3H); 1.1d (3H) |

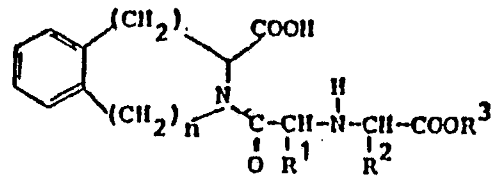

| | n | $R^1$ | $R^2$ | $R^3$ | Methode | NMR |
|---|---|---|---|---|---|---|
| 319 | 0 | $CH_3$ | 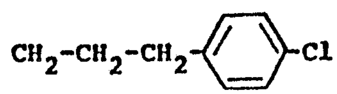 | $C_2H_5$ | B | 7.4-6.5m (8H); 2.7-2.3m (2H); 1.8-1.3m (4H); 1.2d (3H) |
| 320 | 0 | $CH_3$ | 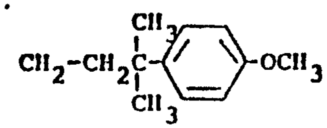 | $C_2H_5$ | B | 7.3-6.3m (8H); 3.9s (3H); 1.9-1.2m (4 1.2d (3H); 1.0s (6H) |
| 321 | 0 | $CH_3$ | 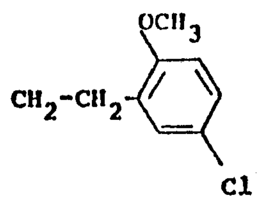 | $C_2H_5$ | B | 7.2-6.3m (7H); 3.9s (3H); 2.7m (2H); 1.8m (2H); 1.2d (3H) |
| 322 | 0 | $CH_3$ | 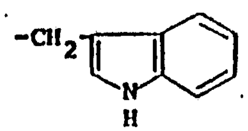 | $C_2H_5$ | B | 7.8-6.5m (10H); 2.9m (2H); 1.2d (3H) |
| 323 | 0 | $CH_3$ | 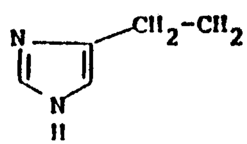 | $C_2H_5$ | B | 7.7-6.5m (6H); 2.9m (2H); 1.7m (2H); 1.2d (3H) |

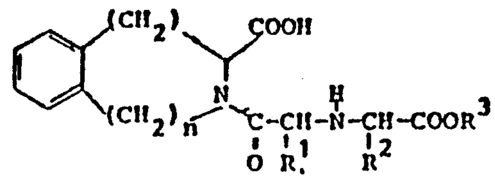

| | n | $R^1$ | $R^2$ | $R^3$ | Methode | NMR |
|---|---|---|---|---|---|---|
| 324 | 0 | $CH_3$ | 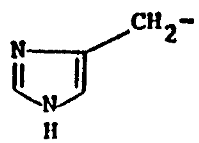 | $C_2H_5$ | B | 7.7-6.5m (6H); 2.9m (2H); 1.2d (3H) |
| 325 1 | 1 | $CH_3$ | $(CH_3)_2-CH-CH_2-CH_2-CH_2$ | $C_2H_5$ | A | 1.9-1.3m (7H); 1.2d (3H); 0.9d (6H) |
| 326 | 1 | $H_2N-(CH_2)_4$ | $(CH_3)_2CH-CH_2-CH_2$ | $C_2H_5$ | B | 2.4m (2H); 1.9-1.3m (11H); 0.9d (6H) |
| 327 | 1 | $H_2N-(CH_2)_3$ | $(CH_3)_2CH-CH_2$ | $C_2H_5$ | B | 2.4m (2H); 1.9-1.3m (7H); 0.9d (6H) |
| 328 | 0 | $FCH_2$ | $(CH_3)_2CH$ | $C_2H_5$ | B | 4.3dd (2H); 1.9m (1H); 0.9d (6H) |

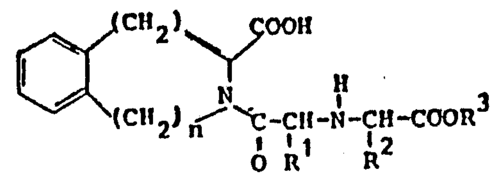

| | n | $R^1$ | $R^2$ | $R^3$ | Methode | NMR |
|---|---|---|---|---|---|---|
| 329 | 1 | $CH_3-CH_2-CH(CH_3)-$ | 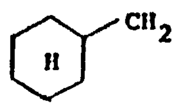 | H | A | 1.9-1.2m (16H); 1.0 d+t (6H) |
| 330 | 1 | $CH_3$ | 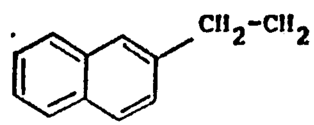 | $C_2H_5$ | A | 7.8-7.0m (11H); 2.7m (2H); 1.5m (2H); 1.2d (3H) |
| 331 | 1 | $CH_3$ | 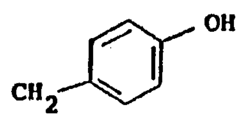 | H | B | 7.3-6.5m (8H); 2.8m (2H); 1.2d (3H) |
| 332 | 1 | $CH_3$ | 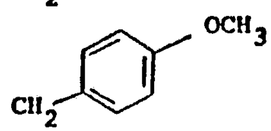 | H | B | 7.3-6.4m (8H); 3.9s (3H); 2.9m (2H); 1.2d (3H) |
| 333 | 1 | $CH_3$ | 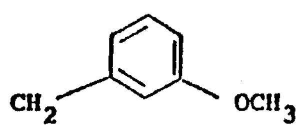 | $C_2H_5$ | B | 7.6-6.4m (8H); 3.8s (3H); 2.9m (2H); 1.2d (3H) |

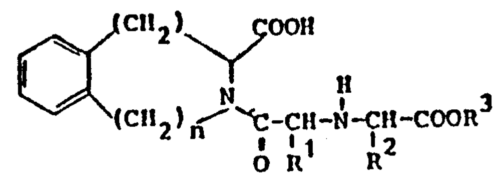

| | n | $R^1$ | $R^2$ | $R^3$ | Methode | NMR |
|---|---|---|---|---|---|---|
| 334 | 1 | $CH_3$ | 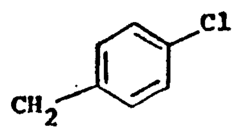 | $C_2H_5$ | B | 7.6-7.0m (8H); 2.9m (2H); 1.2d (3H) |
| 335 | 1 | $CH_3$ | 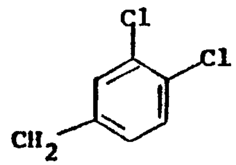 | H | B | 7.8-7.0m (7H); 2.9m (2H); 1.2d (3H) |
| 336 | 1 | $CH_3$ | 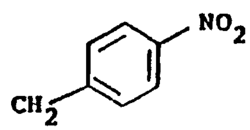 | H | B | 8.3-7.0m (8H); 2.9m (2H); 1.2d (3H) |
| 337 | 1 | $CH_3$ | 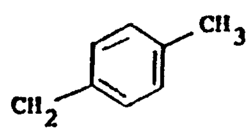 | $C_2H_5$ | B | 7.4-7.0m (8H); 2.9m (2H); 2.3s (3H); 1.2d (3H) |
| 338 | 1 | $CH_3$ | 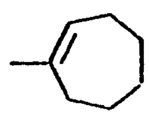 | H | A | 5.3m (1H); 2.3m (4H); 1.8-1.3m (6H); 1.2d (3H) |

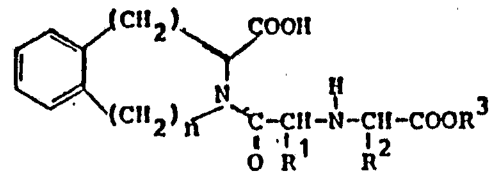

| | n | $R^1$ | $R^2$ | $R^3$ | Methode | NMR |
|---|---|---|---|---|---|---|
| 339 | 1 | $CH_3$ | 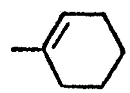 | H | A | 5.4m (1H); 2.3m (4H); 1.8-1.4m (4H); 1.2d (3H) |
| 340 | 1 | $CH_3$ | 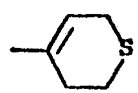 | H | A | 5.5m (1H); 3.9-3.0m (6H); 2.5-2.1m (4H) 1.2d (3H) |
| 341 | 1 | $CH_3$ | 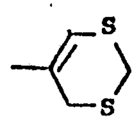 | H | A | 5.9s (1H); 3.9-3.0m (8H); 1.2d (3H) |
| 342 | 1 | $CH_3$ | 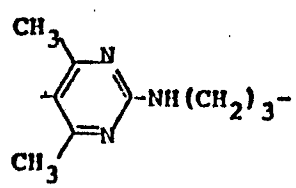 | $C_2H_5$ | A | 7.4s (1H); 2.5m (2H); 2.3s (6H); 1.8-1.2m (4H) |

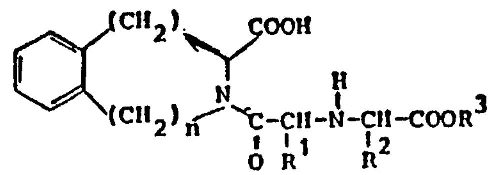

| | n | $R^1$ | $R^2$ | $R^3$ | Methode | NMR |
|---|---|---|---|---|---|---|
| 343 | 1 | $CH_3$ | 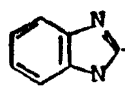$-CH_2CH_2-$ | $C_2H_5$ | A | 7.5-6.8m (8H); 2.8m (2H); 1.5m (2H) |
| 344 | 1 | $CH_3$ | 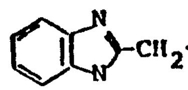$-CH_2-CH_2-$ | H | A | 7.5-6.8m (8H); 2.8m (2H); 1.5m (2H) |

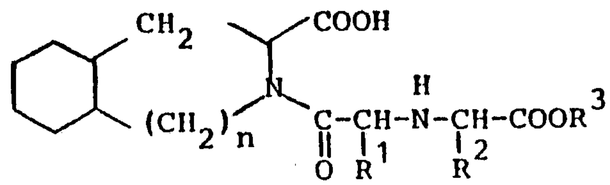

| Nr. | n | $R^1$ | $R^2$ | $R^3$ | Methode | NMR |
|---|---|---|---|---|---|---|
| 345 | 1 | $CH_3$ | 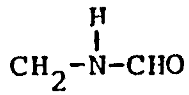 | $C_2H_5$ | A | 8,3s (1H); 2,9 m ((2H); 1,2 d (3H) |
| 346 | 1 | $CH_3$ | 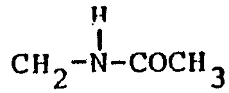 | $C_2H_5$ | A | 2,9 m (2H); 2,1 s (3H); 1,2 d (3H) |
| 347 | 1 | $CH_3$ | 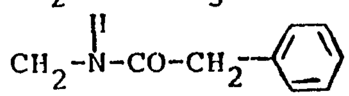 | $C_2H_5$ | A | 3,9-3,0 m (5H); 2,9 m (2H); 1,2 d (3H) |
| 348 | 1 | H | 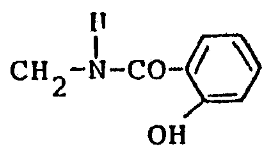 | $C_2H_5$ | A | 7,2-6,8 m (4H); 3,9-3,0m (4H); 2.9 m (2H) |
| 349 | 1 | H | 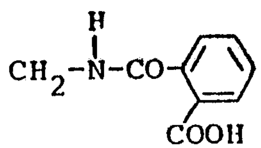 | $C_2H_5$ | A | 7.9 -7.4m (4H); 3.9-3.0m (4H); 2.9 m (2H) |
| 350 | 1 | $CH_3$ | 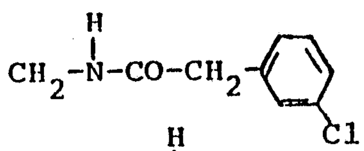 | $C_2H_5$ | A | 7.65-7.1m (4H); 3.9-3.0m (5H); 2.9m (2H); 1.2 d (3H) |
| 351 | 1 | $CH_3$ | 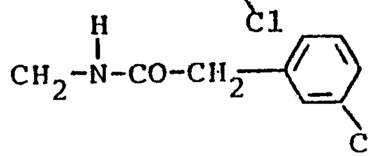 | H | A | 7.65-7.1m (4H); 3.9-3.0m (5H); 2.9m (2H); 1.2 d (3H) |

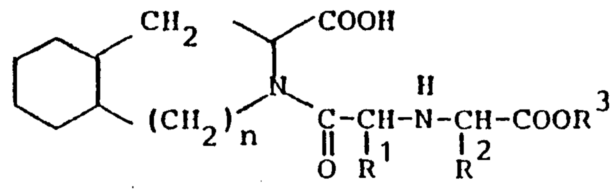

| Nr. | n | $R^1$ | $R^2$ | $R^3$ | Methode | NMR |
|---|---|---|---|---|---|---|
| 352 | 1 | $CH_3$ | 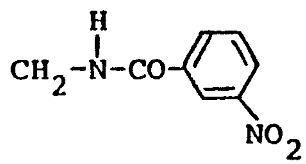 | $C_2H_5$ | A | 8.2-7.6m (4H); 2.9m (2H); 1.2d (3H) |
| 353 | 1 | $CH_3$ | 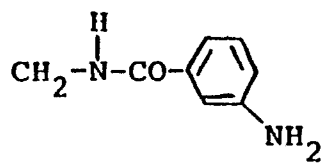 | $C_2H_5$ | A | 7.2-6.8m (4H); 2.9m (2H); 1.2d (3H) |
| 354 | 1 | 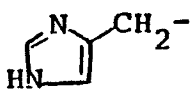 | 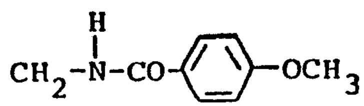 | $C_2H_5$ | A | 13.0s (1H); 7.5-7.0m (6H); 3.9s (3H); 2.9-2.6m (4H) |
| 355 | 1 | $CH_3$ | 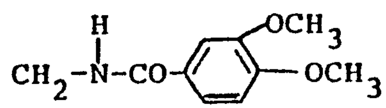 | $C_2H_5$ | A | 7.5-6.9m (3H); 3.9s (6H); 2.9m (2H); 1.2d (3H) |
| 356 | 1 | $CH_3$ | 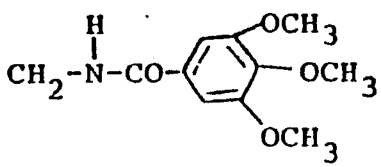 | $C_2H_5$ | A | 7.3-6.8m (2H); 3.9s (9H); 2.9m (2H) 1.2d (3H) |
| 357 | 1 | $CH_3$ | 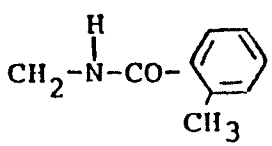 | $C_2H_5$ | A | 7.6-7.0m (4H); 2.9m (2H) 2.4s (3H); 1.2d (3H) |

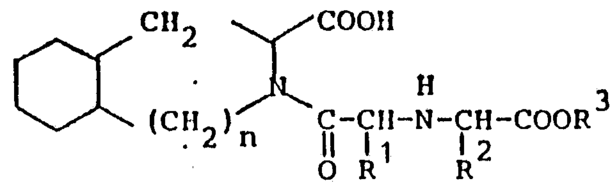

| Nr. | n | $R^1$ | $R^2$ | $R^3$ | Methode | NMR |
|---|---|---|---|---|---|---|
| 358 | 1 | $CH_3$ | 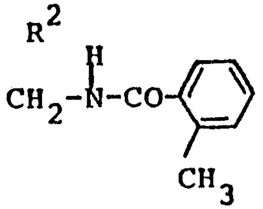 | H | A | 7.4-7.0m (4H); 2.9m (2H); 2.4s (3H); 1.2d (3H) |
| 359 | 1 | $CH_3$ | $CH_2-CH_2-N(CH_3)_2$ | $C_2H_5$ | A | 2.4m (2H); 2.2s (6H); 2.1-1.0m (14H) 1.2d (3H) |
| 360 | 1 | $CH_3$ | 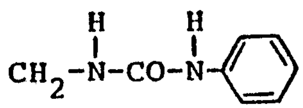 | $C_2H_5$ | A | 7.3-7.0m (5H); 2.9m (2H); 1.2d (3H) |
| 361 | 1 | $CH_3$ | 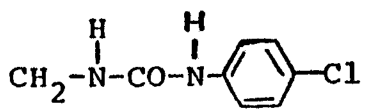 | $C_2H_5$ | A | 8.0-7.0m (4H); 2.9m (2H); 1.2d (3H) |
| 362 | 1 | $CH_3$ | 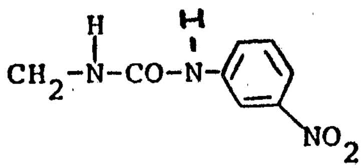 | $C_2H_5$ | A | 8.3-7.1m (4H); 2.9m (2H); 1.2d (3H) |
| 363 | 1 | $(CH_3)_2CH-CH_2$ | 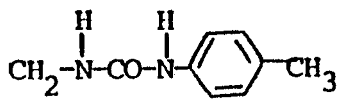 | $C_2H_5$ | A | 7.4-7.0m (4H); 2.9m (2H); 2.3s (3H) 1.0d (6H) |
| 364 | 1 | $CH_3$ | 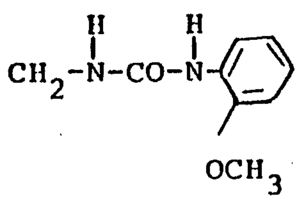 | $C_2H_5$ | A | 7.3-6.7m (4H); 3.8s (3H); 2.9m (2H); 1.2d (3H) |

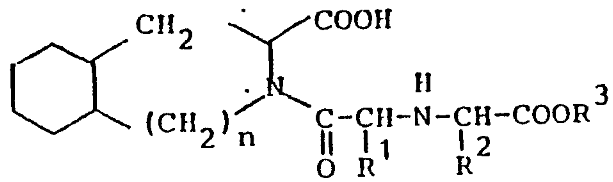

| Nr. | n | $R^1$ | $R^2$ | $R^3$ | Methode | NMR |
|---|---|---|---|---|---|---|
| 365 | 1 | $CH_3$ | 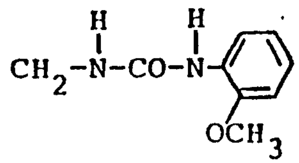 | $C_2H_5$ | A | 7.3-6.7m (4H); 3.8s (3H); 2.9m (2H); 1.2d (3H) |
| 366 | 1 | 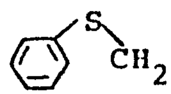 | 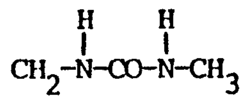 | $C_2H_5$ | A | 7.3-7.0m (5H); 3.0-2.6m+s (7H) |
| 367 | 1 | 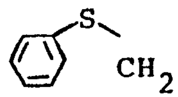 | 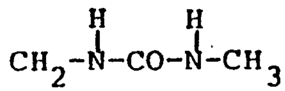 | H | A | 7.3-7.0m (5H); 3.0-2.6 m+s (7H) |
| 368 | 1 | $CH_2F$ | 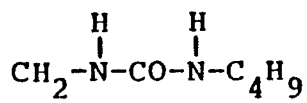 | $C_2H_5$ | A | 5.1-4.3m (4H); 2.9m (2H); 1.0t (3H) |
| 369 | 1 | $CH_3$ | 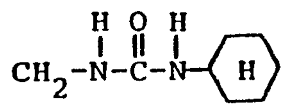 | $C_2H_5$ | A | 3.2-2.9m (3H); 2.4-1.0m (22H); 1.2d (3H) |
| 370 | 1 | $CH_3$ | 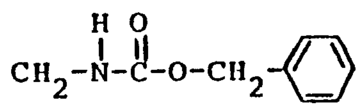 | $C_2H_5$ | A | 7.1s (5H); 5.0s (2H); 2.9m (2H); 1.2d (3H) |
| 371 | 1 | $CH_3$ | 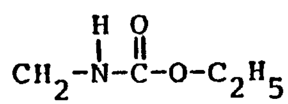 | $C_2H_5$ | A | 4.2q (4H); 2.9m (2H); 1.2t+d (9H) |
| 372 | 1 | $CH_3$ | 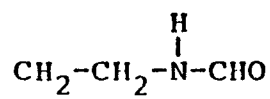 | $C_2H_5$ | G | 8.2-2s (1H); 2.9m (2H); 2.0-1.0m (14 H) |

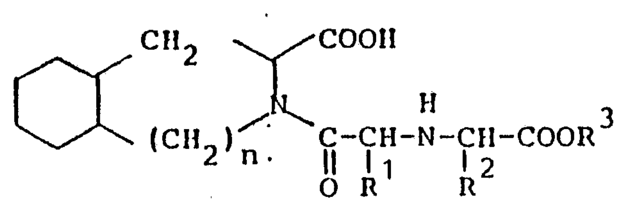

| Nr. | n | $R^1$ | $R^2$ | $R^3$ | Methode | NMR |
|---|---|---|---|---|---|---|
| 373 | 1 | H | $CH_2-CH_2-\overset{H}{N}-COCH_3$ | $C_2H_5$ | G | 3.9-3.0m (5H); 2.9m (2H); 2.1s (3H); 1.5m (2H) |
| 374 | 1 | $CH_3$ | 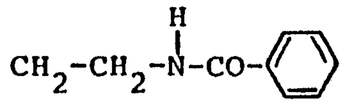 | $C_2H_5$ | G | 7.6-7.0m (5H); 2.9m (2H); 2.0-1.0m (14H) |
| 375 | 1 | $CH_3$ | 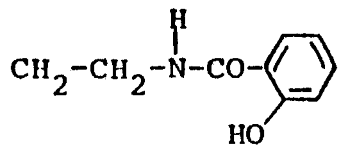 | $C_2H_5$ | G | 7.2-6.8m (4H); 2.9m (2H); 2.0-1.0m (14H) |
| 376 | 1 | 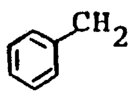 | 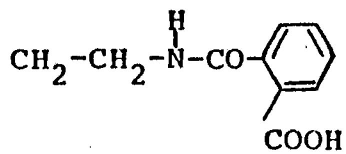 | $C_2H_5$ | G | 7.9-7.4m (9H); 2.9-2.7m (4H); 2.0-1.0m (14H) |
| 377 | 1 | $CH_3$ | 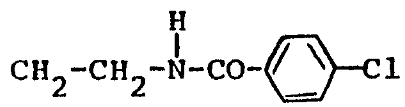 | $C_2H_5$ | G | 8.0-7.1m (4H); 2.9m (2H); 2.0-1.0m (14H) |
| 378 | 1 | $CH_3$ | 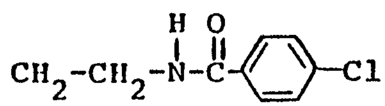 | H | G | 8.0-7.1m (4H); 2.9m (2H); 2.0-1.0m (14H) |
| 379 | 1 | $CH_3$ | 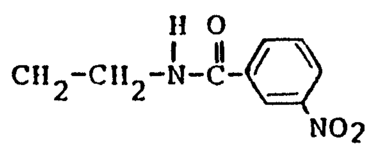 | $C_2H_5$ | G | 8.3-7.6 (4H); 2.9m (2H) 2.0-1.0m (14H) |

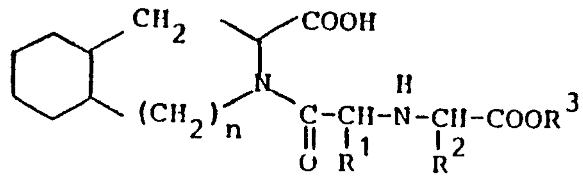

| | n | $R^1$ | $R^2$ | $R^3$ | Methode | NMR |
|---|---|---|---|---|---|---|
| 380 | 1 | $CH_3$ | 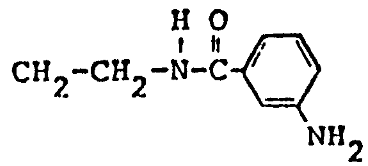 | $C_2H_5$ | G | 7.2-6.8m (4H); 2.9m (2H); 1.2d (3H); 2.0-1.0m (14H) |
| 381 | 1 | $CH_3$ | 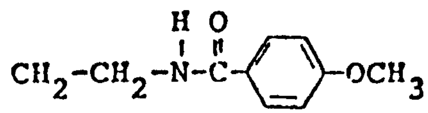 | $C_2H_5$ | G | 7.5-6.8m (4H); 3.8s (3H); 2.9m (2H); 1.2d (3H); 2.0-1.0m (14H) |
| 382 | 1 | $CH_3$ | 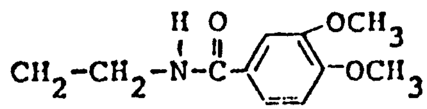 | $C_2H_5$ | G | 7.5-6.9m (3H); 3.9s (6H); 2.9m (2H); 1.2d (3H); 2.0-1.0m (14H) |
| 383 | 1 | 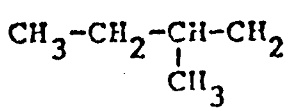 | 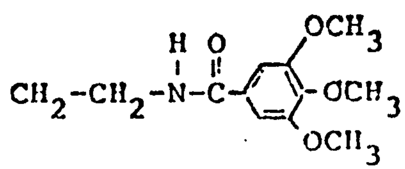 | $C_2H_5$ | G | 7.2-6.8m (6H); 3.9s (9H); 2.9m (2H); 2.0-1.0m (25H) |
| 384 | 1 | $CH_3$ | 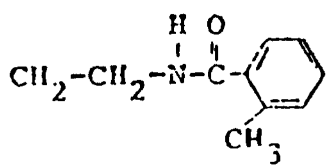 | $C_2H_5$ | G | 7.6-7.2m (4H); 2.9m (2H); 2.3s (3H); 1.2d (3H); 2.0-1.0m (14H) |

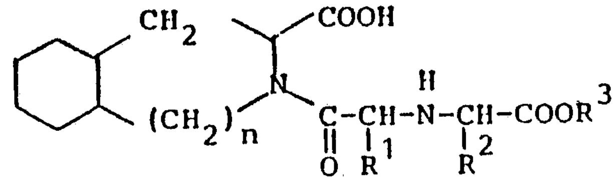

| | n | $R^1$ | $R^2$ | $R^3$ | Methode | NMR |
|---|---|---|---|---|---|---|
| 385 | 1 | $CH_3$ | 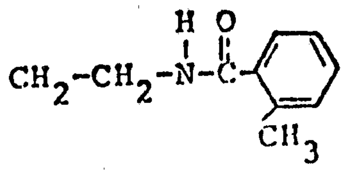 | H | G | 7.6-7.2m (4H); 2.9m (2H); 2.3s (3H); 1.2d (3H); 2.0-1.0m (14H) |
| 386 | 1 | $CH_3$ | $CH_2-CH_2-CH_2-N(C_2H_5)_2$ | $C_2H_5$ | G | 2.6-2.4m (6H); 2.0-1.0m (14H); 1.2d (3H), 0.9t (6H) |
| 387 | 1 | $CH_3$ | 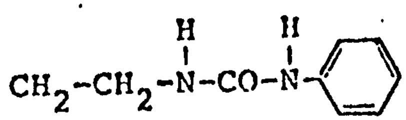 | $C_2H_5$ | G | 7.5-7.0m (5H); 2.9m (2H); 2.0-1.0m (14H); 1.2d (3H) |
| 388 | 1 | $CH_3$ | 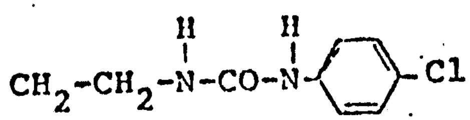 | $C_2H_5$ | G | 7.5-7.0m (4H); 2.9m (2H); 2.0-1.0m (14H); 1.2d (3H) |
| 389 | 1 | $CH_3$ | 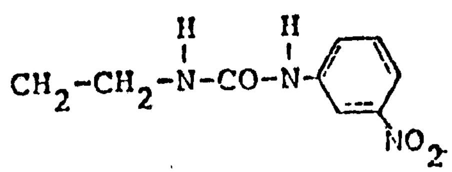 | $C_2H_5$ | G | 8.3-7.3m (4H); 2.9m (2H); 2.2-1.0m (14H); 1.2d (3H) |

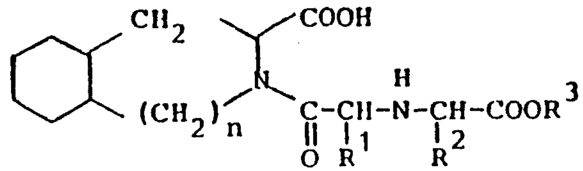

| | n | $R_1$ | $R_2$ | $R_3$ | Methode | NMR |
|---|---|---|---|---|---|---|
| 390 | 1 | $CH_3$ | 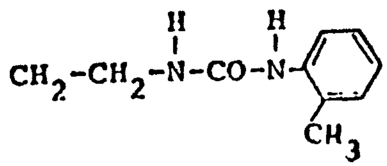 | $C_2H_5$ | G | 7.4-7.0m (4H); 2.9m (2H); 2.3s (3H); 2.1-1.0m (14H) 1.2d (3H) |
| 391 | 1 | $CH_3$ | 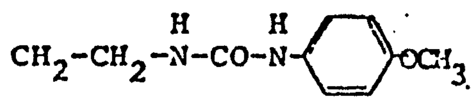 | $C_2H_5$ | G | 7.2-6.5m (4H); 3.9s (3H); 2.9m (2H); 2.0-1.0m (14H); 1.2d (3H) |
| 392 | 1 | $CH_3$ | 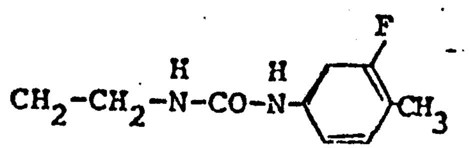 | $C_2H_5$ | G | 7.3-6.9m (3H); 2.9m (2H); 2.3s (3H); 2.0-1.0m (14H) 1.2d (3H) |

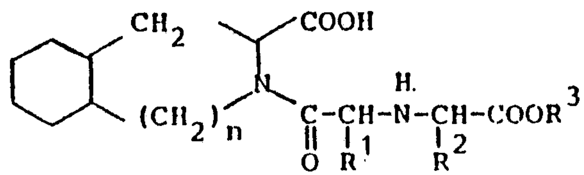

| | n | $R_1$ | $R_2$ | $R_3$ | Methode | NMR |
|---|---|---|---|---|---|---|
| 393 | 1 | $(CH_3)_2CH-CH_2$ | $CH_2-CH_2-\overset{H}{N}-CO-\overset{H}{N}-CH_3$ | $C_2H_5$ | G | 3.0-2.6m (5H); 2.0-1.0m (17H); 1.0d (6H) |
| 394 | 1 | $(CH_3)_2CH-CH_2$ | $CH_2-CH_2-\overset{H}{N}-CO-\overset{H}{N}-C_4H_9$ | $C_2H_5$ | G | 3.0-2.6m (4H); 2.0-1.0m (21H); 0.9+t(9H) |
| 395 | 1 | $(CH_3)_2CH-CH_2$ | $CH_2-CH_2-\overset{H}{N}-CO-\overset{H}{N}-C_4H_9$ | H | G | 3.0-2.6m (4H); 2.0-1.0m (21H); 0.9+t (9H) |
| 396 | 1 | $CH_2F$ | 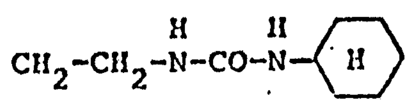 | $C_2H_5$ | G | 5.1-4.3m (4H); 2.9m (3H); 2.0-1.0m (24H) |
| 397 | 1 | $CH_3$ | 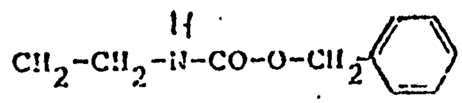 | $C_2H_5$ | G | 7.1s (5H); 5.0s (2H); 2.9m (2H); 2.0-1.0m (14H); 1.2d (3H) |

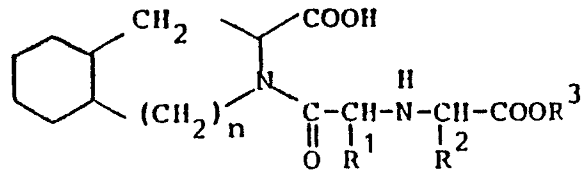

| | n | $R_1$ | $R_2$ | $R_3$ | Methode | NMR |
|---|---|---|---|---|---|---|
| 398 | 1 | $CH_3$ | $CH_2-CH_2-\overset{H}{N}-CO-O-C_2H_5$ | $C_2H_5$ | A | 4.2q (4H); 2.9m (2H); 2.0-1.0m (14H); 1.2+t (9H) |
| 399 | 1 | $CH_3$ | $CH_2-CH_2-\overset{H}{N}-CH_3$ | $C_2H_5$ | A | 2.4m (2H); 2.3s (3H); 2.0-1.0m (14H); 1.2d (3H) |
| 400 | 1 | $CH_3$ | 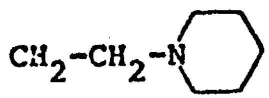 | $C_2H_5$ | A | 2.4m (6H); 2.0-1.0m (20H); 1.2d (3H) |
| 401 | 1 | $CH_3$ | 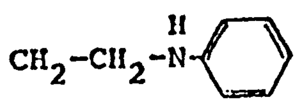 | $C_2H_5$ | A | 7.0-6.5m (5H); 2.5 - 1.0m (16H); 1.2d (3H) |
| 402 | 1 | $(CH_3)_2CH-CH_2$ | 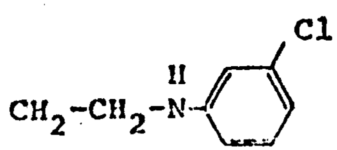 | $C_2H_5$ | A A | 7.2-6.6m (4H); 2.4 - 1.0m (19H); 1.0d (6H) |

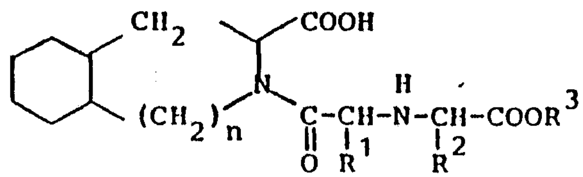

| | n | R1 | R2 | R3 | Methode | NMR |
|---|---|---|---|---|---|---|
| 403 | 1 | 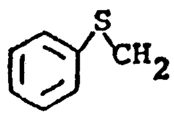 | 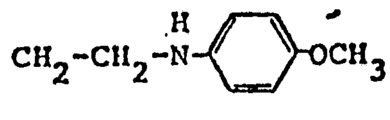 | $C_2H_5$ | A | 7.3-6.4m (9H); 2.5-2.2m (4H); 2.0-1.0m (14H) |
| 404 | 1 | $CH_2F$ | 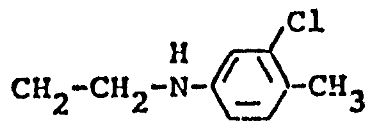 | $C_2H_5$ | A | 7.0-6.6m (3H); 5.1-4.3m (4H); 2.4m (2H); 2.3s (3H); 2.0-1.0m (14H) |
| 405 | 1 | $CH_3$ | 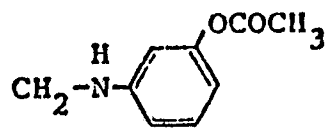 | $C_2H_5$ | A | 7.0-6.5m (4H); 2.5-1.0m (14H); 2.1s (3H); 1.2d (3H) |
| 406 | 1 | $CH_3$ | 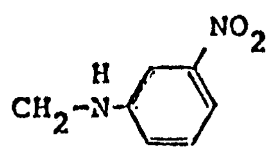 | $C_2H_5$ | A | 7.7-7.0m (4H); 2.5m (2H); 1.2d (3H) |
| 407 | 1 | $CH_3$ | 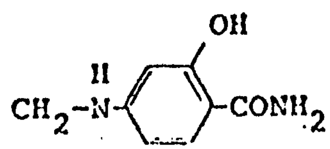 | $C_2H_5$ | A | 7.6-6.7m (3H); 2.4m (2H); 1.2d (3H) |

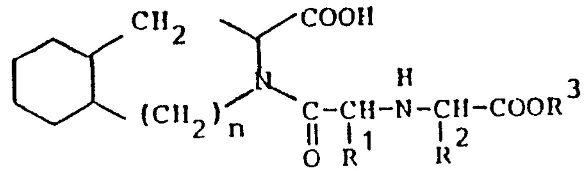

| | n | $R_1$ | $R_2$ | $R_3$ | Methode | NMR |
|---|---|---|---|---|---|---|
| 408 | 1 | $CH_3$ | 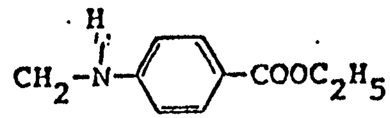 | $C_2H_5$ | A | 7.6-6.8m (4H); 4.2q (4H); 2.5m (2H); 1.2d+t (9H) |
| 409 | 1 | $CH_3$ | 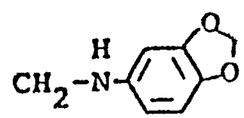 | $C_2H_5$ | A | 6.8-6.2m (3H); 5.0s (2H); 2.5m (2H); 1.2d (3H) |
| 410 | 1 | $CH_3$ | 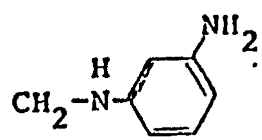 | $C_2H_5$ | A | 7.0-6.5m (8H); 2.5m (2H); 1.2d (3H) |
| 411 | 1 | $CH_3$ | $-CH_2-NH-CH_2-CH_2-COOC_2H_5$ | $C_2H_5$ | A | 4.2q (4H); 2.6-2.3m (6H); 1.2d+t (9H) |
| 412 | 1 | $CH_3$ | $CH_2-CH_2-CH_2-NH_2$ | H | C | 2.3m (2H); 2.0-1.0m (16H); 1.2d (3H) |

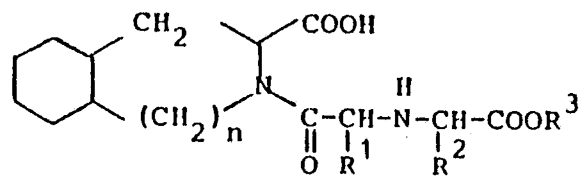

| | n | $R^1$ | $R^2$ | $R^3$ | Methode | NMR |
|---|---|---|---|---|---|---|
| 413 | 1 | $CH_3$ | $CH_2-NH-CH_2-CH_2-N(C_2H_5)_2$ | $C_2H_5$ | A | 2.6-1.0m (22H); 1.2d+t (9H) |
| 414 | 1 | $CH_3$ | 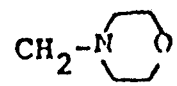 | $C_2H_5$ | A | 3.9-3.0m (12H); 2.3m (2H); 1.2d (3H) |
| 415 | 1 | $CH_3$ | 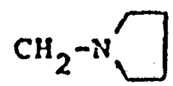 | $C_2H_5$ | A | 2.6-2.3m (6H); 2.1-1.0m (16H); 1.2d (3H) |
| 416 | 1 | $CH_3$ | $CH_2-NH-CH_2-CH_2-CONH_2$ | $C_2H_5$ | A | 2.6-2.2m (6H); 1.2d (3H) |
| 417 | 1 | $CH_3$ | $CH_2-CH_2-CH_2-N(CH_3)_2$ | $C_2H_5$ | A | 2.4m (2H); 2.2s (6H); 2.0 - 1.0 (16H); 1.2d (3H) |
| 418 | 1 | $CH_3$ | $CH_2-NH-CH_2-CONH_2$ | $C_2H_5$ | A | 3.9-3.0m (5H); 2.4m (2H); 1.2d (3H) |

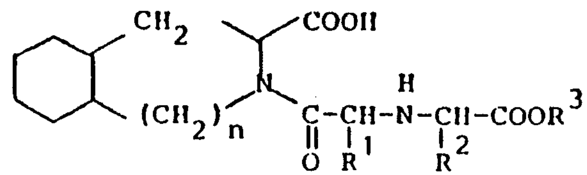

| | n | $R^1$ | $R^2$ | $R^3$ | Methode | NMR |
|---|---|---|---|---|---|---|
| 419 | 1 | $CH_3$ | $(CH_2)_5-NH_2$ | H | C | 2.3m (2H); 2.1-1.0m (20H); 1.2d (3H) |
| 420 | 1 | $H_2N-(CH_2)_4$ | $(CH_2)_4-NH_2$ | H | C | 2.4m (4H); 2.1-1.0m (24H) |
| 421 | 1 | $CH_3$ | $CH_2-CH_2-NH-CH_3$ | $C_2H_5$ | A | 2.4m (2H); 2.2s (3H); 2.0-1.0m (14H); 1.2d (3H) |
| 422 | 1 | $CH_3$ | $CH_2-NH-CH_3$ | $C_2H_5$ | A | 2.4m (2H); 2.1s (3H); 1.2d (3H) |
| 423 | 0 | $CH_3$ | 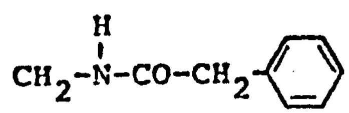 | $C_2H_5$ | A | 7.0s (5H); 4,5-3.0m (5H); 2.9m (2H); 1.2d (3H) |
| 424 | 0 | $CH_3$ | 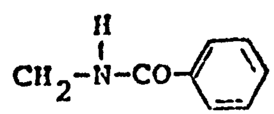 | $C_2H_5$ | A | 7.8-7.3m (5H); 2.9m (2H); 1.2d (3H) |

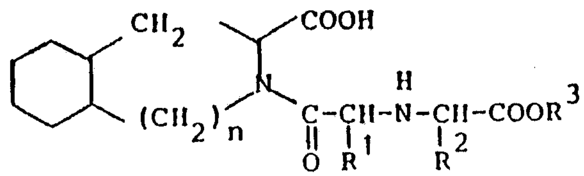

| | n | $R^1$ | $R^2$ | $R^3$ | Methode | NMR |
|---|---|---|---|---|---|---|
| 425 | O | $CH_3$ | 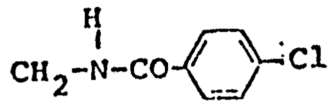 | $C_2H_5$ | A | 7.8-7.2m (4H); 2.9m (2H); 1.2d (3H) |
| 426 | O | $CH_3$ | 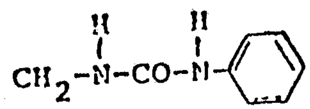 | $C_2H_5$ | A | 7.4-6.8m (5H); 2.9m (2H); 1.2d (3H) |
| 427 | O | $CH_3$ | 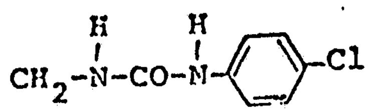 | $C_2H_5$ | A | 7.7-6.5m (4H); 2.9m (2H); 1.2d (3H) |
| 428 | O | $CH_3$ | 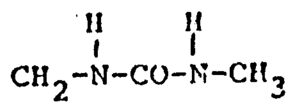 | $C_2H_5$ | A | 3.0-2.6m + s (5H); 1.2d (3H) |
| 429 | O | $CH_3$ | 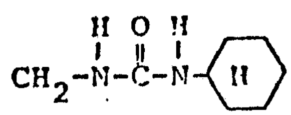 | $C_2H_5$ | A | 4,5-2.9m (6H); 2.4-1.0m (21H); 1.2d (3H) |
| 430 | O | $CH_3$ | 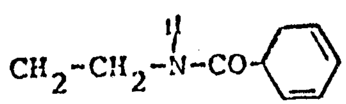 | $C_2H_5$ | A | 7.6-7.1m (5H); 2.9m (2H); 2.0-1.0m (13H); 1.2d (3H) |

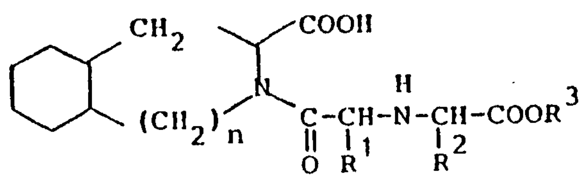

| | n | $R^1$ | $R^2$ | $R^3$ | Methode | NMR |
|---|---|---|---|---|---|---|
| 431 | O | $CH_3$ | 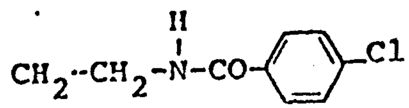 | $C_2H_5$ | A | 7.6-7.2m (4H); 2.9m (2H); 2.0-1.0m (13H); 1.2d (3H) |
| 432 | O | $CH_3$ | 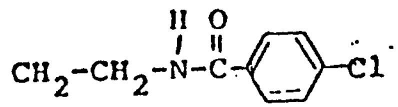 | H | A | 7.6-7.2m (4H); 2.9m (2H); 2.0-1.0m (13H); 1.2d(3H) |
| 433 | O | $CH_3$ | $CH_2-CH_2-CH_2-N(C_2H_5)_2$ | $C_2H_5$ | A | 2.6-2.4m (6H); 2.0 - 1.0m (15H); 1.2d (3H); 1.0t (6H) |
| 434 | O | $CH_3$ | 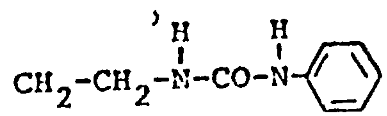 | $C_2H_5$ | G | 7.4-6.5m (5H); 2.9m (2H); 2.0-1.0m (13H); 1.2d (3H) |
| 435 | O | $CH_3$ | 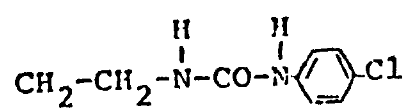 | $C_2H_5$ | G | 7.5-6.8m (4H); 2.9m (2H); 2.0-1.0m (13H); 1.2d(3H) |

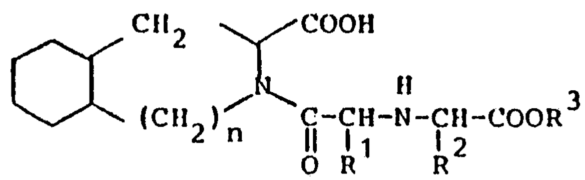

| | n | $R^1$ | $R^2$ | $R^3$ | Methode | NMR |
|---|---|---|---|---|---|---|
| 436 | O | $CH_3$ | 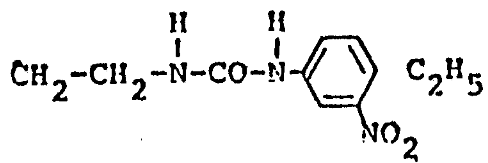 | $C_2H_5$ | G | 7.8-7.2m (4H); 2.9m (2H); 2.0 - 1.0m (13H); 1.2d (3H) |
| 437 | O | $CH_3$- | $CH_2-CH_2-NH-CO-NH-CH_3$ | $C_2H_5$ | G | 3.0-2.6m+s (5H); 2.0-1.0m (13H); 1.2d (3H) |
| 438 | O | $CH_3$ | 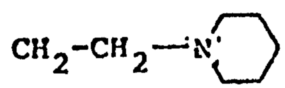 | $C_2H_5$ | A | 2.6-2.3m (6H); 2.0-1.0 m (19H) 1.2d (3H) |
| 439 | O | $CH_3$ | 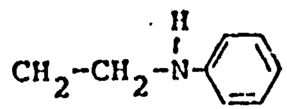 | $C_2H_5$ | A | 6.9-6.5m (5H); 2.4m (2H); 2.0-1.0m (13H); 1.2d (3H) |
| 440 | O | $CH_3$ | 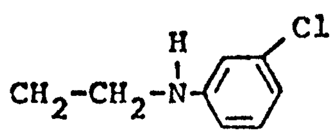 | $C_2H_5$ | A | 6.9-6.5m (4H); 2.4m (2H); 2.0-1.0m (13H); 1.2d (3H) |

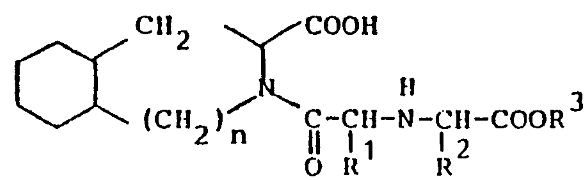

| | n | $R^1$ | $R^2$ | $R^3$ | Methode | NMR |
|---|---|---|---|---|---|---|
| 441 | 0 | $CH_3$ | 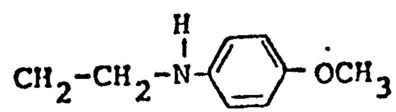 | $C_2H_5$ | A | 7.0-6.4m (4H); 3.9s (3H); 2.4m (2H); 2.0-1.0m (13H); 1.2d (3H) |
| 442 | 0 | $CH_3$ | $(CH_2)_5-NH_2$ | H | C | 2.4m (2H); 2.0-1.0m (19H); 1.2d (3H) |
| 443 | 0 | $CH_3$ | $(CH_2)_4-NH_2$ | H | C | 2.4m (2H); 2.0-1.0m (17H); 1.2d (3H) |
| 444 | 1 | $CH_3$ | 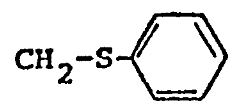 | $C_2H_5$ | A | 7.4-7.0m (5H); 2.7m (2H); 1.2d (3H) |
| 445 | 1 | $CH_3$ | 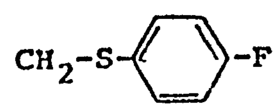 | $C_2H_5$ | A | 7.3-6.8m (4H); 2.7m (2H); 1.2d (3H) |

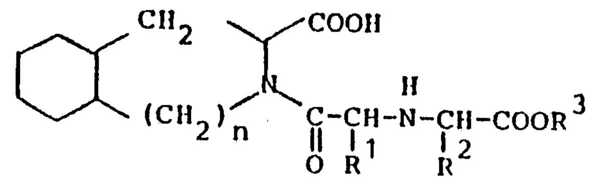

| | n | $R^1$ | $R^2$ | $R^3$ | Methode | NMR |
|---|---|---|---|---|---|---|
| 446 | 1 | $CH_3$ | 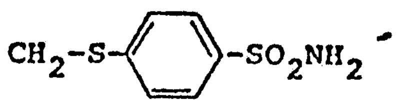 | $C_2H_5$ | A | 2.4m (2H); 1.2d (3H) |
| 447 | 1 | $(CH_3)_2CH-CH_2$ | 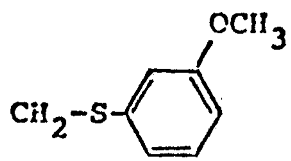 | $C_2H_5$ | A | 8.1-7.1m (4H); 6.5bs (2H); 2.7m (2H); 1.3d (3H) |
| 448 | 1 | 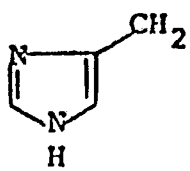 | 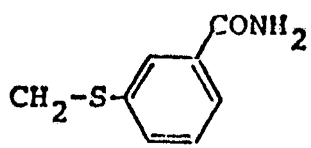 | $C_2H_5$ | A | 7.0-6.7m (4H); 3.8s (3H); 2.7m (2H); 2.0-1.0m (15H); 1.0d (6H) |
| 449 | 1 | 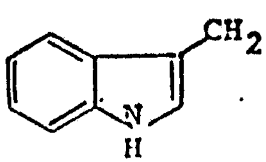 | 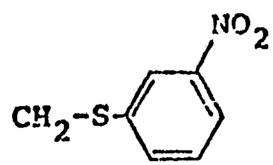 | $C_2H_5$ | A | 13.0s (1H); 7.8-6.8m (6H); 6.0bs (2H); 2.9-2.6m (4H) |

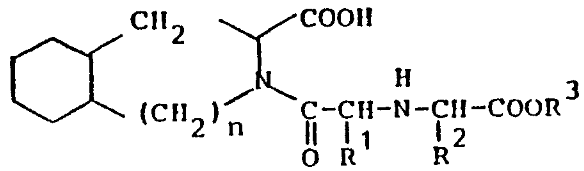

| | n | $R^1$ | $R^2$ | $R^3$ | Methode | NMR |
|---|---|---|---|---|---|---|
| 450 | 1 | 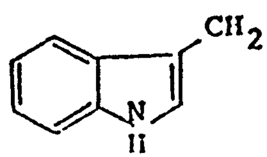 | 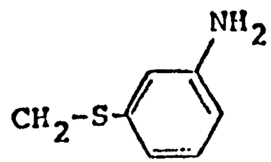 | $C_2H_5$ | A | 8.2m-6.4m (10H); 2.9-2.6m (4H) |
| 451 | 1 | $(CH_3)_2CH-CH_2$ | 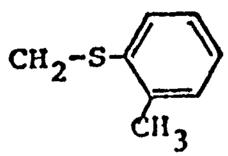 | $C_2H_5$ | A | 7.8-6.4m (10H); 2.9-2.6m (4H) |
| 452 | 1 | $CH_3$ | 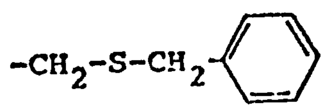 | $C_2H_5$ | A | 7.3-7.0m (4H); 2.7m (2H); 2.3s (3H); 2.0-1.0m (15H); 1.0d (6H) |
| 453 | 1 | 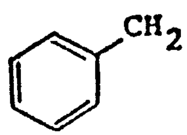 | $-CH_2-S-CH(CH_3)_2$ | $C_2H_5$ | A | 7.3-7.0m (5H); 3.9-3.0+s (5H); 2.4m (2H); 1.2d(3H) |

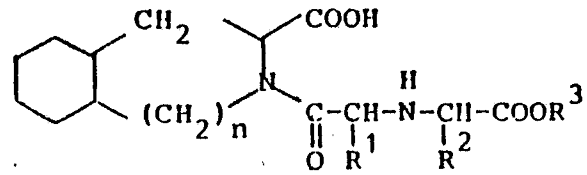

| | n | $R^1$ | $R^2$ | $R^3$ | Methode | NMR |
|---|---|---|---|---|---|---|
| 454 | 1 | $CH_3$ | $-CH_2-S-CH_2-CH_2-N(CH_3)_2$ | $C_2H_5$ | A | 7.1s (5H); 2.6-2.3m (5H); 0.9d (6H) |
| 455 | 1 | $CH_2F$ | $-CH_2-S-CH_2-CH_2-CONH_2$ | $C_2H_5$ | A | 2.6-2.2m+s (12H); 1,2d (3H) |
| 456 | 1 | $CH_3$ | $-CH_2-S-CH_2-CH_2-COOC_2H_5$ | $C_2H_5$ | A | 5.1-4.3m (4H); 2.5-2.2m (6H) |
| 457 | 1 | $CH_3$ | $-CH_2-S-CH_2-CH_2-OC_2H_5$ | $C_2H_5$ | A | 4.2q (4H); 2.5-2.2m (6H); 1.2d+t (9H) |
| 458 | 1 | $CH_3$ | $-CH_2-CH_2-S-$ 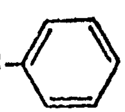 | $C_2H_5$ | A | 3.9-3.0q+m (7H); 2.4-2.2m (4H); 1.2d+t (6H) |

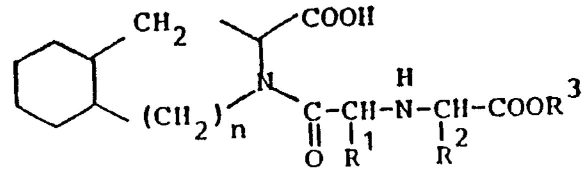

| | n | $R^1$ | $R^2$ | $R^3$ | Methode | NMR |
|---|---|---|---|---|---|---|
| 459 | 1 | $CH_3$ | $-CH_2-CH_2-S-$ 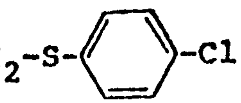 | $C_2H_5$ | A | 7.1s (5H); 2.7m (2H); 2.0-1.0m (14H); 1.2d (3H) |
| 460 | 1 | $CH_3$ | $-CH_2-CH_2-S-$ 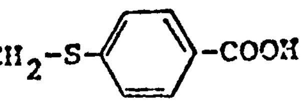 | $C_2H_5$ | A | 7.5-7.0m (4H); 2.7m (2H); 2.0-1.0m (14H); 1.2d (3H) |
| 461 | 1 | $CH_3$ | $CH_2-CH_2-S-$ 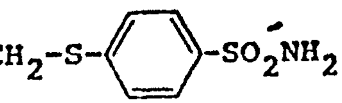 | $C_2H_5$ | A | 7.9-6.9m (4H); 2.7m (2H); 2.1-1.0m (14H); 1.2d (3H) |
| 462 | 1 | $CH_3$ | $CH_2-CH_2-S-$ 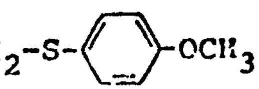 | $C_2H_5$ | A | 8.0-6.9m (4H); 6.5bs (2H); 2.6m (2H); 2.0-1.0m (14H); 1.2d (3H) |

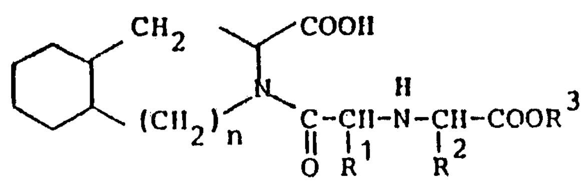

| | n | $R^1$ | $R^2$ | $R^3$ | Methode | NMR |
|---|---|---|---|---|---|---|
| 463 | 1 | $CH_3$ | 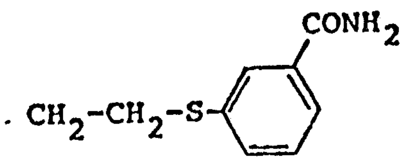 | $C_2H_5$ | A | 6.9-6.4m (4H); 3.8s (3H); 2.7m (2H); 2.1-1.0m (14H); 1.2d (3H) |
| 464 | 1 | $CH_3$ | 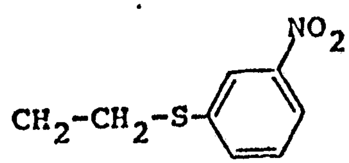 | $C_2H_5$ | A | 7.8-6.9m (4H); 6.0bs (2H); 2.6m (2H); 2.0 - 1.0m (14H); 1.2d (3H) |
| 465 | 1 | $H_2N-(CH_2)_4$ | 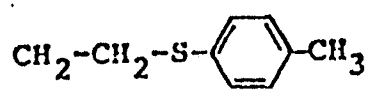 | $C_2H_5$ | B | 8.1-6.9m (4H); 2.7m (2H); 2.0-1.0m(14H); 1.2d (3H) |
| 466 | 1 | $CH_3$ | 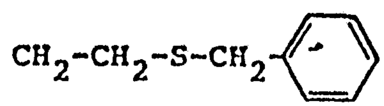 | $C_2H_5$ | A | 7.0-6.8m (4H); 2.7-2.3 m+s (7H); 2.0-1.0m (20H) |

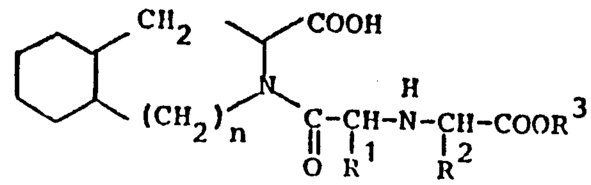

| | n | $R^1$ | $R^2$ | $R^3$ | Methode | NMR |
|---|---|---|---|---|---|---|
| 467 | 1 | $CH_3$ | $CH_2-CH_2-S-CH(CH_3)_2$ | $C_2H_5$ | A | 7.2s (5H); 3.9-3.0m+s (5H); 2.4m (2H); 2.0-1.0m 14H); 1.2d (3H) |
| 468 | 1 | 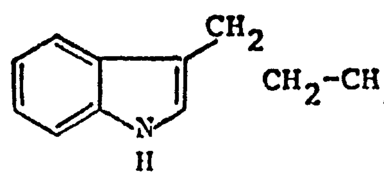 | $CH_2-CH_2-S-CH_2-CH_2-N(CH_3)_2$ | $C_2H_5$ | A | 2.6-2.3m (3H); 2.1-1.0m (14H); 1.2d (3H); 1.0d (6H) |
| 469 | 1 | $CH_3$ | $CH_2-CH_2-S-CH_2-CH_2-CONH_2$ | $C_2H_5$ | A | 7.8-6.5m (6H); 2.8-2.3m (14H); 2.0-1.0m (14H) |
| 470 | 1 | $CH_3$ | 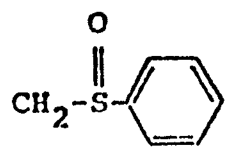 | $C_2H_5$ | D | 6.0bs (2H); 2.5-2.2m (6H); 2.0-1.0m (14H); 1.2d (3H) |

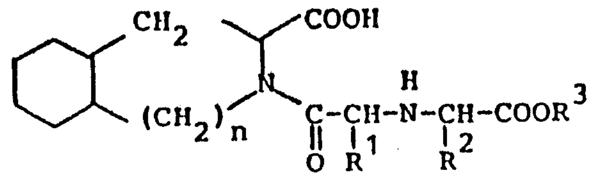

| | n | $R^1$ | $R^2$ | $R^3$ | Methode | NMR |
|---|---|---|---|---|---|---|
| 471 | 1 | $CH_3$ | 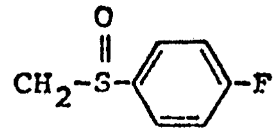 | $C_2H_5$ | D | 7.5-7.0m (5H); 2.6m (2H); 1.2d (3H) |
| 472 | 1 | $CH_3$ | 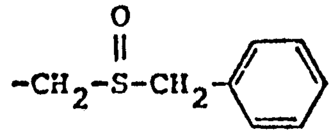 | $C_2H_5$ | D | 7.4-6.9m (4H); 2.6m (2H); 1.2d (3H) |
| 473 | 1 | $CH_3$ | 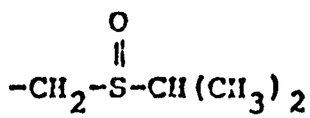 | $C_2H_5$ | D | 7.1s (5H); 4.1s (2H); 2.6m (2H); 1.2d (3H) |
| 474 | 1 | $CH_3$ | 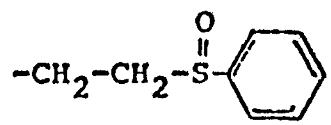 | $C_2H_5$ | D | 2.8-2.5m (3H); 1.2d (3H); 1.0d (6H) |
| 475 | 1 | $CH_3$ | 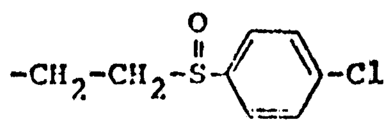 | $C_2H_5$ | D | 7.4-7.0m (5H); 2.6m (2H); 2.0-1.0m(14H); 1.2d (3H) |

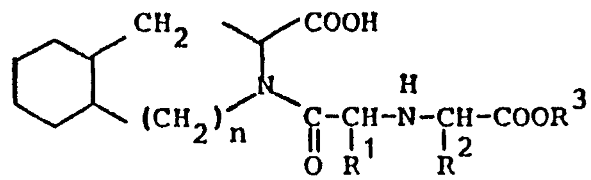

| | n | $R^1$ | $R^2$ | $R^3$ | Methode | NMR |
|---|---|---|---|---|---|---|
| 476 | 1 | $CH_3$ | 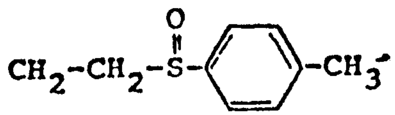 | $C_2H_5$ | D | 7.5-6.9m (4H); 2.6m (2H); 2.0-1.0m (14H); 1.2d (3H) |
| 477 | 1 | $CH_3$ | 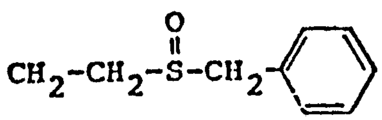 | $C_2H_5$ | D | 7.6-6.9m (4H); 2.6m (2H); 2.3s (3H); 2.0-1.0m (14H); 1.2d (3H) |
| 478 | 1 | $CH_3$ | $CH_2-CH_2-\overset{O}{\overset{\|\|}{S}}-CH(CH_3)_2$ | $C_2H_5$ | D | 7.1s (5H); 4.1s (2H); 2.7m (2H); 2.0-1.0m (14H) 1.2d (3H) |
| 479 | 1 | $CH_3$ | $CH_2-CH_2-\overset{O}{\overset{\|\|}{S}}-CH_2-CH_2-N(CH_3)_2$ | $C_2H_5$ | D | 2.8-2.5m (3H); 2.0-1.0m (14H); 1.2d (3H); 0.9d (6H) |
| 480 | 1 | $CH_2$ | 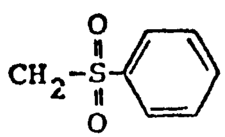 | $C_2H_5$ | E | 2.8-2.2m+s (12H); 2.0 1.0m (14H); 1.2d (3H) |

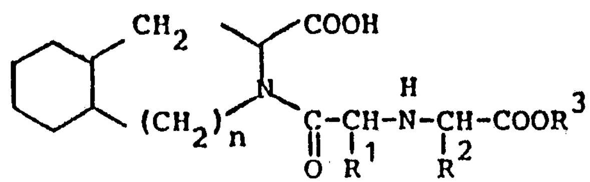

| | n | $R^1$ | $R^2$ | $R^3$ | Methode | NMR |
|---|---|---|---|---|---|---|
| 481 | 1 | $CH_3$ | 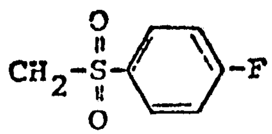 | $C_2H_5$ | E | 7.8-7.1m (5H); 2.8m (2H); 1.2d (3H) |
| 482 | 1 | $CH_3$ | 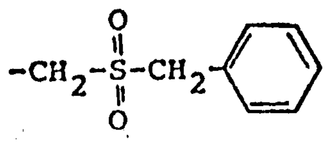 | $C_2H_5$ | E | 7.7-6.9m (4H); 2.8m (2H); 1.2d (3H) |
| 483 | 1 | $CH_3$ | 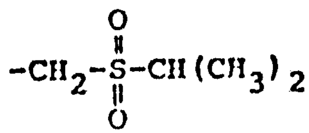 | $C_2H_5$ | E | 7.2s (5H); 5.1-4.3m (4H); 2.8m (2H); 1.2d (3H) |
| 484 | 1 | $CH_3$ | 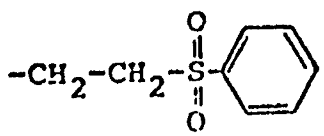 | $C_2H_5$ | E | 3.0-2.7m (3H); 1.2d+t (9H) |
| 485 | 1 | $CH_3$ | 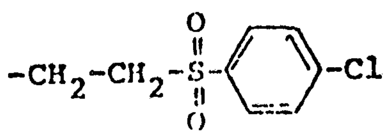 | $C_2H_5$ | E | 7.7-7.2m (5H); 2.8m (2H); 2.0-1.0m(14H); 1.2d (3H) |

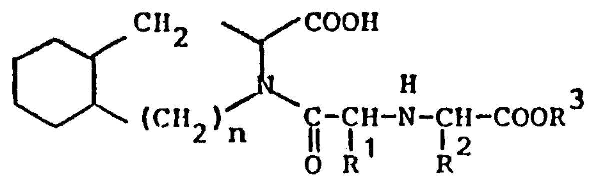

| | n | $R^1$ | $R^2$ | $R^3$ | Methode | NMR |
|---|---|---|---|---|---|---|
| 486 | 1 | $CH_3$ | 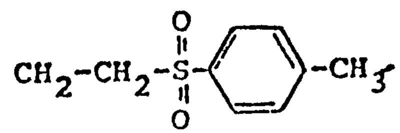 | $C_2H_5$ | E | 8.0-7.1m (4H); 2.8m (2H); 2.0-1.0m (14H); 1.2d (3H) |
| 487 | 1 | $CH_3$ | 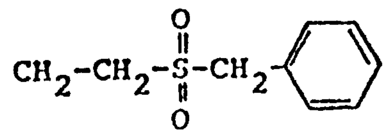 | $C_2H_5$ | E | 7.6-6.9m (4H); 2.8m (2H); 2.3s (3H); 2.0-1.0m(14H); 1.2d (3H) |
| 488 | 1 | $CH_3$ | 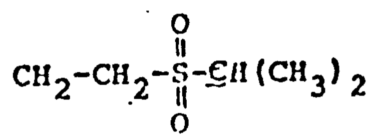 | $C_2H_5$ | E | 7.1s (5H); 4.2s (2H); 2.8m (2H); 2.0-1.0m (14H); 1.2d (3H) |
| 489 | 1 | $CH_3$ | 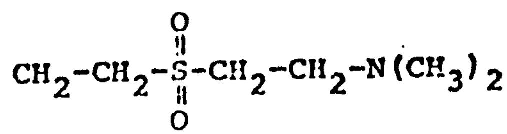 | $C_2H_5$ | E | 3.0-2.7m (3H); 2.0-1.0m (14H); 1.1d+t (9H) |
| 490 | 0 | $CH_3$ | 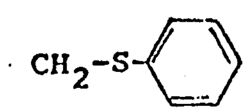 | $C_2H_5$ | A | 3.0-2.2m+s (12H); 2.0-1.0m (14H); 1.2d (3H) |

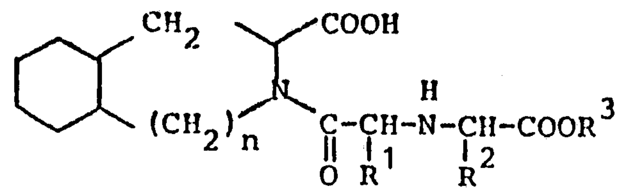

| | n | $R^1$ | $R^2$ | $R^3$ | Methode | NMR |
|---|---|---|---|---|---|---|
| 491 | O | $CH_3$ | 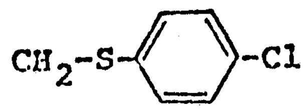 | $C_2H_5$ | A | 6.9s (5H); 2.5m (2H); 1.2d (3H) |
| 492 | O | $CH_3$ | 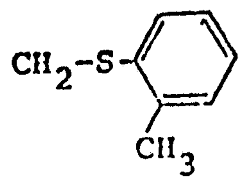 | $C_2H_5$ | A | 7.4-6.8m (4H); 2.5m (2H); 1.2d (3H) |
| 493 | O | $CH_3$ | 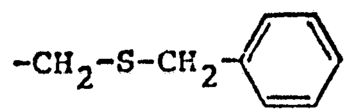 | $C_2H_5$ | A | 7.5-6.8m (4H); 2.5m (2H); 2.3s (3H); 1.2d (3H) |
| 494 | O | $CH_3$ | $-CH_2-S-CH-(CH_3)_2$ | $C_2H_5$ | A | 7.2s (5H); 3.7-3.0m+s (5H); 2.4m (2H); 1.2d(3H) |
| 495 | O | $CH_3$ | 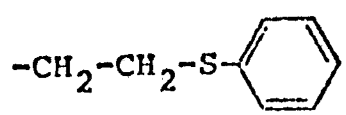 | $C_2H_5$ | A | 2.7-2.3m (3H); 1.2d (3H); 0.9d (6H) |

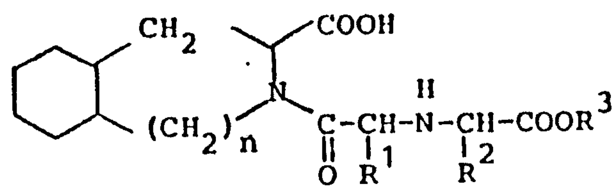

| | n | $R^1$ | $R^2$ | $R^3$ | Methode | NMR |
|---|---|---|---|---|---|---|
| 496 | 1 | $CH_3$ | 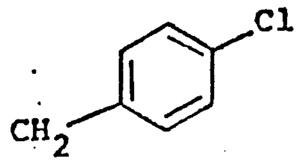 | $C_2H_5$ | B | 7.6-7.0m (4H); 3.9-2.9m (5H); 1.2d (3H) |
| 497 | 1 | $CH_3$ | 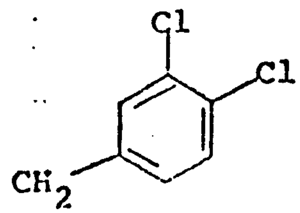 | H | B | 7.8-7.0m (3H); 3.9-2.9m (5H); 1.2d (3H) |
| 498 | 1 | $CH_3$ | 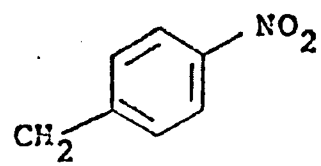 | H | B | 8.3-7.0m (4H); 3.9-2.9m (5H); 1.2d (3H) |
| 499 | 1 | $CH_3$ | 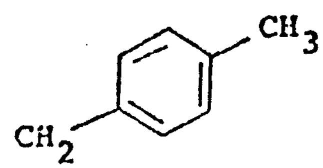 | $C_2H_5$ | B | 7.4-7.0m (4H); 3.9-2.9m (5H); 2.3s (3H); 1.2d(3H) |

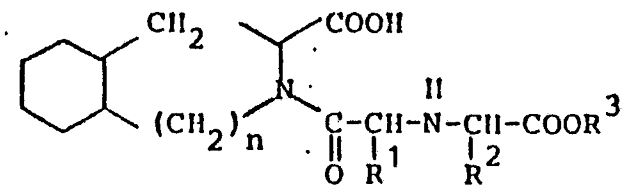

| | n | $R^1$ | $R^2$ | $R^3$ | Methode | NMR |
|---|---|---|---|---|---|---|
| 500 | 1 | $CH_3$ | 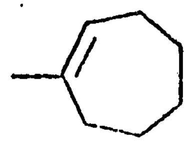 | H | A | 5.3m (1H); 2.3m (4H); 2.0-1.0m (18H); 1.2d (3H) |
| 501 | 1 | $CH_3$ | 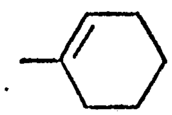 | H | A | 5.4m (1H); 2.3m (4H); 2.0-1.0m (16H); 1.2d (3H) |
| 502 | 1 | $CH_3$ | 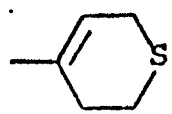 | H | A | 5.5m (1H); 3.9-3.0m (5H); 2.5-1.0m (16H); 1.2d (3H) |
| 503 | 1 | $CH_3$ | 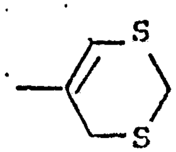 | H | A | 5.9s (1H); 3.9-3.0m (7H); 1.2d (3H) |

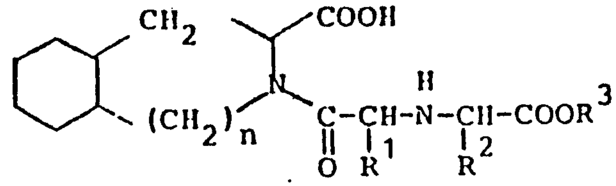

| | n | $R^1$ | $R^2$ | $R^3$ | Methode | NMR |
|---|---|---|---|---|---|---|
| 504 | 1 | $CH_3$ | 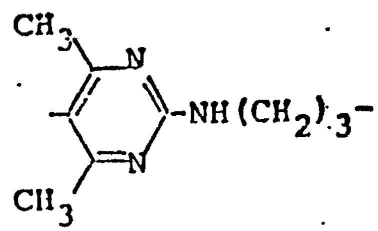 | $C_2H_5$ | A | 7.4s (1H); 2.5m (2H); 2.3s (6H); 2.0-1.0m (16H) |
| 505 | 1 | $CH_3$ | 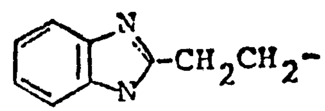 | $C_2H_5$ | A | 7.5-6.8m (4H); 2.8m (2H); 2.0-1.0m (14H); |
| 506 | 1 | $CH_3$ | 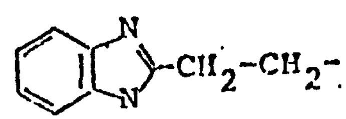 | H | A | 7.5-6.8m (4H); 2.8m (2H); 2.0-1.0m (14H) |

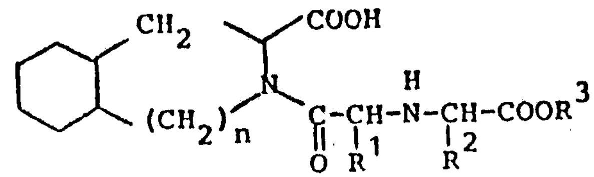

| | n | $R^1$ | $R^2$ | $R^3$ | Methode | NMR |
|---|---|---|---|---|---|---|
| 507 | O | $CH_3$ | 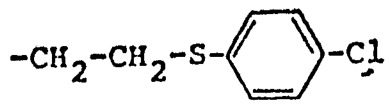 | $C_2H_5$ | A | 7.0s (5H); 2.7m (2H); 2.0-1.0m (13H); 1.2d(3H) |
| 508 | O | $CH_3$ | 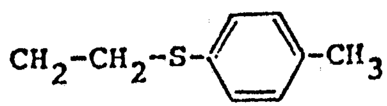 | $C_2H_5$ | A | 7.5-6.5m (4H); 2.7m (2H); 2.0-1.0m (13H); 1.2d (3H) |
| 509 | O | $CH_3$ | 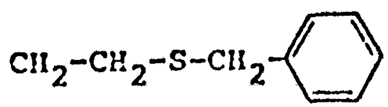 | $C_2H_5$ | A | 7.3-6.5m (4H); 2.7-2.3m+s (5H); 2.0-1.0m (13H); 1.2d (3H) |
| 510 | O | $CH_3$ | $CH_2-CH_2-S-CH(CH_3)_2$ | $C_2H_5$ | A | 4,5-3.0 m+s (5H); 2.4m (2H); 2.0-1.0m (13H); 1.2d (3H) |
| 511 | O | $CH_3$ | 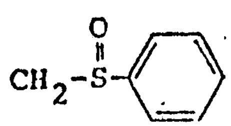 | $C_2H_5$ | D | 2.6-2.3m (3H); 2.0-1.0m (13H); 1.2d (3H); 1.0d (6H) |

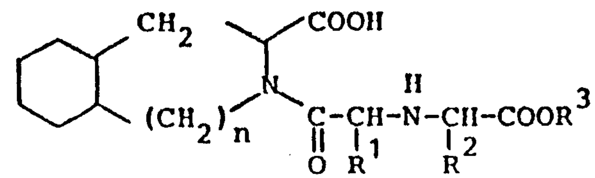

| | n | $R^1$ | $R^2$ | $R^3$ | Methode | NMR |
|---|---|---|---|---|---|---|
| 512 | O | $CH_3$ | 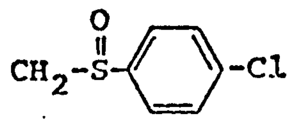 | $C_2H_5$ | D | 7.5-7.0m (5H); 2.6m (2H); 1.2d (3H) |
| 513 | O | $CH_3$ | 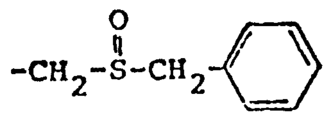 | $C_2H_5$ | D | 7.4-7.0m (4H); 2.6m (2H); 1.2d (3H) |
| 514 | O | $CH_3$ | 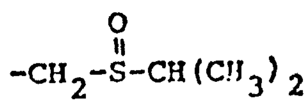 | $C_2H_5$ | D | 7.1s (5H); 4.1s (2H); 2.6m (2H); 1.2d (3H) |
| 515 | O | $CH_3$ | 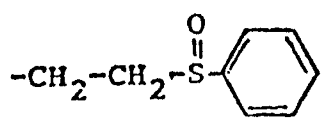 | $C_2H_5$ | D | 2.8-2.5m (3H); 1.2d (3H); 1.0d (6H) |
| 516 | O | $CH_3$ | 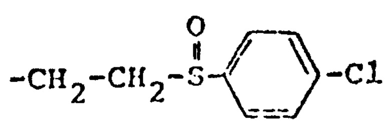 | $C_2H_5$ | D | 7.4-7.0m (5H); 2.6m (2H); 2.0-1.0m(13H); 1.2d (3H) |

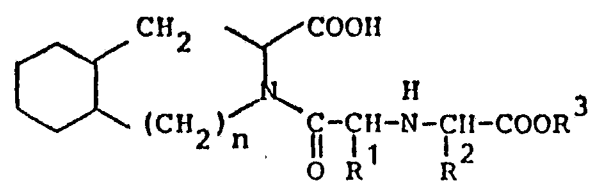

| | n | $R^1$ | $R^2$ | $R^3$ | Methode | NMR |
|---|---|---|---|---|---|---|
| 517 | O | $CH_3$ | 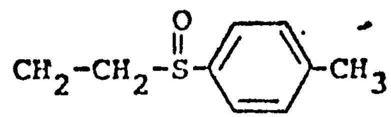 | $C_2H_5$ | D | 7.5-7.0m (4H); 2.6m (2H); 2.0-1.0m (13H); 1.2d (3H) |
| 518 | O | $CH_3$ | 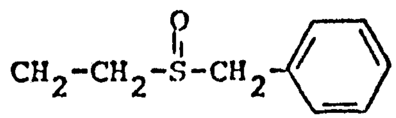 | $C_2H_5$ | D | 7.6-7.0 (4H); 2.6m (2H); 2.3s (3H); 2.1-1.0m (13H); 1.2d (3H) |
| 519 | O | $CH_3$ | 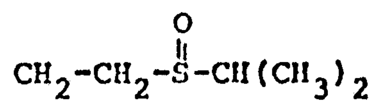 | $C_2H_5$ | D | 7.1s (5H); 4.1s (2H); 2.7m (2H); 2.1-1.0m (13H); 1.2d (3H) |
| 520 | O | $CH_3$ | 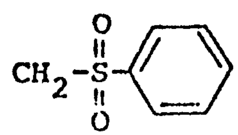 | $C_2H_5$ | E | 2.8-2.5m (3H); 2.0-1.0m (13H); 1.2d (3H); 1.1d(6H) |
| 521 | O | $CH_3$ | 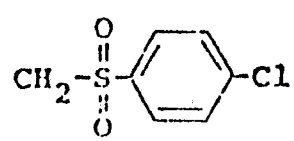 | $C_2H_5$ | E | 7.8-7.3m (5H); 2.8m (2H); 1.2d (3H) |

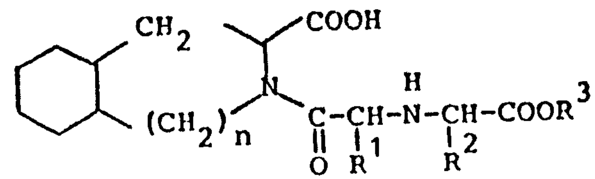

| | n | $R^1$ | $R^2$ | $R^3$ | Methode | NMR |
|---|---|---|---|---|---|---|
| 522 | O | $CH_3$ | 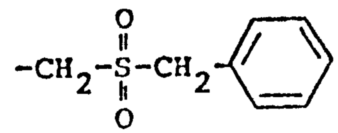 | $C_2H_5$ | E | 7.7-7.2m (4H); 2.8m (2H); 1.2d (3H) |
| 523 | O | $CH_3$ | 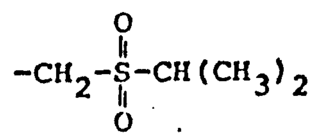 | $C_2H_5$ | E | 7.1s (5H); 4.3s (2H); 2.8m (2H); 1.2d (3H) |
| 524 | O | $CH_3$ | 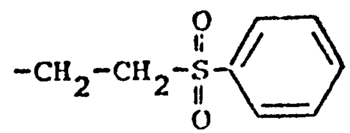 | $C_2H_5$ | E | 3.0-2.7m (3H); 1.2d+t (9H) |
| 525 | O | $CH_3$ | 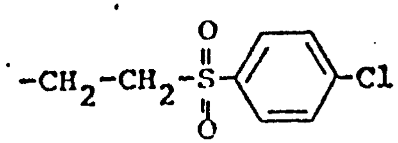 | $C_2H_5$ | E | 7.7-7.2m (5H); 2.8m (2H); 2.0-1.0m (13H); 1.2d (3H) |
| 526 | O | $CH_3$ | 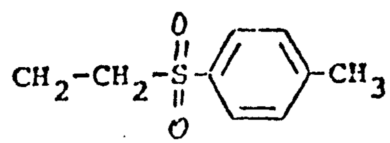 | $C_2H_5$ | E | 8.0-7.3m (4H); 2.8m (2H); 2.0-1.0m (13H);1.2d (3H) |

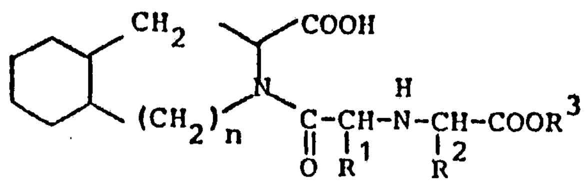

| | n | $R^1$ | $R^2$ | $R^3$ | Methode | NMR |
|---|---|---|---|---|---|---|
| 527 | 0 | $CH_3$ | 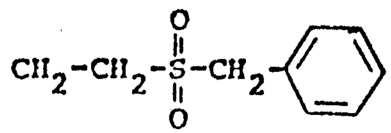 | $C_2H_5$ | E | 7.1s (5H); 4.2s (2H); 2.8m (2H); 2.1-1.0m (13H) |
| 528 | 0 | $CH_3$ | 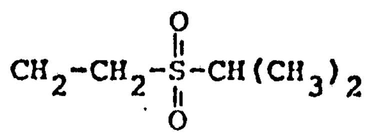 | $C_2H_5$ | E | 3.0-2.7m (3H); 2.0 - 1.0m (13H); 1.1d+t (9H) |
| 529 | 1 | $CH_3$ | $-CH_2-OH$ | $C_2H_5$ | A | 3.9-3.0m (5H); 1.2d (3H) |
| 530 | 1 | $CH_3$ | 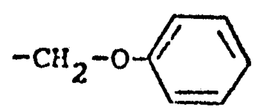 | $C_2H_5$ | H | 7.0s (5H); 3.9-3.0m (5H); 1.2d (3H) |
| 531 | 1 | $CH_3$ | 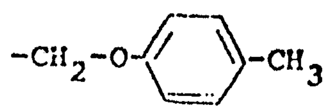 | $C_2H_5$ | H | 7.3-6.6m (4H); 3.9-3.0m (5H); 2.3s (3H); 1.2d(3H) |

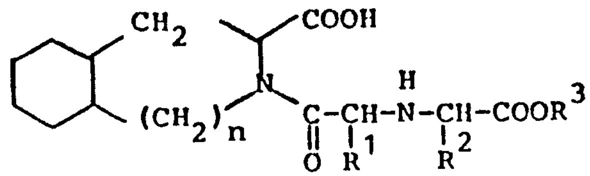

| | n | $R^1$ | $R^2$ | $R^3$ | Methode | NMR |
|---|---|---|---|---|---|---|
| 532 | 1 | $CH_3$ | 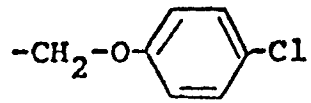 | $C_2H_5$ | H | 7.3-6.7m (4H); 3.9-3.0m (5H); 1.2d (3H) |
| 533 | 1 | $H_2N-(CH_2)_4$ | 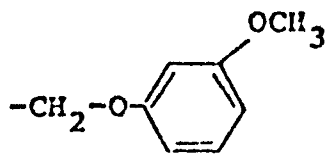 | $C_2H_5$ | B | 6.8-6.3m (4H); 3.9-3.0 m+s (8H); 2.4m (2H); 2.0-1.0m (18H) |
| 534 | 1 | $H_2N-(CH_2)_3$ | 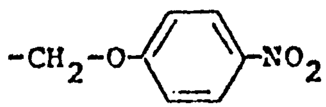 | $C_2H_5$ | B | 8.2-6.8m (4H); 3.9-3.0m (5H); 2.4m (2H); 2.0-1.0m (16H) |
| 535 | 1 | $(CH_3)_2-CH-CH_2-$ | 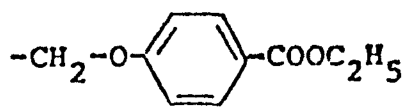 | $C_2H_5$ | A | 7.6-6.8m (4H); 4.2q(4H); 3.9-3.0m (5H); 2.0 - 1.0m (15H); 1.2t (6H); 1.0d (6H) |
| 536 | 1 | $CH_3$ | 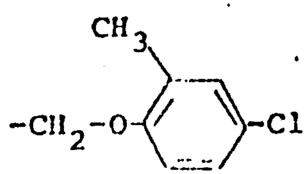 | $C_2H_5$ | A | 7.2-6.8m (3H); 3.9-3.0m (5H); 2.3s (3H); 1.2d (3H) |

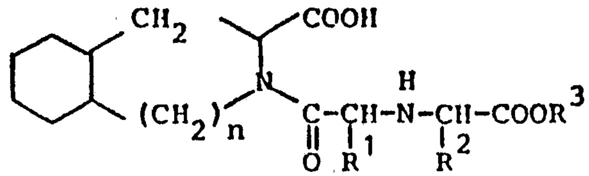

| | n | $R^1$ | $R^2$ | $R^3$ | Methode | NMR |
|---|---|---|---|---|---|---|
| 537 | 1 | $CH_3$ | 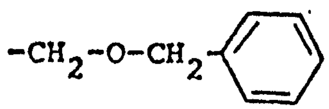 | $C_2H_5$ | A | 7.0s (5H); 4.0s (2H); 3.9-3.0m (5H); 1.3d (3H) |
| 538 | 1 | $CH_3$ | 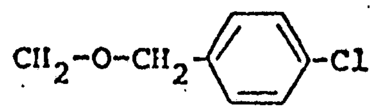 | $C_2H_5$ | A | 7.4-6.9m (4H); 4.0s (2H) 3.9-3.0m (5H); 1.2d (3H) |
| 539 | 1 | $H_2N-(CH_2)_4$ | 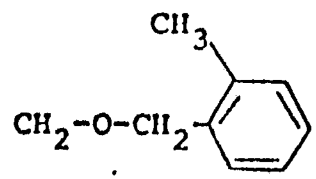 | $C_2H_5$ | B | 7.4-7.0m (4H); 4.0s (2H); 3.9-3.0m (5H); 2.5-2.2d+t (5H); 2.0-1.0m (18H) |
| 540 | 1 | 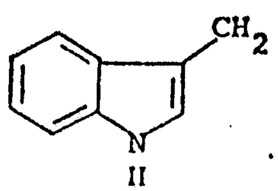 | 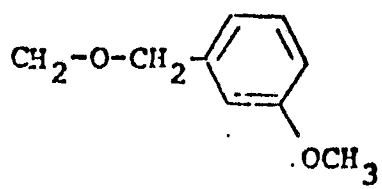 | $C_2H_5$ | A | 7.8-6.4m (10H); 4.0s (2H); 3.8s (3H): 3.9-3.0m (5H); 2.7m (2H) |
| 541 | 1 | H | 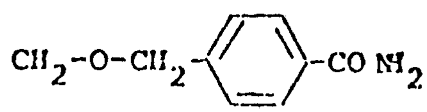 | $C_2H_5$ | A | 7.8-7.0m (4H); 6.0bs(2H); 4.0s (2H); 3.9-3.0m (6H) |

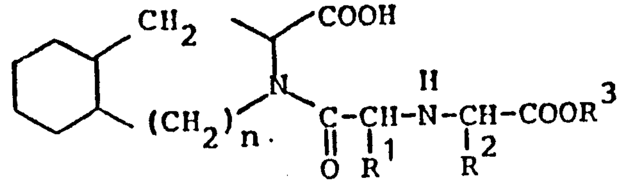

| | n | $R^1$ | $R^2$ | $R^3$ | Methode | NMR |
|---|---|---|---|---|---|---|
| 542 | 1 | $HS-CH_2$ | 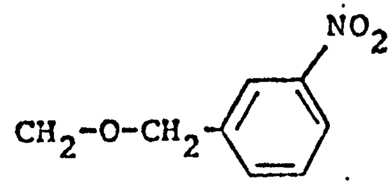 | $C_2H_5$ | B | 8.2-7.0m (4H); 4.0s(2H); 3.9-3.0m (5H); 2.3m(2H) |
| 543 | 1 | $HS-CH_2$ | 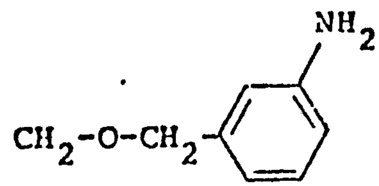 | $C_2H_5$ | B | 7.2-6.4m (4H); 4.0s (2H); 3.9-3.0m (5H); 2.3m (2H) |
| 544 | 1 | $CH_3$ | $CH_2-O-CH(CH_3)_2$ | $C_2H_5$ | H | 3.9-3.0m (6H); 1.2d (3H); 0.9d (6H); |
| 545 | 1 | $CH_3$ | $CH_2-O-CH_2-CH_2-N(CH_3)_2$ | $C_2H_5$ | A | 3.9-3.0m (7H); 2.4-2.1m+s (8H); 1.2d (3H) |
| 546 | 1 | $CH_3$ | 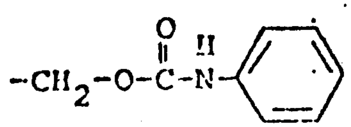 | $C_2H_5$ | A | 7.6-6.9m (5H); 4.0m (2H); 1.2d (3H) |

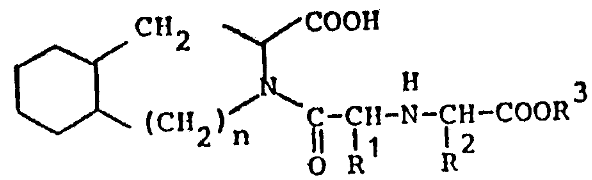

| | n | $R^1$ | $R^2$ | $R^3$ | Methode | NMR |
|---|---|---|---|---|---|---|
| 547 | 1 | $CH_3$ | $CH_2-COOH$ | H | A | 2.1-1.0m (14H); 1.2d (3H) |
| 548 | 1 | $CH_3$ | $CH_2-CONH_2$ | H | A | 6.5bs (2H); 2.1-1.0m (14H); 1.2d (3H) |
| 549 | 1 | $CH_3$ | $CH_2-CON(CH_3)_2$ | H | A | 3.9-3.0m+2s (9H); 2.1-1.0m (14H); 1.2d (3H) |
| 550 | 1 | 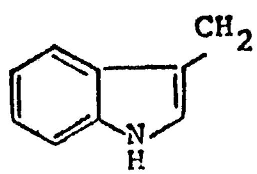 | 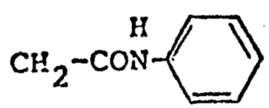 | H | A | 7.8-6.4m (11H); 2.8 - 1.0m (16H) |
| 551 | 1 | $CH_3$ | 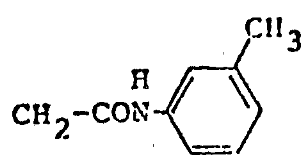 | H | A | 7.3-6.9m (4H); 2.3s(3H); 2.2-1.0m (14H);1.2d (3H) |

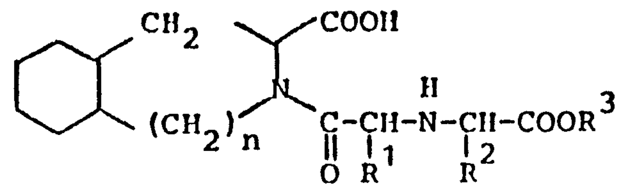

| | n | $R^1$ | $R^2$ | $R^3$ | Methode | NMR. |
|---|---|---|---|---|---|---|
| 552 | 1 | $CH_3$ | 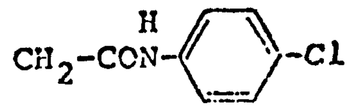 | H | F | 7.7-6.9m (4H); 2.3m (2H); 1.2d (3H) |
| 553 | 1 | 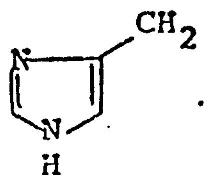 | 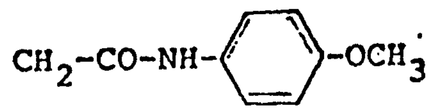 | H | F | 13.0s (1H); 7.5-6.3m (6H); 3.9s (3H); 2.7m (2H); 2.1-1.0m (14H) |
| 554 | 1 | $CH_3$ | 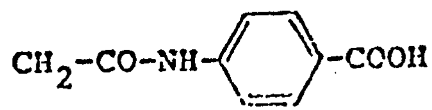 | H | F | 7.7-6.6m (4H); 2.1-1.0m (14H); 1.2d (3H) |
| 555 | 1 | $CH_3$ | 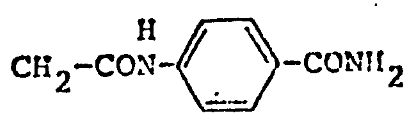 | H | F | 7.7-6.6m (4H);2.1-1.0m (14H); 1.2d (3H) |
| 556 | 1 | $CH_3$ | 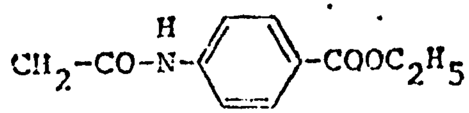 | H | F | 7.7-6.6m (4H); 4.2q (2H); 2.1-1.0m(14H); 1.2d+t (6H) |

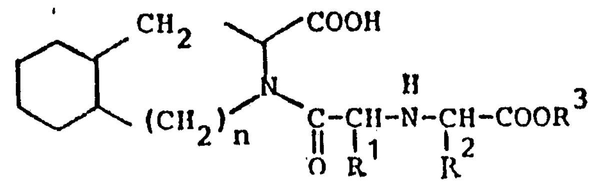

| | n | $R^1$ | $R^2$ | $R^3$ | Methode | NMR |
|---|---|---|---|---|---|---|
| 557 | 1 | $CH_3$ | 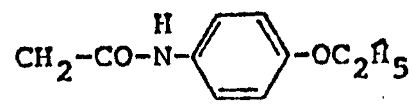 | H | F | 7.3-6.4m(4H); 3.5q (2H); 2.1-1.0m (14H); 1.2d+t (6H) |
| 558 | 1 | $CH_3$ | 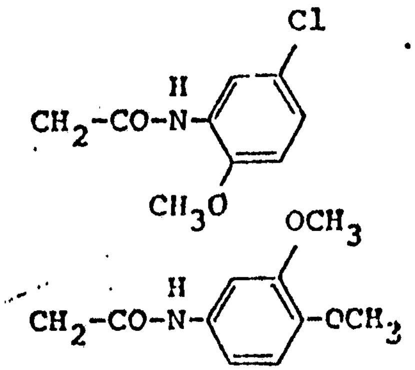 | H | F | 7.2-6.4m (3H); 3.9s (3H); 2.1-1.0m (14H); 1.2d(3H) |
| 559 | 1 | $CH_3$ | | H | F | 7.1-6.3m (3H); 3.8s (6H); 2.1-1.0m (14H); 1.2d(3H) |
| 560 | 1 | $CH_3$ | 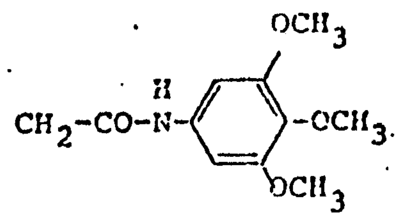 | H | F | 7.0-6.2m (2H); 3.9s (9H); 2.1-1.0m (14H); 1.2d (3H) |
| 561 | 1 | $CH_3$ | $CH_2-COOC_2H_5$ | H | F | 4.2q (2H); 2.0-1.0m (14H); 1.2d+t (6H) |

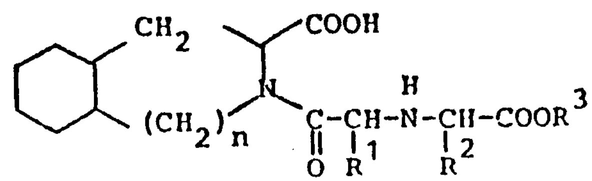

| | n | $R^1$ | $R^2$ | $R^3$ | Methode | NMR |
|---|---|---|---|---|---|---|
| 562 | 1 | $CH_3$ | $CH_2-CO-O-CH(CH_3)_2$ | H | F | 4.8septett (1H); 2.1 - 1.0m (14H); 1.2d (3H); 0.9d (6H) |
| 563 | 1 | $CH_3$ | $CH_2-CO-O-CH_2-$ 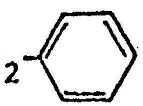 | H | F | 7.2s (5H); 5.3s (2H); 2.1-1.0m (14H); 1.2d (3H) |
| 564 | 1 | $CH_3$ | $CH_2-CH_2-COOH$ | $C_2H_5$ | A | 2.1-1.0m (16H); 1.2d (3H) |
| 565 | 1 | $CH_3$ | $CH_2-CH_2-CONH_2$ | $C_2H_5$ | A | 2.1-1.0m (16H); 1.2d (3H) |
| 566 | 1 | $CH_3$ | $CH_2-CH_2-CON(CH_3)_2$ | $C_2H_5$ | A | 3.02s (6H); 2.0-1.0m(14H) 1.2d (3H) |

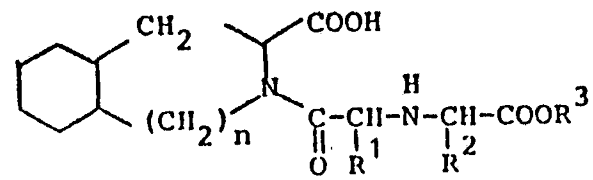

| | n | $R^1$ | $R^2$ | $R^3$ | Methode | NMR |
|---|---|---|---|---|---|---|
| 567 | 1 | $CH_3$ | 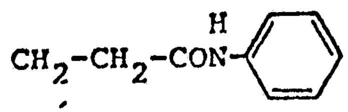 | $C_2H_5$ | A | 7.1s (5H); 2.1-1.0m (16H) 1.2d (3H) |
| 568 | 1 | $CH_3$ | 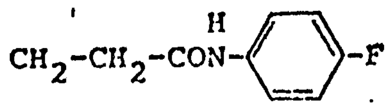 | $C_2H_5$ | A | 7.3-6.9m (4H); 2.1-1.0m (16H); 1.2d (3H) |
| 569 | 1 | $H_2N-(CH_2)_4$ | 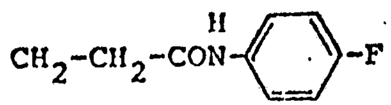 | $C_2H_5$ | B | 7.3-6.8m (4H); 2.4-2.1m (4H); 2.0-1.0m (20H) |
| 570 | 1 | $CH_3$ | 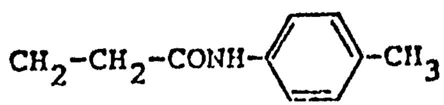 | $C_2H_5$ | A | 7.2-6.4m (4H); 3.9s (3H); 2.1-1.0m (16H); 1.2d(3H) |
| 571 | 1 | $CH_3$ | 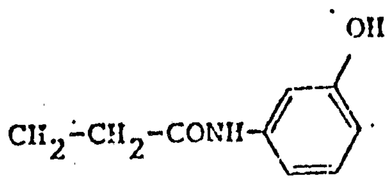 | H | A | 7.3-6.3m (4H); 2.1-1.0m (16H); 1.2d (3H) |

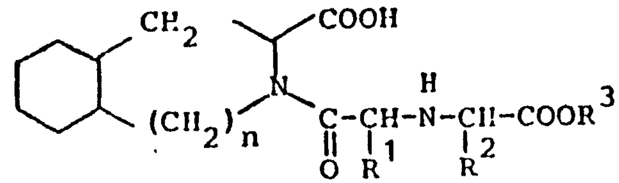

| | n | $R^1$ | $R^2$ | $R^3$ | Methode | NMR. |
|---|---|---|---|---|---|---|
| 572 | 1 | $CH_3$ | 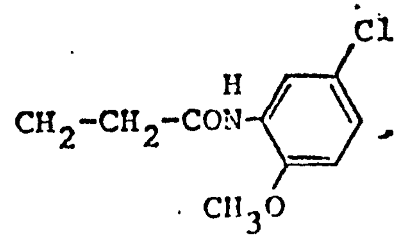 | H | A | 7.2-6.4m (3H); 3.9s(3H); 2.1-1.0m (16H); 1.2d (3H) |
| 573 | 1 | $CH_3$ | 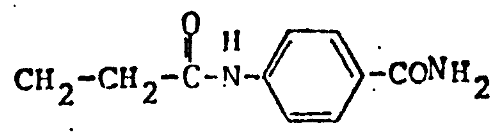 | H | A | 7.7-7.0m (4H); 2.1-1.0m (16H); 1.2d (3H) |
| 574 | 1 | $CH_3$ | 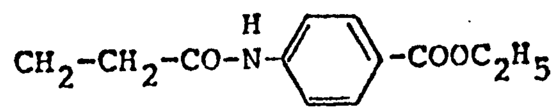 | H | A | 7.6-7.0m (4H); 4.2q (2H); 2.1-1.0m (16H); 1.2d+t (6H) |
| 575 | 1 | $CH_3$ | 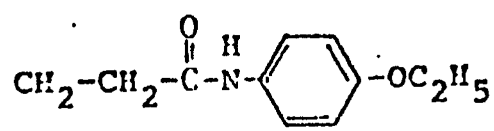 | H | A | 7.3-6.4m (4H); 3.9-3.0m m+q (5H); 2.1-1.0m (16H); 1.2d+t (6H) |
| 576 | 1 | $CH_3$ | 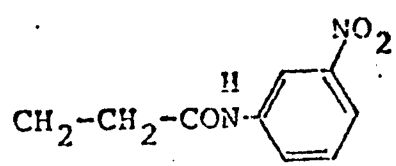 | H | A | 8.2-6.9m (4H); 2.1-1.0m (16H); 1.2d (3H) |

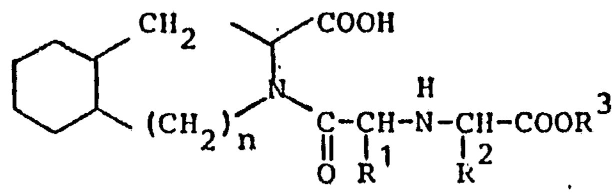

| | n | $R^1$ | $R^2$ | $R^3$ | Methode | NMR |
|---|---|---|---|---|---|---|
| 577 | 1 | $CH_3$ | 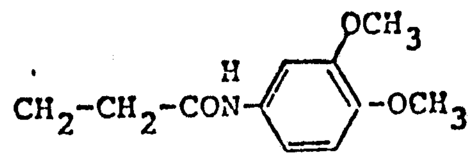 | $C_2H_5$ | A | 7.1-6.3m (3H); 3.8s (6H); 2.1-1.0m (16H); 1.2d(3H) |
| 578 | 1 | $CH_3$ | 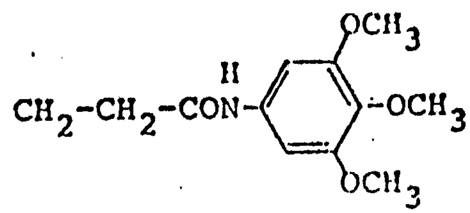 | $C_2H_5$ | A | 7.0-6.2m (2H); 3.9s (9H); 2.1-1.0m (16H); 1.2d (3H) |
| 579 | 1 | $CH_3$ | $CH_2-CH_2-CON(H)-CH(CH_3)_2$ | H | A | 2.8-2.2m (3H); 2.0-1.0m (14H); 1.2d (3H); 1.0d (6H) |
| 580 | 1 | $CH_3$ | 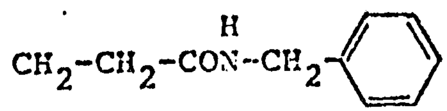 | H | A | 7.2s (5H); 5.1-4.3m (4H); 2.1-1.0m (16H); 1.2d (3H) |
| 581 | 1 | $CH_3$ | 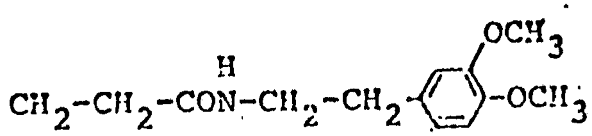 | $C_2H_5$ | A | 7.1-6.3m (3H); 3.9s (6H); 2.9-1.0m (20H); 1.2d(3H) |

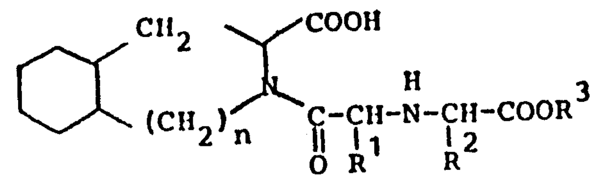

| | n | $R^1$ | $R^2$ | $R^3$ | Methode | NMR |
|---|---|---|---|---|---|---|
| 582 | O | $CH_3$ | $CH_2-OH$ | $C_2H_5$ | A | 4.5 –3.0m (5H); 1.2d(3H) |
| 583 | O | $CH_3$ | $-CH_2-O-$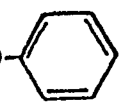 | $C_2H_5$ | A | 6.9s (5H); 4.5-3.0m (5H); 1.2d (3H) |
| 584 | O | $CH_3$ | $-CH_2-O-CH_2-$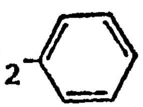 | $C_2H_5$ | A | 7.2s (5H); 4.0s (2H); 4.5-3.0m (5H); 1.2d (3H) |
| 585 | O | $CH_3$ | $CH_2-O-CH_2-$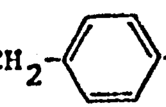$-Cl$ | $C_2H_5$ | A | 7.4-6.5m (4H); 4.0s (2H); 4.5-3.0m (5H); 1.2d (3H) |
| 586 | O | $CH_3$ | $CH_2-O-CH(CH_3)_2$ | $C_2H_5$ | A | 4.5-3.0m (6H); 1.2d (3H); 0.9d (6H) |

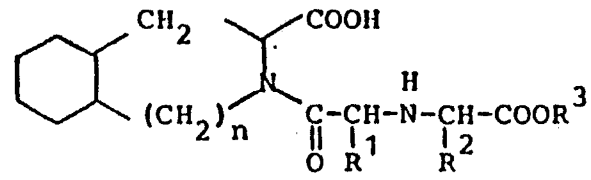

| | n | $R^1$ | $R^2$ | $R^3$ | Methode | NMR |
|---|---|---|---|---|---|---|
| 587 | O | $CH_3$ | $CH_2-O-CH_2-CH_2-N(CH_3)_2$ | $C_2H_5$ | A | 4.5 -3.0m (7H); 2.4-2.1 m+s (8H); 1.2d (3H) |
| 588 | O | $CH_3$ | 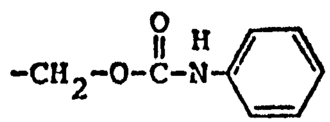 | $C_2H_5$ | A | 7.3s (5H); 4.5-3.0m (5H) 1.2d (3H) |
| 589 | O | $CH_3$ | $CH_2-COOH$ | H | A | 2.1-1.0m (13H); 1.2d (3H) |
| 590 | O | $CH_3$ | $CH_2-CONH_2$ | H | F | 6.5s (2H); 2.1-1.0m (13H) 1.2d (3H) |
| 591 | O | $CH_3$ | $CH_2-CON(CH_3)_2$ | H | F | 4.5-3.0m+2s (9H); 2.1 - 1.0m (13H); 1.2d (3H) |

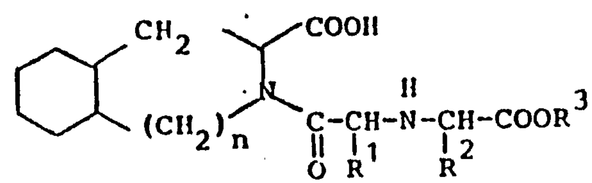

| | n | $R^1$ | $R^2$ | $R^3$ | Methode | NMR |
|---|---|---|---|---|---|---|
| 592 | 0 | $CH_3$ | 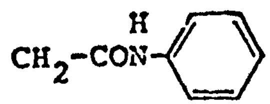 | H | F | 7.0s (5H); 2.1-1.0m(13H) 1.2d (3H) |
| 593 | 0 | $CH_3$ | 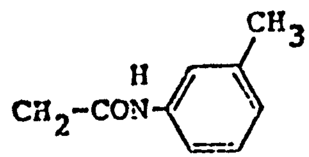 | H | F | 7.3-6.5m (4H); 2.3s (3H); 2.2-1.0m (13H); 1.2d (3H) |
| 594 | 0 | $CH_3$ | 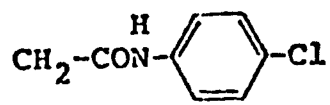 | H | F | 7.7-6.5m (4H); 2.3m (2H); 1.2d (3H) |
| 595 | 0 | $CH_3$ | $CH_2-COOC_2H_5$ | H | F | 4.2q (2H); 2.0-1.0m (13H) 1.2d+t (6H) |
| 596 | 0 | $CH_3$ | $CH_2-CH_2-COOH$ | $C_2H_5$ | A | 2.1-1.0m (15H); 1.2d (3H) |

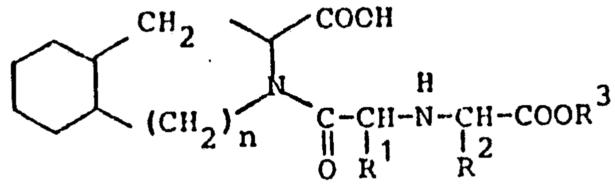

| | n | $R^1$ | $R^2$ | $R^3$ | Methode | NMR |
|---|---|---|---|---|---|---|
| 597 | O | $CH_3$ | $CH_2-CH_2-CONH_2$ | $C_2H_5$ | A | 2.1-1.0m (15H); 1.2d (3H) |
| 598 | O | $CH_3$ | $CH_2-CH_2-CON(CH_3)_2$ | $C_2H_5$ | A | 3.02s (6H); 2.0-1.0m (13H); 1.2d (3H) |
| 599 | O | $CH_3$ | $CH_2-CH_2-CON(H)-$ 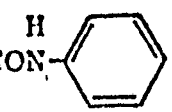 | $C_2H_5$ | A | 7.0s (5H); 2.1-1.0m (15H); 1.2d (3H) |
| 600 | O | $CH_3$ | $CH_2-CH_2-CON(H)-$ 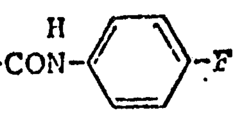 | H | A | 7.3-6.8m (4H); 2.1-1.0m (15H); 1.2d (3H) |
| 601 | O | $CH_3$ | $CH_2-CH_2-C(=O)-N(H)-$ 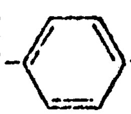 $-OC_2H_5$ | $C_2H_5$ | A | 7.3-6.4m (4H); 3.6q (2H); 2.1-1.0m (15H); 1.2 d+t (6H) |

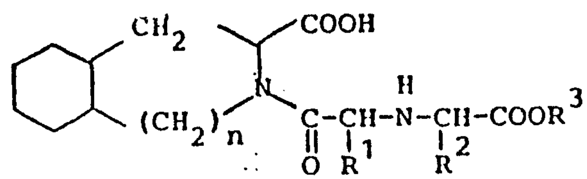

| | n | $R^1$ | $R^2$ | $R^3$ | Methode | NMR |
|---|---|---|---|---|---|---|
| 602 | 0 | $CH_3$ | $CH_2-CH_2-CONH-CH(CH_3)_2$ | $C_2H_5$ | A | 2.8-2.[illegible] (3H); 2.0-1.0m (13H); 1.2d (3H); 1.0d(6H) |
| 603 | 0 | $CH_3$ | 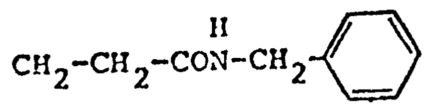 | $C_2H_5$ | A | 7.0s (5H); 5.1-4.3m+s (5H); 2.1-1.0m (15H); 1.2d (3H) |
| 604 | 0 | $CH_3$ | 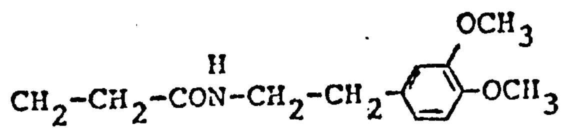 | $C_2H_5$ | A | 7.0-6.2m (3H); 3.9s (6H); 2.9-1.0m (19H); 1.2d (3H) |
| 605 | 1 | $CH_3$ | 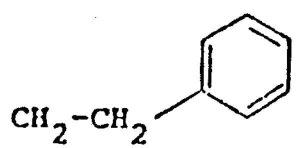 | $C_2H_5$ | B | 7.2s (5H); 2.6m (2H); 2.0-1.0m (14H); 1.2d (3H) |

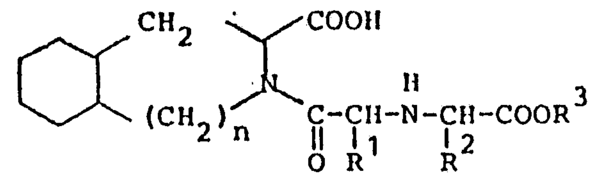

| | n | $R^1$ | $R^2$ | $R^3$ | Methode | NMR |
|---|---|---|---|---|---|---|
| 606 | 1 | $CH_3$ | 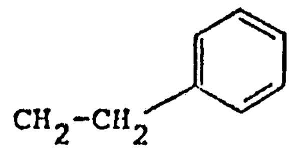 | H | B | 7.2s (5H); 2.6m (2H); 2.0-1.0m (14H); 1.2d (3H) |
| 607 | 1 | $CH_3$ | 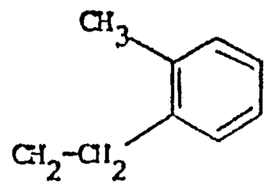 | $C_2H_5$ | B | 7.4-7.0m (4H); 2.6m (2H); 2.3s (3H); 2.0-1.0m (14H); 1.2d (3H) |
| 608 | 1 | $HO-CH_2$ | 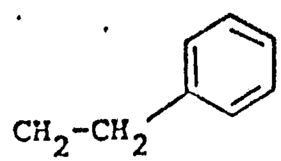 | $C_2H_5$ | B | 7.2s (5H); 2.8-2.5m (4H); 2.0-1.0m (14H) |
| 609 | 1 | $H_2N-(CH_2)_4-$ | 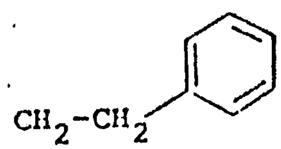 | H | B | 7.2s (5H); 2.8-2.3m (4H); 2.0-1.0m (20H) |

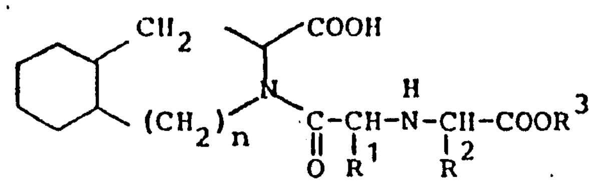

| | n | $R^1$ | $R^2$ | $R^3$ | Methode | NMR |
|---|---|---|---|---|---|---|
| 610 | 1 | $H_2N(CH_2)_3-$ | 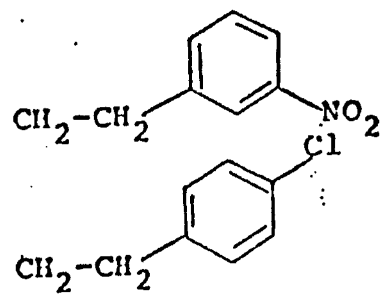 | H | B | 8.2-7.1m (4H); 2.8-2.3m (4H); 2.0-1.0m (18H) |
| 611 | 1 | $CH_3$ | | $C_2H_5$ | B | 7.5-6.9m (4H); 2.7m (2H); 2.0-1.0m (14H); 1.2d(3H) |
| 612 | 1 | $CH_3$ | 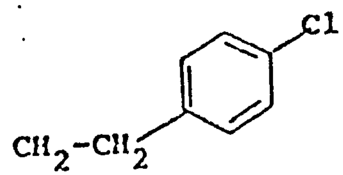 | H | B | 7.5-6.9m (4H); 2.7m (2H); 2.0-1.0m (14H); 1.2d (3H) |
| 613 | 1 | $CH_3$ | 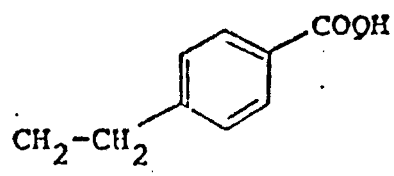 | H | B | 7.8-7.1m (4H); 2.7m (2H); 2.0-1.0m (14H); 1.2d(3H) |
| 614 | 1 | 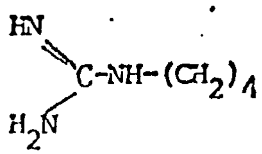 | 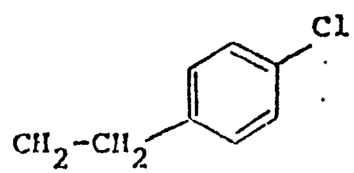 | $C_2H_5$ | B | 7.5-7.0m (4H); 2.8-2.6m (4H); 2.0-1.0m (20H) |

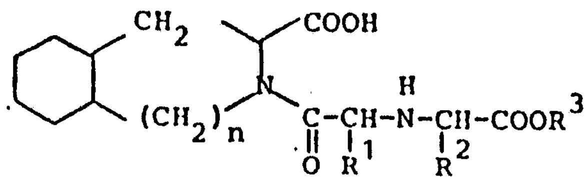

| | n | $R^1$ | $R^2$ | $R^3$ | Methode | NMR |
|---|---|---|---|---|---|---|
| 615 | 1 | $H_2N-(CH_2)_2$ | 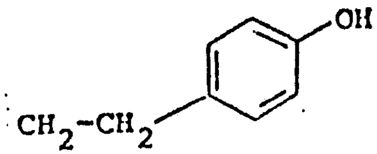 | H | B | 7.2-6.5m (4H); 2.8-2.3m (4H); 2.0-1.0m (16H) |
| 616 | 1 | $H_2N-(CH_2)_5$ | 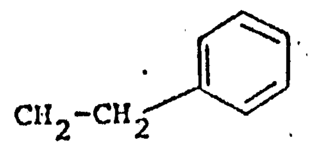 | H | B | 7.2bs (5H); 2.7-2.3m (4H) 2.0-1.0m (22H) |
| 617 | 1 | $H_2N-(CH_2)_4$ | 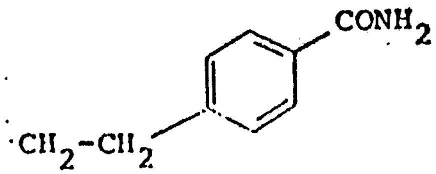 | H | B | 7.8-7.1m (4H); 2.7-2.3m (4H); 2.0-1.0m (20H) |
| 618 | 1 | $H_2N-CH_2$ | 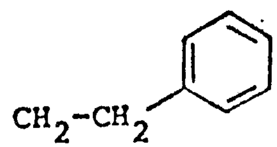 | H | B | 7.2bs (5H); 2.7-2.3m(4H); 2.0-1.0m (14H) |

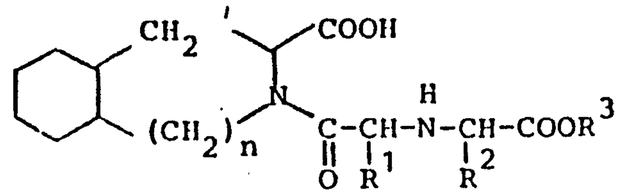

| | n | $R^1$ | $R^2$ | $R^3$ | Methode | NMR |
|---|---|---|---|---|---|---|
| 619 | 1 | $CH_3$ | 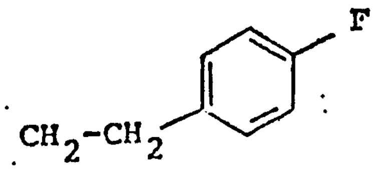 | $C_2H_5$ | B | 7.2-6.8m (4H); 2.7m (2H); 2.0-1.0m (14H); 1.2d(3H) |
| 620 | 1 | $CH_3$ | 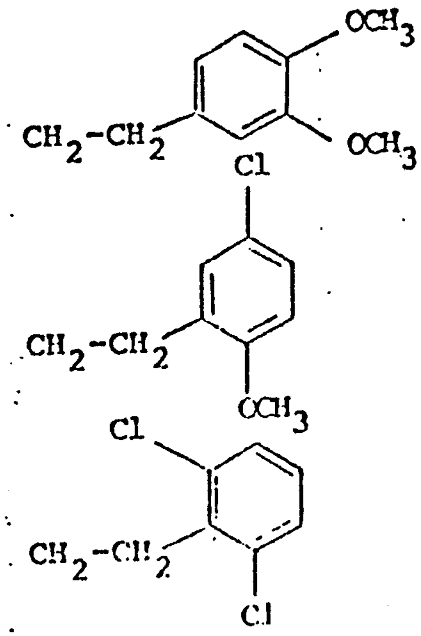 | $C_2H_5$ | B | 7.2-6.3m (3H); 3.9s (6H); 2.7m (2H); 2.0 - 1.0m (14H); 1.2d (3H) |
| 621 | 1 | $CH_3$ | | $C_2H_5$ | B | 7.3-6.4m (3H); 3.8s(3H) 2.6m (2H); 2.0-1.0m(14H) |
| 622 | 1 | $CH_3$ | | $C_2H_5$ | B | 7.4-6.8m (3H); 2.7m (2H); 2.0-1.0m (14H); 1.2d (3H) |

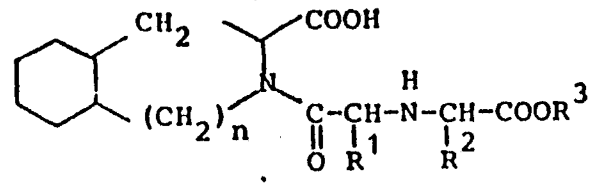

| | n | $R^1$ | $R^2$ | $R^3$ | Methode | NMR |
|---|---|---|---|---|---|---|
| 623 | 1 | 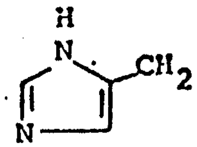 | 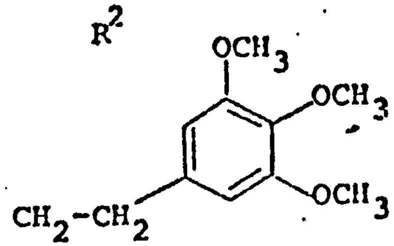 | $C_2H_5$ | B | 13.1s (1H); 7.5-6.2m (4H); 3.9s (9H); 2.8-2.3m (4H); 2.0-1.0m (14H) |
| 624 | 1 | $CH_3$ | 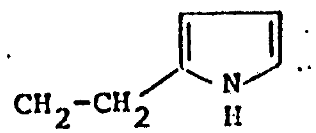 | $C_2H_5$ | B | 7.3-6.0m (3H); 2.8m (2H); 2.0-1.0m (14H); 1.2d (3H) |
| 625 | 1 | $CH_3$ | 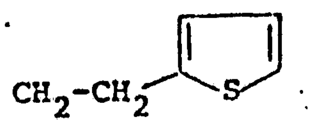 | $C_2H_5$ | B | 7.3-6.9m (3H); 2.9m (2H); 2.0-1.0m (14H); 1.2d (3H) |
| 626 | 1 | $H_2N-(CH_2)_4$ | 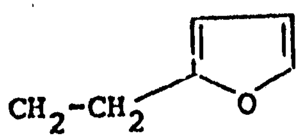 | H | B | 7.4-6.3m (3H); 2.8-2.3m (4H); 2.0-1.0m (20H) |
| 627 | 1 | $CH_3$ | 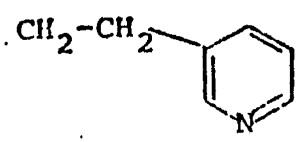 | $C_2H_5$ | B | 8.6-7.1m (4H); 2.9m (2H); 2.0-1.0m (14H); 1.2d (3H) |

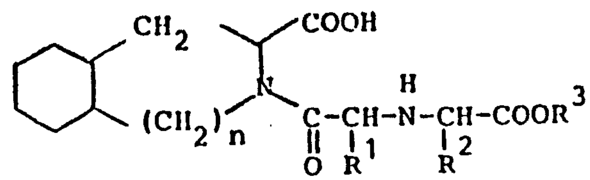

| | n | $R^1$ | $R^2$ | $R^3$ | Methode | NMR |
|---|---|---|---|---|---|---|
| 628 | 1 | $CH_3$ | 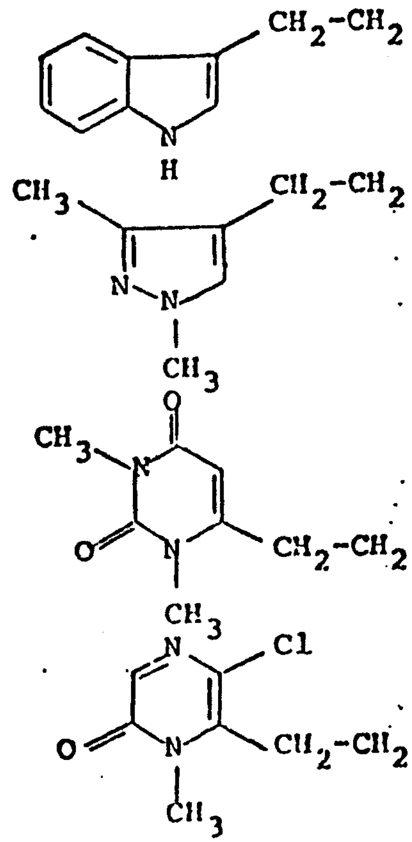 | $C_2H_5$ | B | 7.8-6.5m (6H); 2.9m (2H); 2.0-1.0m (14H); 1.2d (3H) |
| 629 | 1 | $CH_3$ | | $C_2H_5$ | B | 6.7s (1H); 3.9-3.0m+s (6H); 2.8m (2H); 2.0s (3H); 2.0-1.0m (14H); 1.2d (3H) |
| 630 | 1 | $CH_3$ | | $C_2H_5$ | B | 5.2s (1H); 3.2 2s (6H); 2.9m (2H); 2.0-1.0m (14H) 1.2d (3H) |
| 631 | 1 | $CH_3$ | | $C_2H_5$ | B | 8.1s (1H); 3.2s (3H); 2.9m (2H); 2.0-1.0m (14H); 1.2d (3H) |

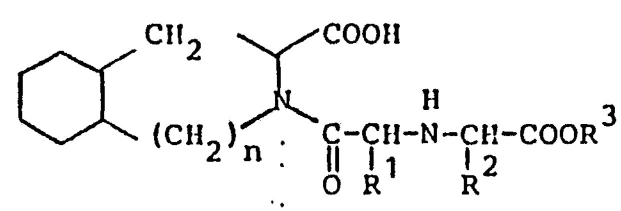

| | n | $R^1$ | $R^2$ | $R^3$ | Methode | NMR |
|---|---|---|---|---|---|---|
| 632 | 1 | $CH_3$ | 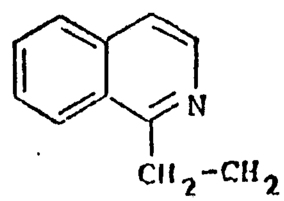 | $C_2H_5$ | B | 8.5-7.5m (6H); 3.9-3.0m (5H); 2.0-1.0m (14H); 1.2d (3H) |
| 633 | 1 | $CH_3$ | 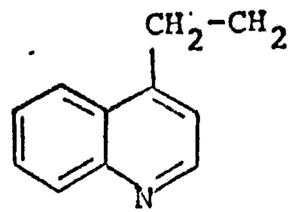 | $C_2H_5$ | B | 8.8-7.4m (6H); 3.9-3.0m (5H); 2.0-1.0m (14H); 1.2d (3H) |
| 634 | 1 | $CH_3$ | 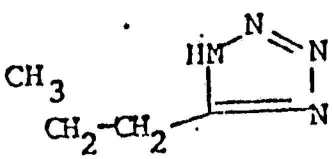 | $C_2H_5$ | B | 13.0s (1H); 3.9-2.9m (5H) 2.0-1.0m (14H); 1.2d (3H) |
| 635 | 1 | $CH_3$ | 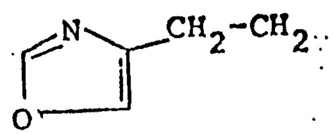 | $C_2H_5$ | B | 7.9-7.4 2s (2H); 2.8m (2H) 2.0-1.0m (14H); 1.2d (3H) |

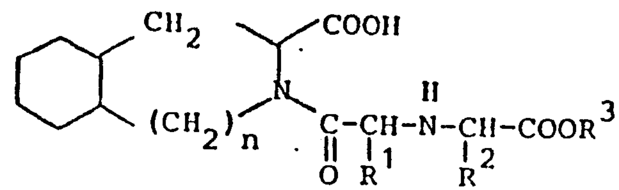

| | n. | $R^1$ | $R^2$ | $R^3$ | Methode | NMR |
|---|---|---|---|---|---|---|
| 636 | 1 | $CH_3$ | 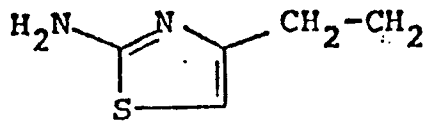 | $C_2H_5$ | B | 8.0s (1H); 2.8m (2H); 2.0-1.0m (14H); 1.2d(3H) |
| 637 | 1 | $CH_3$ | 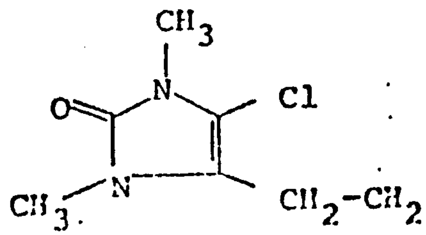 | $C_2H_5$ | B | 3.2 2s (6H); 3.9-3.0m (5H); 2.0-1.0m (14H); 1.2d (3H) |
| 638 | 1 | $CH_3$ | 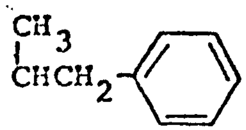 | $C_2H_5$ | B | 7.2bs (5H); 2.7m (2H); 2.2-1.0m (13H); 1.2d (3H); 1.0d (3H) |
| 639 | 1 | $H_2N-(CH_2)_4$ | 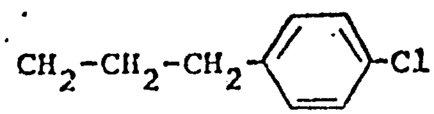 | H | B | 7.4-7.0m (4H); 2.7-2.3m (4H); 2.0-1.0m (22H) |

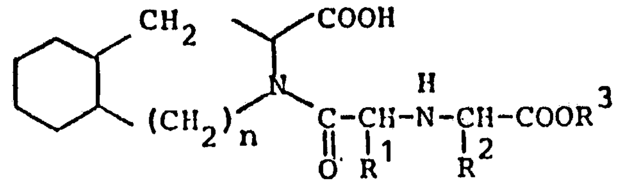

| | n | $R^1$ | $R^2$ | $R^3$ | Methode | NMR |
|---|---|---|---|---|---|---|
| 640 | 1 | $CH_3$ | 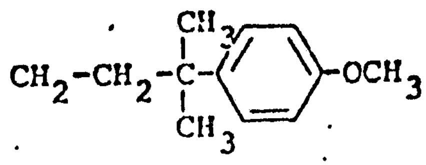 | $C_2H_5$ | B | 7.3-6.3m (4H); 3.9s (3H); 2.0-1.0m (16H); 1.2d (3H); 1.0s (6H) |
| 641 | 1 | 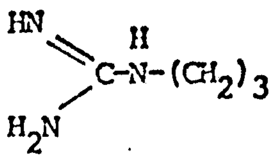 | 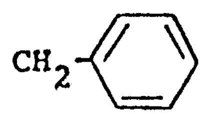 | H | B | 7.2bs (5H); 2.9-2.6m (4H); 2.0-1.0m (16H) |
| 642 | 1 | H | 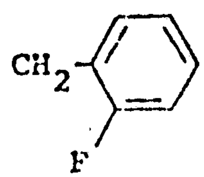 | H | B | 7.4-6.9m (4H); 3.9-3.0m (4H); 2.7m (2H) |
| 643 | 1 | H | 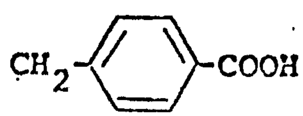 | H | B | 7.8-7.0m (4H); 3.9-3.0m (4H); 2.7m (2H) |

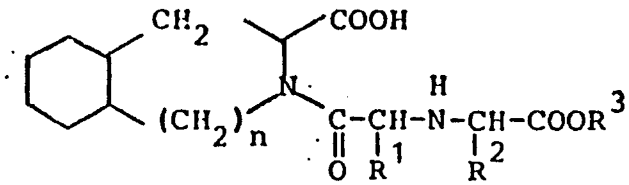

| | n | $R^1$ | $R^2$ | $R^3$ | Methode | NMR |
|---|---|---|---|---|---|---|
| 644 | 1 | H | 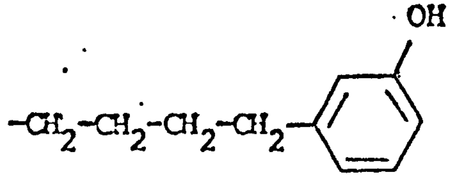 | H | B | 7.3-6.5m (4H); 3.9-3.0m (4H); 2.7m (2H); 2.0-1.0m (18H) |
| 645 | 1 | $CH_3$ | 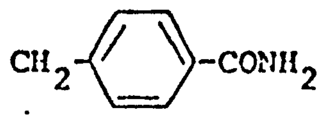 | H | B | 7.8-7.0m (4H); 2.8m (2H); 1.2d (3H) |
| 646 | 1 | $CH_3$ | 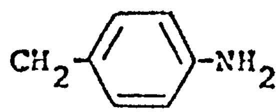 | H | B | 7.2-6.4m (4H); 2.7m (2H); 1.2d (3H) |
| 647 | 1 | $CH_3$ | 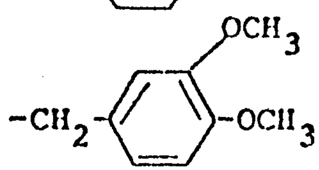 | H | B | 7.2-6.3m (3H); 3.9s (6H); 2.7m (2H); 1.2d (3H) |

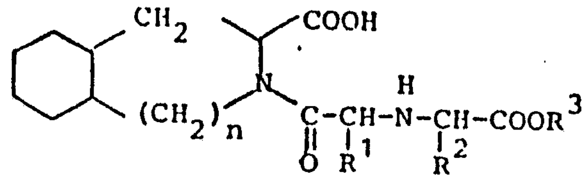

| | n | $R^1$ | $R^2$ | $R^3$ | Methode | NMR |
|---|---|---|---|---|---|---|
| 648 | 1 | $(CH_3)_2CH-CH_2$ | 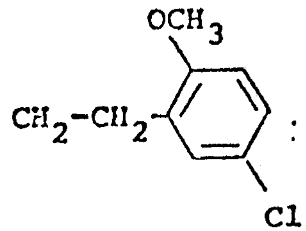 | $C_2H_5$ | B | 7.2-6.4m (3H); 3.8s (3H); 2.8m (2H); 2.0-1.0m (17H) 0.9d (6H) |
| 649 | 1 | $CH_3$ | 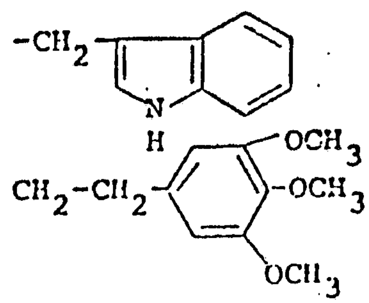 | $C_2H_5$ | B | 7.8-6.5m (6H); 2.9m (2H); 1.2d (3H) |
| 650 | 1 | $(CH_3)_2CH-CH_2$ | | H | B | 7.2-6.2m (3H); 3.9s (9H); 2.7m (2H); 2.0 - 1.0m (17H); 0.9d (6H) |
| 651 | 1 | $CH_3$ | 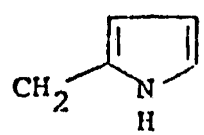 | H | B | 7.3-6.0m (3H); 2.8m (2H); 1.2d (3H) |
| 652 | 1 | $CH_3$ | 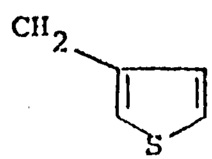 | H | B | 7.3-6.9m (3H); 2.9m (2H); 1.2d (3H) |

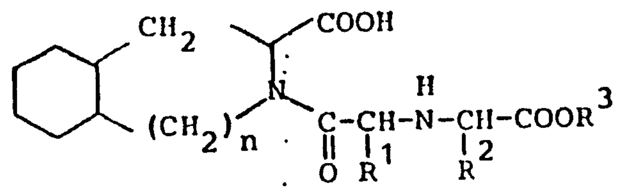

| | n | $R^1$ | $R^2$ | $R^3$ | Methode | NMR |
|---|---|---|---|---|---|---|
| 653 | 1 | $CH_3$ | 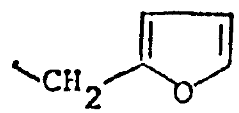 | H | B | 7.4-6.3m (3H); 2.8m (2H); 1.2d (3H) |
| 654 | 1 | $CH_3$ | 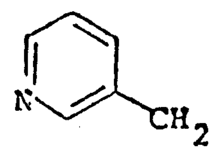 | H | B | 8.6-7.1m (4H); 2.9m (2H); 1.2d (3H) |
| 655 | 1 | $CH_3$ | 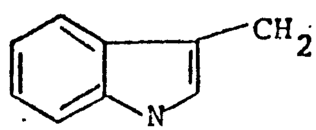 | H | B | 7.8-6.5m (6H); 2.9m (2H); 1.2d (3H) |
| 656 | 1 | $(CH_3)_2CH-CH_2$ | 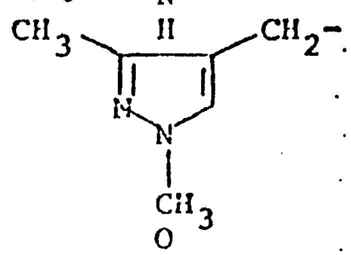 | $C_2H_5$ | B | 6.5s (1H); 3.9-3.0m+s (6H) 2.8m (2H); 2.0s (3H); 2.0-1.0m (15H); 1.0d (6H) |
| 657 | 1 | H | 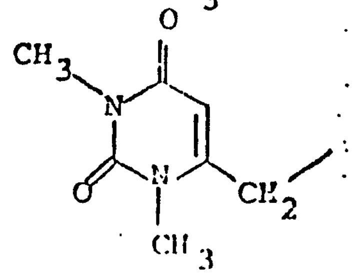 | $C_2H_5$ | B | 5.2s (1H); 3.2s (6H); 3.9-2.9m (5H) |

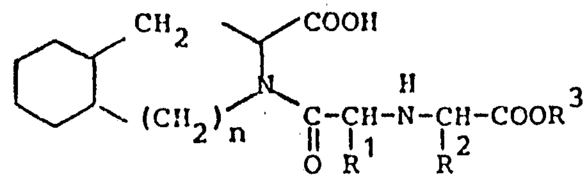

| | n | $R^1$ | $R^2$ | $R^3$ | Methode | NMR |
|---|---|---|---|---|---|---|
| 658 | 1 | $CH_3$ | 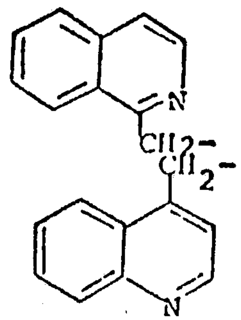 | H | B | 8.5-7.5m (6H); 3.9-3.0m (5H); 1.2d (3H) |
| 659 | 1 | $CH_3$ | | H | B | 8.8-7.4m (6H); 3.9-3.0m (5H); 1.2d (3H) |
| 660 | 1 | $CH_3$ | 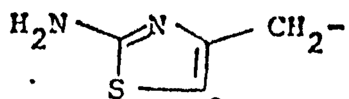 | H | B | 8.0s (1H); 3.9-2.8m (5H) 1.2d (3H) |
| 661 | 1 | $CH_3$ | 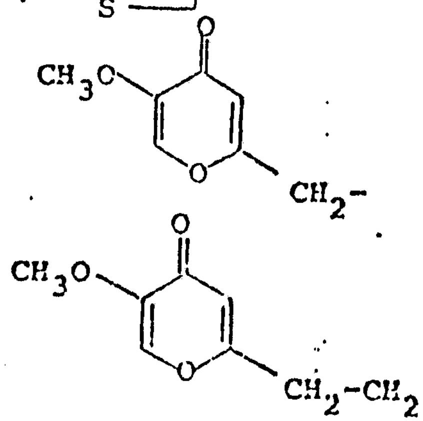 | H | B | 8.1s (1H); 6.4s (1H); 3.7s (3H); 3.9-2.9m(5H) 1.2d (3H) |
| 662 | 1 | $(CH_3)_2CH-CH_2$ | | H | B | 8.1s (1H); 6.4s (1H); 3.7s (3H); 3.9-2.9m (5H); 2.0-1.0m (17H); 1.0d (6H) |

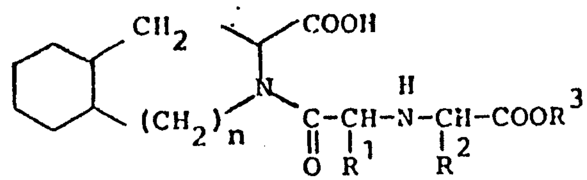

| | n | $R^1$ | $R^2$ | $R^3$ | Methode | NMR |
|---|---|---|---|---|---|---|
| 663 | 1 | $CH_3$ | 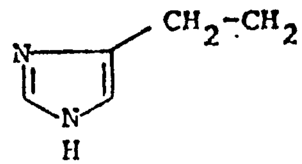 | $C_2H_5$ | B | 7.7-7.1m (2H); 3.9-2.9m (5H); 2.0-1.0m (14H) 1.2d (3H) |
| 664 | 1 | $CH_3$ | 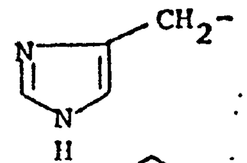 | $C_2H_5$ | B | 7.7-7.1m (2H); 3.9-2.9m (5H); 1.2d (3H) |
| 665 | 0 | $CH_3$ | 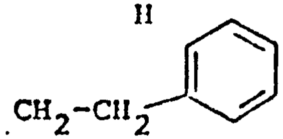 | $C_2H_5$ | B | 7.2-6.5m (5H); 2.7m (2H); 2.0-1.0m (13H); 1.2d (3H) |
| 666 | 0 | $CH_3$ | 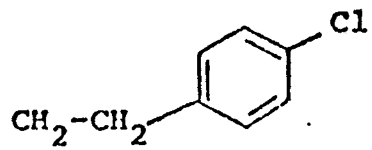 | H | B | 7.5-6.5m (4H); 2.7m (2H); 2.0-1.0m (13H); 1.2d(3H) |
| 667 | 0 | $CH_3$ | 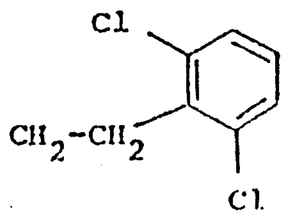 | H | B | 7.6-6.5m (3H); 4.5-2.8m (5H); 2.0-1.0m (13H); 1.2d (3H) |

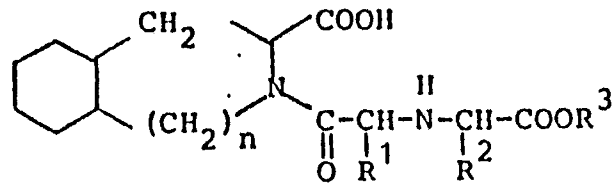

| | n | $R^1$ | $R^2$ | $R^3$ | Methode | NMR |
|---|---|---|---|---|---|---|
| 668 | 0 | $CH_3$ | 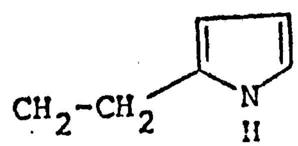 | H | B | 7.3-6.0m (3H); 2.8m (2H); 2.0-1.0m (13H); 1.2d(3H) |
| 669 | 0 | $CH_3$ | 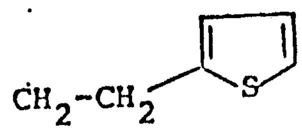 | H | B | 7.3-6.5m (3H); 4.5-2.9m (5H); 2.0-1.0m (13H); 1.2d (3H); |
| 670 | 0 | $CH_3$ | 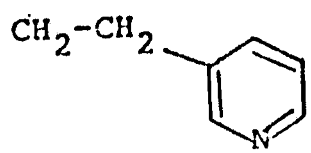 | H | B | 8.6-6.5m (4H); 4.5-2.9m 2.0-1.0m (13H); 1.2d (3H) |
| 671 | 0 | $CH_3$ | 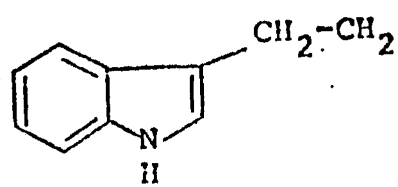 | $C_2H_5$ | B | 7.8-6.5m (6H); 4.5-2.9m (5H); 2.0-1.0m (13H); 1.2d (3H) |

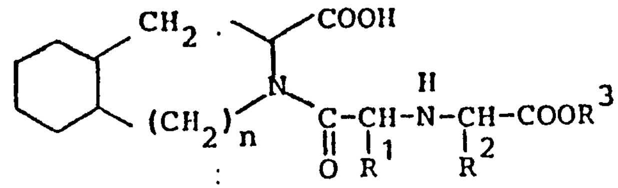

| | n | $R^1$ | $R^2$ | $R^3$ | Methode | NMR |
|---|---|---|---|---|---|---|
| 672 | O | $CH_3$ | 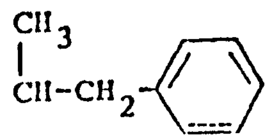 | $C_2H_5$ | B | 7.2-6.5m (5H); 4.5 -2.9m (5H); 2.0-1.0m (12H); 1.2d (3H); 1.1d (3H) |
| 673 | O | $CH_3$ | 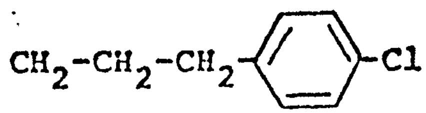 | $C_2H_5$ | B | 7.4-6.5m (4H); 2.7-2.3m (2H); 2.0-1.0m (15H); 1.2d (3H) |
| 674 | O | $CH_3$ | 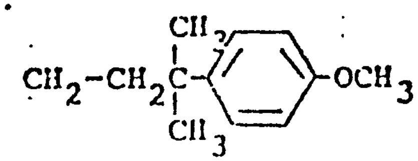 | $C_2H_5$ | B | 7.3-6.3m (4H); 3.9s (3H); 2.0-1.0m (15H); 1.2d (3H); 1.0s (6H) |
| 675 | O | $CH_3$ | 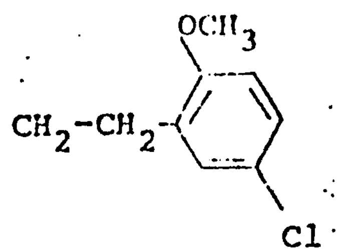 | $C_2H_5$ | B | 7.2-6.3m (3H); 3.0s (3H); 2.7m (2H); 2.0-1.0m (13H); 1.2d (3H) |

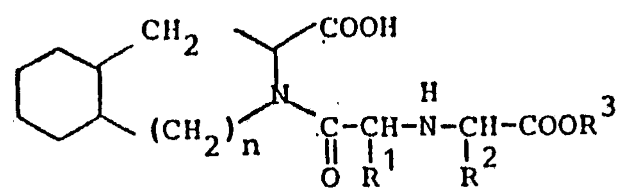

| | n | $R^1$ | $R^2$ | $R^3$ | Methode | NMR |
|---|---|---|---|---|---|---|
| 676 | O | $CH_3$ | 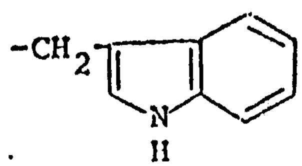 | $C_2H_5$ | B | 7.8-6.5m (6H); 4.5 -2.9m (5H); 1.2d (3H) |
| 677 | O | $CH_3$ | 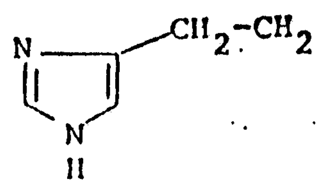 | $C_2H_5$ | B | 7.7-6.5m (2H); 4.5-2.9m (5H); 2.0-1.0m (13H); 1.2d (3H) |
| 678 | O | $CH_3$ | 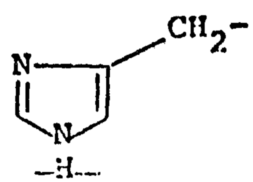 | $C_2H_5$ | B | 7.7-6.5m (2H); 4.5 -2.9m (5H); 1.2d (3H) |
| 679 | 1 | $CH_3$ | $(CH_3)_2-CH-CH_2-CH_2-CH_2$ | $C_2H_5$ | A | 2.0-1.0m (19H); 1.2d (3H); 0.9d (6H) |

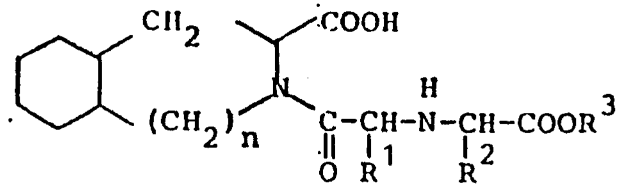

| | n | $R^1$ | $R^2$ | $R^3$ | Methode | NMR |
|---|---|---|---|---|---|---|
| 680 | 1 | $H_2N-(CH_2)_4$ | $(CH_3)_2CH-CH_2-CH_2$ | $C_2H_5$ | B | 2.4m (2H); 2.0-1.0m (23H); 0.9d (6H) |
| 681 | 1 | $H_2N-(CH_2)_3$ | $(CH_3)_2CH-CH_2$ | $C_2H_5$ | B | 2.0-1.0m (19H); 0.9d (6H) |
| 682 | O | $FCH_2$ | $(CH_3)_2CH$ | $C_2H_5$ | B | 4.3d (2H); 2.0-1.0m (12H); 0.9d (6H) |
| 683 | 1 | $CH_3-CH_2-\underset{CH_3}{CH}-$ | 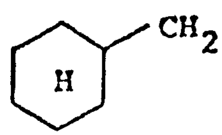 | H | A | 2.0-1.0m (28H); 1.0d+t (6H) |

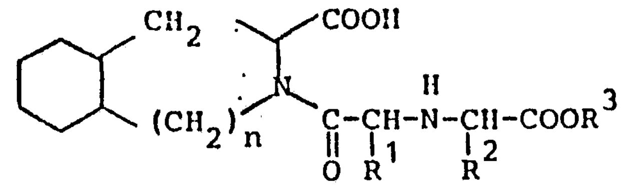

| | n | $R^1$ | $R^2$ | $R^3$ | Methode | NMR |
|---|---|---|---|---|---|---|
| 684 | 1 | $CH_3$ | 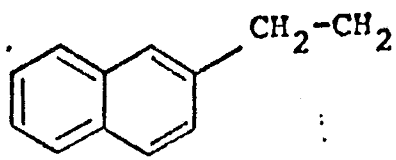 | $C_2H_5$ | A | 7.8-7.0m (7H); 2.7m (2H); 2.0-1.0m (14H); 1.2d(3H) |
| 685 | 1 | $CH_3$ | 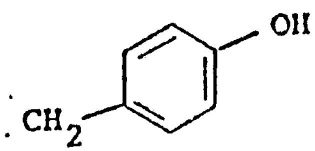 | H | B | 7.3-6.5m (4H); 2.8m (2H); 1.2d (3H) |
| 686 | 1 | $CH_3$ | 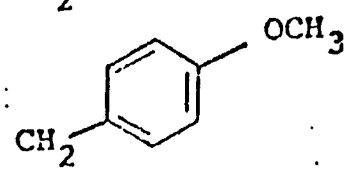 | H | B | 7.3-6.4m (4H); 3.9s (3H); 3.9-2.9m (5H); 1.2d (3H) |
| 687 | 1 | $CH_3$ | 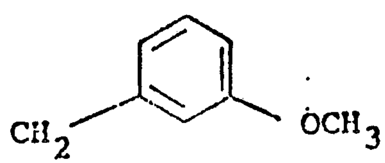 | $C_2H_5$ | B | 7.6-6.4m (4H); 3.8s (3H); 3.9-2.9m (5H); 1.2d (3H) |

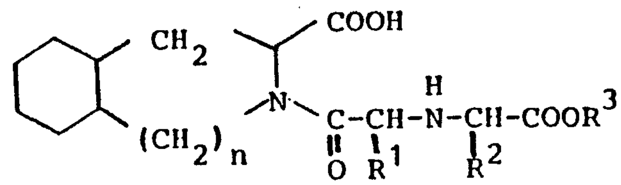

| | n | $R^1$ | $R^2$ | $R^3$ | Methode | NMR |
|---|---|---|---|---|---|---|
| 688 | O | $CH_3$ | 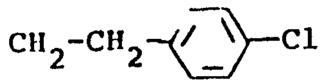 | $C_2H_5$ | B | 7.5-6.5m (4H); 2.7m (2H; 2.0-1.0m (13H); 1.2d (3H) |
| 689 | O | $CH_3$ | 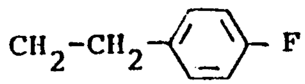 | $C_2H_5$ | B | 7.5-6.6m (4H); 2.7m (2H); 2.0-1.0m (13H); 1.2d (3H) |
| 690 | O | $CH_3$ | 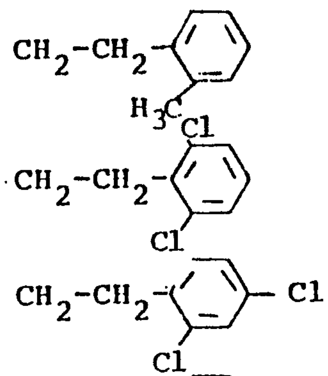 | $C_2H_5$ | B | 7.4-7.0m (4H); 2.6m (2H); 2.3s (3H); 2.0-1.0m (13H); 1.2d (3H) |
| 691 | O | $CH_3$ | | $C_2H_5$ | B | 7.6-6.5m (3H); 4.5-2.8m (5H); 2.0-1.0m (13H); 1.2d (3H) |
| 692 | O | $CH_3$ | | $C_2H_5$ | B | 7.5-6.5m (3H); 4.5-2.8m (5H); 2.0-1.0m (13H); 1.2d (3H) |
| 693 | O | $CH_3$ | 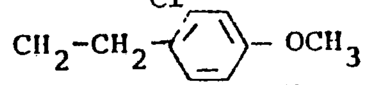 | $C_2H_5$ | B | 7.3-6.4m (4H; 4.5-2.8m (8H); 2.0-1.0m (13H); 1.2d (3H) |
| 694 | O | $CH_3$ | 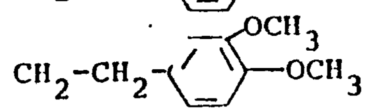 | $C_2H_5$ | B | 7.2-6.3m (3H), 3.9s (6H); 4.5-2.5m (5H); 2.0-1.0m (13H) |
| 695 | O | $CH_3$ | 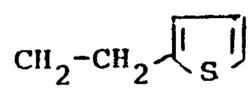 | $C_2H_5$ | B | 7.3-6.5m (3H); 4.5-2.9m (5H); 2.0-1.0m (13H); 1.2d (3H) |

## Ansprüche

1. Verbindung der Formel I

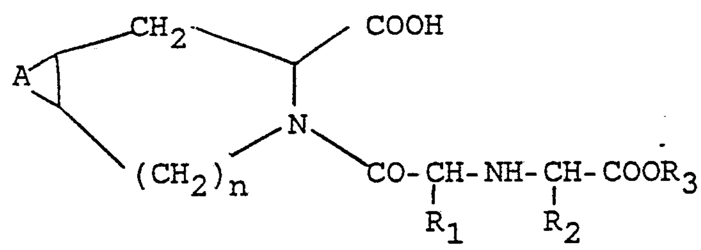

(I)

in welcher
n = 0 und 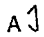 einen Benzol-oder Cyclohexanring oder
n = 1 und 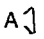 einen Cyclohexanring bedeuten,
$R_1$ und $R_2$ gleich oder verschieden sind und
a) Alkyl oder Alkenyl mit bis zu 6 C-Atomen bedeuten, welche jeweils gegebenenfalls substituiert sind durch einen oder mehrere gleiche oder verschiedene Substituenten aus der Reihe
1. $(C_5$-$C_7)$-Cycloalkyl,
2. $(C_5$-$C_7)$-Cycloalkenyl,
3. Fluor,
4. Mercapto,
5. Hydroxy,
6. $(C_1$-$C_3$-Alkoxy, das gegebenenfalls substituiert ist durch
    6.1 Methoxy
    6.2 Ethoxy
    6.3 Carboxy
    6.4 Carbamoyl
    6.5 Amino
    6.6 $(C_1$-$C_6)$-Alkylamino oder
    6.7 Dimethylamino,
7. Aryloxy, das gegebenenfalls substituiert ist durch die unter a) 6.1-6.6 genannten Reste oder durch
    7.1 Halogen,
    7.2 Nitro,
    7.3 Ethoxycarbonyl und/oder
    7.4 Methyl,
8. Aralkyloxy mit 1 oder 2 C-Atomen im Alkylteil, das im Alkylteil gegebenenfalls wie unter a) 6.1-6.6 beschrieben substituiert ist und das im Arylteil gegebenenfalls wie vorstehend im Alkylteil, wie unter a) 7.1 oder 7.2 beschrieben oder durch Methyl substituiert ist,
9. Amino,
10. Monoalkylamino mit bis zu 7 C-Atomen, das im Alkylrest gegebenenfalls substituiert ist durch
    10.1 Hydroxy,
    10.2 Carboxy,
    10.3 Carbamoyl,
    10.4 Ethoxycarbonyl,
    10.5 Amino,
    10.6 $(C_1$-$C_6)$-Alkylamino,
    10.7 Di-$(C_1$-$C_6)$-alkylamino,
    10.8 Biperidino oder
    10.9 Morpholino,
11. Dialkylamino mit bis zu 7 C-Atomen, das im Alkylrest gegebenenfalls wie unter a) 10.1-10.9 beschrieben substituiert ist,
12. Cycloalkylamino mit bis zu 7 C-Atomen, das im Alkylrest gegebenenfalls wie unter a) 10.1-10.9 beschrieben substituiert ist,

13. Dicycloalkylamino mit bis zu 7 C-Atomen, das im Alkylrest gegebenenfalls wie unter a) 10.1-10.9 beschrieben substiuiert ist,
14. ($C_1$-$C_6$)-Alkoxycarbonylamino,
15. Aryloxycarbonylamino, das im Arylteil gegebenenfalls mono-oder disubstituiert ist durch
15.1 ($C_1$-C )-Alkyl,
15.2 ($C_1$-$C_2$)-Alkoxy,
15.3 Methylendioxy,
15.4 Amino,
15.5 Hydroxy,
15.6 Acetoxy,
15.7 Carboxy,
15.8 Carbamoyl,
15.9 Ethoxycarbonyl,
15.10 Halogen oder
15.11 Nitro,
16. Aralkyloxycarbonyl, das im Arylteil gegebenenfalls wie unter a) 15.1-15.11 beschrieben substituiert ist,
17. Phenylcarbamoyloxy,
18. ($C_1$-$C_6$)-Alkylureido,
19. Cyclohexylureido,
20. Arylureido, das im Arylteil gegebenenfalls wie unter a) 15.1-15.11 beschrieben durch gleiche oder verschiedene Reste substituiert ist,
21. Aralkylureido, das im Arylteil gegebenenfalls wie unter a) 15.1-15.11 beschrieben substituiert ist,
22. Formyl,
23. Alkanoylamino mit bis zu 10 C-Atomen,
24. Aroylamino mit bis zu 10 C-Atomen, das im Arylteil gegebenenfalls wie unter a) 15.1-15.11 beschrieben bis zu trisubstituiert ist,
25. Aralkanoylamino mit bis zu 10 C-Atomen, das im Arylteil gegebenenfalls wie unter a) 15.1-15.11 beschrieben substituiert ist,
26. Arylamino, das im Arylteil gegebenenfalls wie unter a) 15.1-15.11 durch gleiche oder verschiedene Reste substituiert ist,
27. 4,6-Dimethyl-2-pyrimidyl-amino,
28. Aralkylamino, das im Arylteil gegebenenfalls wie unter a) 15.1-15.11 beschrieben substituiert ist,
29. Alkylmercapto, Alkylsulfinyl oder Alkylsulfonyl mit bis zu 7 C-Atomen, das jeweils im Alkylteil gegebenenfalls substituiert ist durch
29.1 Methoxy,
29.2 Ethoxy,
29.3 Hydroxy,
29.4 Carboxy,
29.5 Carbamoyl,
29.6 Ethoxycarbonyl
29.7 Amino,
29.8 ($C_1$-$C_6$)-Alkylamino oder
29.9 Dimethylamino,
30. Phenylmercapto, Phenylsulfinyl oder Phenylsulfonyl, das im Arylteil gegebenenfalls wie unter a) 29.1-29.8 beschrieben oder durch
30.1 Halogen,
30.2 Nitro,
30.3 Sulfamoyl oder
30.4 Methyl substituiert ist,
31. Benzylmercapto, Benzylsulfinyl oder Benzylmefonyl, das im Alkylteil gegebenenfalls wie unter a) 29.1-29.8 und das im Arylteil gegebenenfalls wie vorstehender Alkylteil oder wie unter a) 30.1-30.3 beschrieben substituiert ist,
32. Carboxy,
33. Alkyloxycarbonyl mit bis zu 3-Alkyl-C-Atomen,
34. Benzyloxycarbonyl,
35. Carbamoyl,
36. Alkylcarbamoyl mit bis zu 6 C-Atomen,
37. Cycloalkylcarbamoyl mit bis zu 6 C-Atomen,

38. Cycloalkenylcarbamoyl mit bis zu 6 C-Atomen,
39. Dialkylcarbamoyl mit bis zu 6 C-Atomen,
40. Arylcarbamoyl, das im Arylteil gegebenenfalls substituiert ist durch
    40.1 Halogen,
    40.2 ($C_1$-$C_6$)-Alkyl,
    40.3 ($C_1$-$C_6$)-Alkoxy,
    40.4 Carboxy,
    40.5 Carbamoyl,
    40.6 Ethoxycarbonyl,
    40.7 Hydroxy und/oder
    40.8 Nitro,
41. Aralkylcarbamoyl, das im Arylteil gegebenenfalls substituiert ist durch
    41.1 Halogen,
    41.2 ($C_1$-$C_6$)-Alkyl oder
    41.3 ($C_1$-$C_6$)-Alkoxy,
42. Guanidino,
43. Phenyl, Naphthyl, Dihydronaphthyl oder Tetrahydronaphthyl, die jeweils gegebenenfalls bis zu trisubstituiert sind durch
    43.1 Halogen,
    43.2 Hydroxy,
    43.3 Acetoxy,
    43.4 Carboxy,
    43.5 Carbamoyl,
    43.6 Sulfamoyl,
    43.7 Nitro,
    43.8 Methyl,
    43.9 Ethyl,
    43.10 Methoxy,
    43.11 Ethoxy und/oder
    43.12 Amino,
44. einen 5-bis 7-gliedrigen monocyclischen oder 9-bis 10-gliedrigen bicyclischen Heterocyclen-Rest, gegebenenfalls 1 bis 2 S-oder O-Atome und/oder 4 N-Atome pro Ring enthaltend, der gegebenenfalls substituiert durch
    44.1 Halogen,
    44.2 Sauerstoff,
    44.3 Hydroxy,
    44.4 Carboxy,
    44.5 Carbamoyl,
    44.6 Sulfamoyl,
    44.7 Nitro,
    44.8 Alkyl mit bis zu 0 C-Atomen,
    44.9 Aralkyl mit bis zu 9 C-Atomen,
    44.10 Methoxy,
    44.11 Ethoxy und/oder
    44.12 Amino;

b) Cycloalkyl oder Cycloalkenyl mit je 5-7 C-Atomen bedeuten;

c) Aryl oder teilhydriertes Aryl bedeuten;

d) ein zu mono-oder bicyclischer Heterocyclen-Rest mit 5-7 bzw. 8-10 Gliedern, davon 1-2 -S-oder -O- und/oder bis zu 4 N-Atomen bedeuten, das gegebenenfalls wie unter a) 44.1-44.11 beschrieben substituiert ist

oder

e) $R_1$ Wasserstoff bedeutet und

$R_2$ wie unter a)-d) beschrieben definiert ist und

$R_3$ Wasserstoff, Alkyl mit 1-6 C-Atomen, Alkenyl mit 2-6 C-Atomen, Aralkyl mit 7-14 C-Atomen inklusive p-Nitrobenzyl bedeutet,

wobei, falls im einzelnen nicht anders definiert, Aryl 6-10 C-Atome aufweist und gegebenenfalls durch Halogen, ($C_1$-$C_6$)-Alkyl oder ($C_1$-$C_6$)-Alkoxy substituiert ist, und

Aralkyl Benzyl oder Phenethyl bedeutet und gegebenenfalls durch Halogen, ($C_1$-$C_6$)-Alkyl oder ($C_1$-$C_6$)-

Alkoxy im Phenylkern substituiert ist,
oder deren physiologisch verträglichen Salze,
A) wobei eine Verbindung der Formel I ausgenommen ist, in welcher die Wasserstoffatome an den Brückenkopfpositionen 3a und 7a zueinander cis-konfiguriert sind und worin
n = 0,
A⌉ einen Cyclohexanring,
$R_1$ Methyl,
$R_2$ 2-Phenyl-ethyl und
$R_3$ Wasserstoff oder Alkyl mit 1-3 C-Atomen bedeuten
B) und wobei, falls von der Definition gemäß A) noch nicht umfaßt, weiterhin folgende Verbindungen ausgenommen sind:
B1) 1-[N-(1(RS)-Carboxylethyl)-(S)-alanyl]-octahydroindol-2-carbonsäure,
B2) 1-[N-[1(RS)-Ethoxycarbonyl-2-(1(RS)-ethoxycarbonylethylmercapto)-ethyl]-(S)-alanyl-octahydroindol-2-(S)-carbonsäure,
B3) 1-[N-[1(RS)-Ethoxycarbonyl-2-ethoxycarbonylethylmercapto)-ethyl]-(S)-alanyl-octahydroindol-2-(S)-carbonsäure,
B4) 1-[N-(1-Methoxycarbonyl-3-phenyl-propyl)-S-alanyl]-cis, syn-octahydroindol-2(S)-carbonsäure,
B5) 1-[N-(1-Carboxy-3-phenyl-propyl)-(S)-alanyl]-cis, syn-octahydroindol-2-(S)-carbonsäure,
B6) 1-[N-(3-p-Chlorphenyl-1-ethoxycarbonyl-propyl)-(S)-alanyl]-cis, syn-octahydroindol-2(S)-carbonsäure,
B7) 1-[N-(1-Carboxy-3-p-chlorphenyl-propyl)-(S)-alanyl]-cis, syn-octahydroindol-2(S)-carbonsäure,
B8) 1-[N-(1-Carboxy-2-phenyl-ethyl)-(S)-alanyl]-cis, syn-octahydroindol-2(S)-carbonsäure,
B9) 1-[N-(1-Carboxy-3-(3-indolyl)-propyl]-(S)-alanyl]-cis, syn-octahydroindol-2(S)-carbonsäure,
B10) 1-[N-(1-Ethoxycarbonyl-2-(3-indolyl)-ethyl]-(S)-alanyl]-cis, syn-octahydroindol-2(S)-carbonsäure,
B11) 1-[N-(1-Carboxy-2-phenyl-ethyl)-(S)-alanyl]-cis, syn-octahydroindol-2(S)-carbonsäure,
B12) 1-[N-(1-(Ethoxycarbonyl-2-phenoxy-ethyl)-(S)-alanyl]-cis, syn-octahydroindol-2(S)-carbonsäure,
B13) 1-[N-(1-Carboxy-2-phenyl-mercapto-ethyl]-(S)-alanyl]-cis, syn-octahydroindol-2(S)-carbonsäure,
B14) 1-[N-(1-Carboxy-ethyl)-(S)-alanyl]-cis, syn-octahydroindol-2(S)-carbonsäure,
B15) 1-[N-(1-Carboxy-2-cyclohexyl-ethyl)-(S)-alanyl]-cis, syn-octahydroindol,2(S)-carbonsäure,
B16) 1-[N-(1-Carboxy-5-methyl-hexyl)-(S)-alanyl]-cis, syn-octahydroindol-2(S)-carbonsäure,
B17) 1-[N-(1,3-Dicarboxypropyl-(S)-alanyl]-cis, syn-octahydroindol-2(S)-carbonsäure,
B18) 1-[N-1-Ethoxycarbonyl-3-phenyl-propyl]-(S)-alanyl]-cis, syn-octahydroindol-2(S)-carbonsäure,
B19) 1-[N-(1-Ethoxycarbonyl-2-phenyl-ethyl]-(S)-alanyl]-cis, syn-octahydroindol-2(S)-carbonsäure,
B20) 1-[N-(1-Ethoxycarbonyl-2-phenylmercapto-ethyl)-(S)-alanyl]-cis, syn-octahydroindol-2(S)-carbonsäure,
B21) 1-[N-(1-Methoxycarbonyl-3-phenylpropyl)-(S)-lysyl]-cis, syn-octahydroindol-2(S)-carbonsäure,
B22) 1-[N-(2-Benzyloxy-1-ethoxycarbonyl-ethyl)-(S)-alanyl]-cis, syn-octahydroindol-2(S)-carbonsäure,
B23) 1-[N-(2-Benzylmercapto-1-ethoxycarbonyl-ethyl)-(S)-alanyl]-cis, syn-octahydroindol-2(S)-carbonsäure,
B24) 1-[N-1-Ethoxycarbonyl-ethyl)-(S)-alanyl]-cis-syn-octahydroindol-2(S)-carbonsäure,
B25) 1-[N-(2-Cyclohexyl-1-ethoxycarbonyl-ethyl]-(S)-alanyl]-cis, syn-octahydroindol-2(S)-carbonsäure,
B26) 1-[N-1-Ethoxycarbonyl-5-methyl-hexyl)-(S)-alanyl]-cis, syn-octahydroindol-2(S)-carbonsäure,
B27) 1-[N-2-Benzyloxy-1-carboxy-ethyl]-(S)-alanyl]-cis, syn-octahydroindol-2(S)-carbonsäure,
B28) 1-[N-(2-Benzylmercapto-1-carboxy-ethyl)-(S)-alanyl]-cis, syn-octahydroindol-2(S)-carbonsäure,
B29) 1-[N-(2-p-Chlorbenzyloxy-1-ethoxycarbonyl-ethyl]-(S)-alanyl]-cis, syn-octahydroindol-2(S)-carbonsäure,
B30) 1-[N-(2-p-Chlorbenzyloxy-1-ethoxycarbony-ethyl]-(S)-alanyl]-cis, syn-octahydroindol-2(S)-carbonsäure,
B31) 1-[N-(2-Benzyoxy-1-(S)-ethoxycarbonyl-ethyl)-(S)-alanyl]-3a(S), 7a(S)-octahydroindol-2(S)-carbonsäure, und
B32) 1-[N-2-Benzylmercapto-1-(R)-ethoxycarbonyl-ethyl)-(S)-alanyl]-3a(S), 7a(S)-octahydroindol-2(S)-carbonsäure,
sowie die physiologisch verträglichen Salze der unter A) und B) definierten Verbindungen.

2. Verbindung gemäß Anspruch 1, in welcher $R_1$ Methyl und $R_2$ Phenethyl oder durch Halogen, Methyl oder Methoxy substituiertes Phenethyl ist.

3. Verbindung der Formel Ia

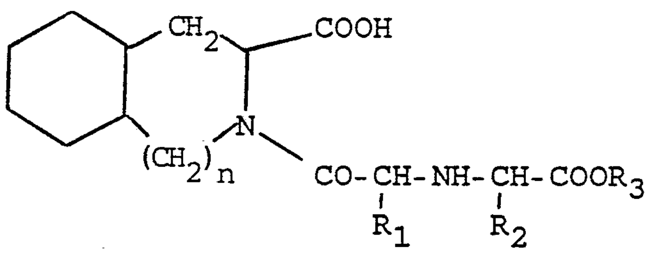

(Ia)

in welcher n, $R_1$, $R_2$ und $R_3$ wie im Anspruch 1 definiert sind, oder deren physiologisch verträglichen Salze.

4. Verbindung der Formel Ib)

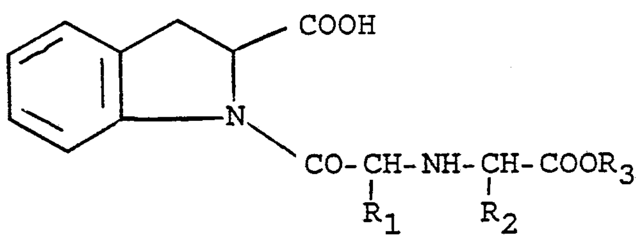

(Ib)

in welcher $R_1$, $R_2$ und $R_3$ wie im Anspruch 1 definiert sind, oder deren physiologisch verträglichen Salze.

5. Verbindung gemäß einem der Ansprüche 1-4, in welcher $R_3$ Wasserstoff, Ethyl, Butyl, t-Butyl, Benzyl, p-Nitrobenzyl bedeutet.

6. Verbindung gemäß einem der Ansprüche 1 oder 3-5, in welcher $R_1$ die Seitenkette einer natürlich vorkommenden L-Aminosäure oder deren Ethers, Esters oder Amids ist.

7. Verbindung gemäß Anspruch 6, in welcher $R_1$ Methyl, Isobutyl, Methylthioethyl, Carboxymethyl, Carboxyethyl, Amino-n-butyl, Guanidino-n-propyl, Imidazol-4-ethyl, Benzyl, 4-Hydroxybenzyl oder 3-Indolmethyl bedeutet.

8. Verbindung gemäß einem der Ansprüche 1 oder 3-5, in welcher $R_1$ Methyl, Ethyl, n-Propyl, Isopropyl, n-Butyl, Isobutyl, tert.-Butyl, geradkettiges oder verzweigtes Pentyl oder Hexyl, Cyclopentyl, Cyclohexyl, Cycloheptyl, Cyclopentenyl, Cyclohexenyl, Cycloheptenyl, Phenyl, Naphthyl, Di-oder Tetrahydronaphthyl, Benzyl, Phenethyl, p-Fluorphenethyl, o-Methylphenethyl, p-Methoxyphenethyl, 2,4-Dichlorphenethyl oder Cyclohexylethyl bedeutet.

9. Verbindung gemäß einem der Ansprüche 1 oder 3-5, in welcher $R_2$ Methyl, Ethyl, n-Propyl, Isopropyl, n-Butyl, Isobutyl, tert.-Butyl, geradkettiges oder verzweigtes Pentyl oder Hexyl, Cyclopentyl, Cyclohexyl, Cycloheptyl, Cyclopentyl, Cyclohexenyl, Cycloheptenyl, Phenyl, Naphthyl, Di-oder Tetrahydronaphthyl, Benzyl, Phenethyl, p-Fluorphenethyl, o-Methylphenethyl, p-Methoxyphenethyl, 2,4-Dichlorphenethyl oder Cyclohexylethyl bedeutet.

10. Verbindung gemäß einem der Ansprüche 1-9, in welcher die zwei oder drei chiralen Zentren an α-C-Atomen in der L-Konfiguration vorliegen.

11. Verfahren zur Herstellung einer Verbindung der Formel I, gemäß einem der Ansprüche 1-10, dadurch gekennzeichnet, daß man

(a) eine Verbindung der Formel II mit einer Verbindung der Formel III umsetzt.

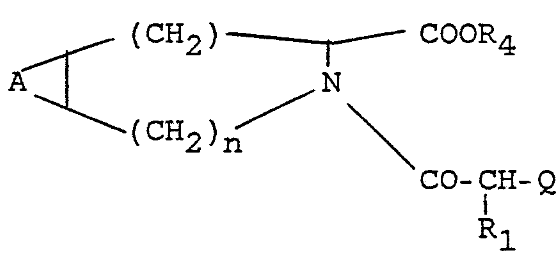

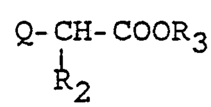

(II) (III)

in denen n, A◁ , $R_1$, $R_2$ und $R_3$ wie im Anspruch 1 definiert sind, ein Q eine nucleofuge Gruppe und das andere Q $-NH_2$ bedeutet und $R_4$ für H, Methyl, Ethyl, Benzyl oder tert.-Butyl steht, und gegebenenfalls einen erhaltenen Ester in die Carbonsäure ($R_2$ und/oder $R_4$ = Wasserstoff) überführt oder

(b) eine Verbindung der Formel IV

POOC - CH -NH - CH -COOP (IV)
$R_1$ $R_2$

worin $R_1$ und $R_2$ wie im Anspruch 1 definiert sind, P H bedeutet, eines der beiden P jedoch auch die anderen Bedeutungen von $R_3$ im Anspruch 1 inklusive tert.-Butyl haben kann, mit einer Verbindung der Formel V

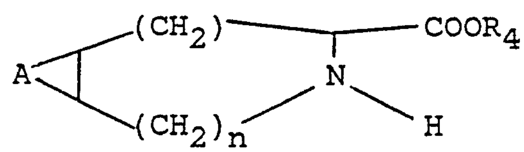

(V)

worin n und A◁ wie im Anspruch 1 und $R_4$ wie oben definiert sind, in Gegenwart eines Kondensationsmittels umsetzt und gegebenenfalls eine oder beide Estergruppen in die Carbonsäure überführt oder

(c) eine Verbindung der Formel VI mit einer Verbindung der Formel VII umsetzt

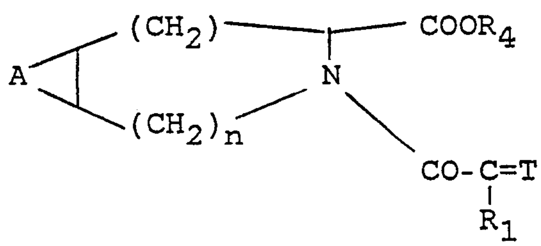

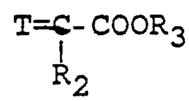

(VI) (VII)

in denen n, A◁ , $R_1$, $R_2$ und $R_3$ wie im Anspruch 1 und $R_4$ wie oben definiert sind, ein T für ein Wasserstoffatom und eine $NH_2$-Gruppe und das andere T für ein Sauerstoffatom steht, und die erhaltene Schiff'sche Base reduziert,
in den nach (a)-(c) erhaltenen Verbindungen Schwefelfunktion gegebenenfalls zum Sulfoxid oder zum Sulfon oxidiert
und die so erhaltene Verbindung der Formel I gegebenenfalls in ihr Salz überführt.

12. Mittel, enthaltend eine Verbindung gemäß einem der Ansprüche 1-10 oder deren physiologisch verträgliches Salz.

13. Verbindung gemäß einem der Ansprüche 1-10 zur Anwendung als Heilmittel.

14. Verbindung gemäß eienm der Ansprüche 1-10 zur Anwendung als Heilmittel bei der Behandlung des Bluthochdrucks.

Patentansprüche für den folgenden Vertragsstaat: AT

1. Verfahren zur Herstellung einer Verbindung der Formel I

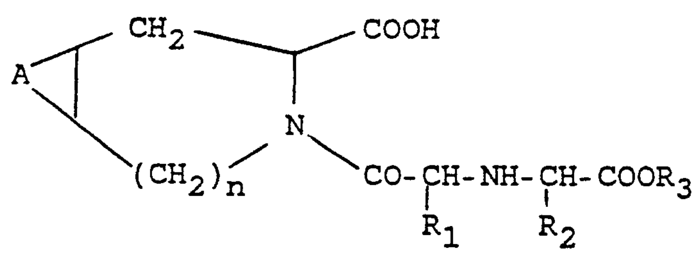

(I)

in welcher

n = 0 und A⌉ einen Benzol-oder Cyclohexanring oder
n = 1 und A⌉ einen Cyclohexanring bedeuten,
$R_1$ und $R_2$ gleich oder verschieden sind und
a) Alkyl oder Alkenyl mit bis zu 6 C-Atomen bedeuten, welche jeweils gegebenenfalls substituiert sind durch einen oder mehrere gleiche oder verschiedene Substituenten aus der Reihe
1. ($C_5$-$C_7$)-Cycloalkyl,
2. ($C_5$-$C_7$)-Cycloalkenyl,
3. Fluor,
4. Mercapto,
5. Hydroxy,
6. ($C_1$-$C_3$-Alkoxy, das gegebenenfalls substituiert ist durch
6.1 Methoxy
6.2 Ethoxy
6.3 Carboxy
6.4 Carbamoyl
6.5 Amino
6.6 ($C_1$-$C_6$)-Alkylamino oder
6.7 Dimethylamino,
7. Aryloxy, das gegebenenfalls substituiert ist durch die unter a) 6.1-6.6 genannten Reste oder durch
7.1 Halogen,
7.2 Nitro,
7.3 Ethoxycarbonyl und/oder
7.4 Methyl,
8. Aralkyloxy mit 1 oder 2 C-Atomen im Alkylteil, das im Alkylteil gegebenenfalls wie unter a) 6.1-6.6 beschrieben substituiert ist und das im Arylteil gegebenenfalls wie vorstehen im Alkylteil, wie unter a) 7.1 oder 7.2 beschrieben oder durch Methyl substituiert ist,
9. Amino,
10. Monoalkylamino mit bis zu 7 C-Atomen, das im Alkyrest gegebenenfalls substituiert ist durch
10.1 Hydroxy,
10.2 Carboxy,
10.3 Carbamoyl,
10.4 Ethoxycarbonyl,
10.5 Amino,
10.6 ($C_1$-$C_6$)-Alkylamino,
10.7 Di-($C_1$-$C_6$)-alkylamino,
10.8 Biperidino oder
10.9 Morpholino,
11. Dialkylamino mit bis zu 7 C-Atomen, das im Alkylrest gegebenenfalls wie unter a) 10.1-10.9 beschrieben substituiert ist,
12. Cycloalkylamino mit bis zu 7 C-Atomen, das im Alkylrest gegebenenfalls wie unter a) 10.1-10.9 beschrieben substituiert ist,
13. Dicycloalkylamino mit bis zu 7 C-Atomen, das im Alkylrest gegebenenfalls wie unter a) 10.1-10.9 beschrieben substituiert ist,
14. ($C_1$-$C_6$)-Alkoxycarbonylamino,
15. Aryloxycarbonylamino, das im Arylteil gegebenenfalls mono-oder disubstituiert ist durch
15.1 ($C_1$-$C_6$)-Alkyl,
15.2 ($C_1$-$C_6$)-Alkoxy,
15.3 Methylendioxy,
15.4 Amino,
15.5 Hydroxy,
15.6 Acetoxy,
15.7 Carboxy,
15.8 Carbamoyl,
15.9 Ethoxycarbonyl,
15.10 Halogen oder
15.11 Nitro,
16. Aralkyloxycarbonyl, das im Arylteil gegebenenfalls wie unter a) 15.1-15.11 beschrieben substituiert ist,
17. Phenylcarbamoyloxy,

18. ($C_1$-$C_6$)-Alkylureido,
19. Cyclohexylureido,
20. Arylureido, das im Arylteil gegebenenfalls wie unter a) 15.1-15.11 beschreiben durch gleiche oder verschiedene Reste substituiert ist,
21. Aralkylureido, das im Arylteil gegebenenfalls wie unter a) 15.1-15.11 beschrieben substituiert ist,
22. Formyl,
23. Alkanoylamino mit bis zu 10 C-Atomen,
24. Aroylamino mit bis zu 10 C-Atomen, das im Arylteil gegebenenfalls wie unter a) 15.1-15.11 beschrieben bis zu trisubstituiert ist,
25. Aralkanoylamino mit bis zu 10 C-Atomen, das im Arylteil gegebenenfalls wie unter a) 15.1-15.11 beschrieben substituiert ist,
26. Arylamino, das im Arylteil gegebenenfalls wie unter a) 15.1-15.11 durch gleiche oder verschiedene Reste substituiert ist,
27. 4,6-Dimethyl-2-pyrimidyl-amino,
28. Aralkylamino, das im Arylteil gegebenenfalls wie unter a) 15.1-15.11 beschrieben substituiert ist,
29. Alkylmercapto, Alkylsulfinyl oder Alkylsulfonyl mit bis zu 7 C-Atomen, das jeweils im Alkylteil gegebenenfalls substituiert ist durch
  29.1 Methoxy,
  29.2 Ethoxy,
  29.3 Hydroxy,
  29.4 Carboxy,
  29.5 Carbamoyl,
  29.6 Ethoxycarbonyl
  29.7 Amino,
  29.8 ($C_1$-$C_6$)-Alkylamino oder
  29.9 Dimethylamino,
30. Phenylmercapto, Phenylsulfinyl oder Phenylsulfonyl, das im Arylteil gegebenenfalls wie unter a) 29.1-29.8 beschrieben oder durch
  30.1 Halogen,
  30.2 Nitro,
  30.3 Sulfamoyl oder
  30.4 Methyl substituiert ist,
31. Benzylmercapto, Benzylsulfinyl oder Benzylmefonyl, das im Alkylteil gegebenenfalls wie unter a) 29.1-29.8 und das im Arylteil gegebenenfalls wie vorstehender Alkylteil oder wie unter a) 30.1-30.3 beschrieben substituiert ist,
32. Carboxy,
33. Alkyloxycarbonyl mit bis zu 3-Alkyl-C-Atomen,
34. Benzyloxycarbonyl,
35. Carbamoyl,
36. Alkylcarbamoyl mit bis zu 6 C-Atomen,
37. Cycloalkylcarbamoyl mit bis zu 6 C-Atomen,
38. Cycloalkenylcarbamoyl mit bis zu 6 C-Atomen,
39. Dialkylcarbamoyl mit bis zu 6 C-Atomen,
40. Arylcarbamoyl, das im Arylteil gegebenenfalls substituiert ist durch
  40.1 Halogen,
  40.2 ($C_1$-$C_6$)-Alkyl,
  40.3 ($C_1$-$C_6$)-Alkoxy,
  40.4 Carboxy,
  40.5 Carbamoyl,
  40.6 Ethoxycarbonyl,
  40.7 Hydroxy und/oder
  40.8 Nitro,
41. Aralkylcarbamoyl, das im Arylteil gegebenenfalls substituiert ist durch
  41.1 Halogen
  41.2 ($C_1$-$C_6$)-Alkyl oder
  41.3 ($C_1$-$C_6$)-Alkoxy,
42. Guanidino,
43. Phenyl, Naphthyl, Dihydronaphthyl oder Tetrahydronaphthyl, die jeweils gegebenenfalls bis zu trisubstituiert sind durch

43.1 Halogen,
43.2 Hydroxy,
43.3 Acetoxy,
43.4 Carboxy,
43.5 Carbamoyl,
43.6 Sulfamoyl,
43.7 Nitro,
43.8 Methyl,
43.9 Ethyl,
43.10 Methoxy,
43.11 Ethoxy und/oder
43.12 Amino,

44. einen 5-bis 7-gliedrigen monocyclischen oder 9-bis 10-gliedrigen bicyclischen Heterocyclen-Rest, gegebenenfalls 1 bis 2 S-oder O-Atome und/oder 4 N-Atome pro Ring enthaltend, der gegebenenfalls substituiert durch

44.1 Halogen,
44.2 Sauerstoff,
44.3 Hydroxy,
44.4 Carboxy,
44.5 Carbamoyl,
44.6 Sulfamoyl,
44.7 Nitro,
44.8 Alkyl mit bis zu 0 C-Atomen,
44.9 Aralkyl mit bis zu 9 C-Atomen,
44.10 Methoxy,
44.11 Ethoxy und/oder
44.12 Amino;

b) Cycloalkyl oder Cycloalkenyl mit je 5-7 C-Atomen bedeuten;
c) Aryl oder teilhydriertes Aryl bedeuten;
d) ein zu mono-oder bicyclischer Heterocyclen-Rest mit 5-7 bzw. 8-10 Gliedern, davon 1-2 -S-oder -O- und/oder bis zu 4 N-Atomen bedeuten, das gegebenenfalls wie unter a) 44.1-44.11 beschrieben substituiert ist
oder
e) $R_1$ Wasserstoff bedeutet und
$R_2$ wie unter a)-d) beschrieben definiert ist und
$R_3$ Wasserstoff, Alkyl mit 1-6 C-Atomen, Alkenyl mit 2-6 C-Atomen, Aralkyl mit 7-14 C-Atomen inklusive p-Nitrobenzyl bedeutet,

wobei, falls im einzelnen nicht anders definiert, Aryl 6-10 C-Atome aufweist und gegebenenfalls durch Halogen, $(C_1-C_6)$-Alkyl oder $(C_1-C_6)$-Alkoxy substituiert ist, und
Aralkyl Benzyl oder Phenethyl bedeutet und gegebenenfalls durch Halogen, $(C_1-C_6)$-Alkyl oder $(C_1-C_6)$-Alkoxy im Phenylkern substituiert ist,
oder deren physiologisch verträglichen Salzen,

A) wobei eine Verbindung der Formel I ausgenommen ist, in welcher die Wasserstoffatome an den Brückenkopfpositionen 3a und 7a zueinander cis-konfiguriert sind und worin
n = 0,
A] einen Cyclohexanring,
$R_1$ Methyl,
$R_2$ 2-Phenyl-ethyl und
$R_3$ Wasserstoff oder Alkyl mit 1-3 C-Atomen bedeuten

B) und wobei, falls von der Definition gemäß A) noch nicht umfaßt, weiterhin folgende Verbindungen ausgenommen sind:
B1) 1-[N-(1(RS)-Carboxylethyl)-(S)-alanyl]-octahydroindol-2-carbonsäure,
B2) 1-[N-[1(RS)-Ethoxycarbonyl-2-(1(RS)-ethoxycarbonylethylmercapto)-ethyl]-(S)-alanyl-octahydroindol-2-(S)-carbonsäure,
B3) 1-[N-[1(RS)-Ethoxycarbonyl-2-ethoxycarbonylethylmercapto)-ethyl]-(S)-alanyl-octahydroindol-2-(S)-carbonsäure,
B4) 1-[N-(1-Methoxycarbonyl-3-phenyl-propyl)-(S)-alanyl]-cis, syn-octahydroindol-2-(S)-carbonsäure,

B5) 1-[N-(1-Carboxy-3-phenyl-propyl)-(S)-alanyl]-cis, syn-octahydroindol-2-(S)-carbonsäure,
B6) 1-[N-(3-p-Chlorphenyl-1-ethoxycarbonyl-propyl)-(S)-alanyl]-cis, syn-octahydroindol-2(S)-carbonsäure,
B7) 1-[N-(1-Carboxy-3-p-chlorphenyl-propyl)-(S)-alanyl]-cis, syn-octahydroindol-2-(S)-carbonsäure,
B8) 1-[N-(1-Carboxy-2-phenyl-ethyl)-(S)-alanyl]-cis, syn-octahydroindol-2-(S)-carbonsäure,
B9) 1-[N-(1-Carboxy-3-(3-indolyl)-propyl]-(S)-alanyl]-cis, syn-octahydroindol-2(S)-carbonsäure,
B10) 1-[N-(1-Ethoxycarbonyl-2-(3-indolyl)-ethyl]-(S)-alanyl]-cis, syn-octahydroindol-2(S)-carbonsäure,
B11) 1-[N-(1-Carboxy-2-phenyl-ethyl)-(S)-alanyl]-cis, syn-octahydroindol-2(S)-carbonsäure,
B12) 1-[N-(1-Ethoxycarbonyl-2-phenoxy-ethyl)-(S)-alanyl]-cis, syn-octahydroindol-2(S)-carbonsäure,
B13) 1-[N-(1-Carboxy-2-phenyl-mercapto-ethyl]-(S)-alanyl]-cis, syn-octahydroindol-2-(S)-carbonsäure,
B14) 1-[N-(1-Carboxy-ethyl)-(S)-alanyl]-cis, syn-octahydroindol-2-(S)-carbonsäure,
B15) 1-[N-(1-Carboxy-2-cyclohexyl-ethyl)-(S)-alanyl]-cis, syn-octahydroindol,2(S)-carbonsäure,
B16) 1-[N-(1-Carboxy-5-methyl-hexyl)-(S)-alanyl]-cis, syn-octahydroindol-2(S)-carbonsäure,
B17) 1-[N-(1,3-Dicarboxypropyl-(S)-alanyl]-cis, syn-octahydroindol-2(S)-carbonsäure,
B18) 1-[N-1-Ethoxycarbonyl-3-phenyl-propyl]-(S)-alanyl]-cis, syn-octahydroindol-2(S)-carbonsäure,
B19) 1-[N-(1-Ethoxycarbonyl-2-phenyl-ethyl]-(S)-alanyl]-cis, syn-octahydroindol-2(S)-carbonsäure,
B20) 1-[N-(1-Ethoxycarbonyl-2-phenylmercapto-ethyl)-(S)-alanyl]-cis, syn-octahydroindol-2(S)-carbonsäure,
B21) 1-[N-(1-Methoxycarbonyl-3-phenylpropyl)-(S)-lysyl]-cis, syn-octahydroindo-2(S)-carbonsäure,
B22) 1-[N-(2-Benzyloxy-1-ethoxycarbonyl-ethyl)-(S)-alanyl]-cis, syn-octahydroindol-2(S)-carbonsäure,
B23) 1-[N-(2-Benzylmercapto-1-ethoxycarbonyl-ethyl)-(S)-alanyl]-cis, syn-octahydroindol-2(S)-carbonsäure,
B24) 1-[N-1-Ethoxycarbonyl-ethyl)-(S)-alanyl]-cis-syn-octahydroindol-2(S)-carbonsäure,
B25) 1-[N-(2-Cyclohexyl-1-ethoxycarbonyl-ethyl]-(S)-alanyl]-cis, syn-octahydroindol-2(S)-carbonsäure,
B26) 1-[N-1-Ethoxycarbonyl-5-methyl-hexyl)-(S)-alanyl]-cis, syn-octahydroindol-2(S)-carbonsäure,
B27) 1-[N-2-Benzyloxy-1-carboxy-ethyl]-(S)-alanyl]-cis, syn-octahydroindol-2(S)-carbonsäure,
B28) 1-[N-(2-Benzylmercapto-1-carboxy-ethyl)-(S)-alanyl]-cis, syn-octahydroindol-2(S)-carbonsäure,
B29) 1-[N-(2-p-Chlorbenzyloxy-1-ethoxycarbonyl-ethyl]-(S)-alanyl]-cis, syn-octahydroindol-2(S)-carbonsäure,
B30) 1-[N-(2-p-Chlorbenzyloxy-1-ethoxycarbony-ethyl]-(S)-alanyl]-cis, syn-octahydroindol-2(S)-carbonsäure,
B31) 1-[N-(2-Benzyoxy-1-(S)-ethoxycarbonyl-ethyl)-(S)-alanyl]-3a(S), 7a(S)-octahydroindol-2(S)-carbonsäure,
und
B32) 1-[N-2-Benzylmercapto-1-(R)-ethoxycarbonyl-ethyl)-(S)-alanyl]-3a(S), 7a(S)-octahydroindol-2(S)-carbonsäure,

sowie die physiologisch verträglichen Salze der unter A) und B) definierten Verbindungen, dadurch gekennzeichnet, daß man
(a) eine Verbindung der Formel II mit einer Verbindung der Formel III umsetzt

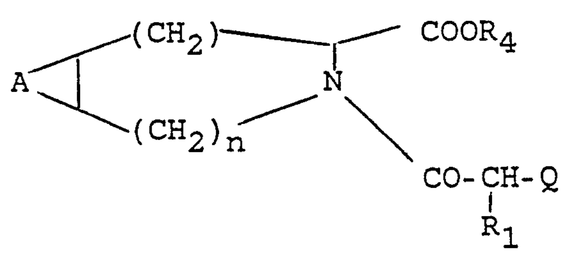
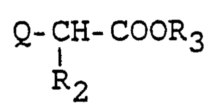

(II) (III)

in denen n, A] , $R_1$, $R_2$ und $R_3$ wie oben definiert sind, ein Q eine nucleofuge Gruppe und das andere Q $-NH_2$ bedeutet und $R_4$ für H, Methyl, Ethyl, Benzyl oder tert.-Butyl steht, und gegebenenfalls einen erhaltenen Ester in die Carbonsäure ($R_3$ und/oder $R_4$ = Wasserstoff) überführt oder
(b) eine Verbindung der Formel IV

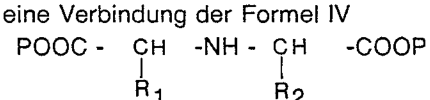 (IV)

worin $R_1$ und $R_2$ wie oben definiert sind, P H bedeutet, eines der beiden P jedoch auch die anderen Bedeutungen von $R_3$ inklusive tert.-Butyl haben kann, mit einer Verbindung der Formel V

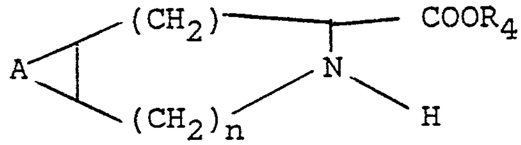

(V)

worin n , 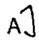 und $R_4$ wie oben definiert sind, in Gegenwart eines Kondensationsmittels umsetzt und gegebenenfalls eine oder beide Estergruppen in die Carbonsäure überführt oder
(c) eine Verbindung der Formel VI mit einer Verbindung der Formel VII umsetzt

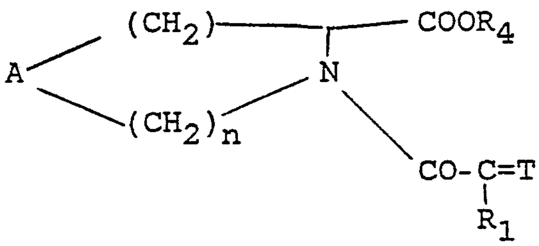

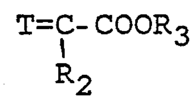

(VI) (VII)

in denen n, A , $R_1$, $R_2$ , $R_3$ und $R_4$ wie oben definiert sind, ein T für ein Wasserstoffatom und eine $NH_2$-Gruppe und das andere T für ein Sauerstoffatom steht, und die erhaltene Schiff'sche Base reduziert,
in den nach (a)-(c) erhaltenen Verbindungen Schwefelfunktionen gegebenenfalls zum Sulfoxid oder zum Sulfon oxidiert
und die so erhaltene Verbindung der Formel I gegebenenfalls in ihr Salz überführt.

2. Verfahren gemäß Anspruch 1, dadurch gekennzeichnet, daß eine Verbindung der Formel I hergestellt wird, in welcher $R_1$ Methyl und $R_2$ Phenethyl oder durch Halogen, Methyl oder Methoxy substituiertes Phenethyl ist.

3. Verfahren gemäß Anspruch 1 zur Herstellung einer Verbindung der Formel Ia

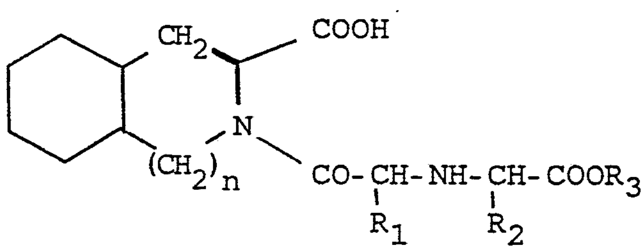

(Ia)

in welcher n, $R_1$, $R_2$ und $R_3$ wie im Anspruch 1 definiert sind,
oder deren physiologisch verträglichen Salzen.

4. Verfahren gemäß Anspruch 1 zur Herstellung einer Verbindung der Formel Ib)

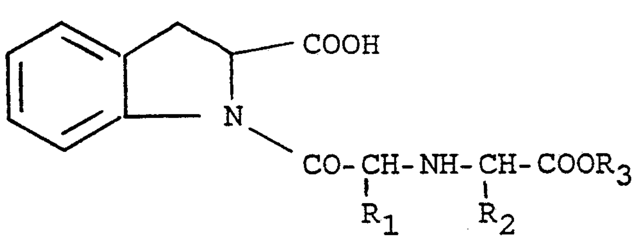

(Ib)

in welcher $R_1$, $R_2$ und $R_3$ wie im Anspruch 1 definiert sind,
oder deren physiologisch verträglichen Salzen.

5. Verfahren gemäß einem der Ansprüche 1-4, dadurch gekennzeichnet, da0 eine Verbindung der Formel I hergestellt wird, in welcher $R_3$ Wasserstoff, Ethyl, Butyl, t-Butyl, Benzyl, p-Nitrobenzyl bedeutet.

6. Verfahren gemäß einem der Ansprüche 1 oder 3-5, dadurch gekennzeichnet, daß eine Verbindung der Formel I hergestellt wird, in welcher $R_1$ die Seitenkette einer natürlich vorkommenden L-Aminosäure oder deren Ethers, Esters oder Amids ist.

7. Verfahren gemäß Anspruch 6, dadurch gekennzeichnet, daß eine Verbindung der Formel I hergestellt wird, in welcher $R_1$ Methyl, Isobutyl, Methylthioethyl, Carboxymethyl, Carboxyethyl, Amino-n-butyl, Guanidino-n-propyl, Imidazol-4-ethyl, Benzyl, 4-Hydroxybenzyl oder 3-Indolmethyl bedeutet.

8. Verfahren gemäß einem der Ansprüche 1 oder 3-5, dadurch gekennzeichnet, daß eine Verbindung der Formel I hergestellt wird, in welcher $R_1$ Methyl, Ethyl, n-Propyl, Isopropyl, n-Butyl, Isobutyl, tert.-Butyl, geradkettiges oder verzweigtes Pentyl oder Hexyl, Cyclopentyl, Cyclohexyl, Cycloheptyl, Cyclopentenyl, Cyclohexenyl, Cycloheptenyl, Phenyl Naphthyl, Di-oder Tetrahydronaphthyl, Benzyl, Phenethyl, p-Fluorphenethyl, o-Methylphenethyl, p-Methoxyphenethyl, 2,4-Dichlorphenethyl oder Cyclohexylethyl bedeutet.

9. Verfahren gemäß einem der Ansprüche 1 oder 3-5, dadurch gekennzeichnet, daß eine Verbindung der Formel I hergestellt wird, in welcher $R_2$ Methyl, Ethyl, n-Propyl, Isopropyl, n-Butyl, Isobutyl, tert.-Butyl, geradkettiges oder verzweigtes Pentyl oder Hexyl, Cyclopentyl, Cyclohexyl, Cycloheptyl, Cyclopentenyl, Cyclohexenyl, Cycloheptenyl, Phenyl, Napththyl, Di-oder Tetrahydronaphthyl, Benzyl, Phenethyl, p-Fluorphenethyl, o-Methylphenethyl, p-Methoxyphenethyl, 2,4-Dichlorphenethyl oder Cyclohexylethyl bedeutet.

10. Verfahren gemäß einem der Ansprüche 1-9, dadurch gekennzeichnet, daß eine Verbindung der Formel I hergestellt wird, in welcher die zwei oder drei chiralen Zentren an $\alpha$-C-Atomen in der L-Konfiguration vorliegen.

11. Verfahren zur Herstellung eines Mittels, enthaltend eine Verbindung der Formel I gemäß einem der Ansprüche 1-10 oder deren physiologisch verträgliches Salz, dadurch gekennzeichnet, daß man diese in eine geeignete Darreichungsform bringt.

12. Verfahren gemäß einem der Ansprüche 1-10 zur Herstellung einer Verbindung der Formel I zur Anwendung als Heilmittel.

13. Verfahren gemäß einem der Ansprüche 1-10 zur Herstellung einer Verbindung der Formel I zur Anwendung als Heilmittel bei der Behandlung des Bluthochdrucks.